# EUROPEAN PATENT SPECIFICATION

(11) **EP 4 069 255 B1**
(45) Date of publication and mention of the grant of the patent: **01.10.2025**
(21) Application number: 20897387.5
(22) Date of filing: 04.12.2020
(51) Int. Cl.: A61K 31/7088, A61K 31/713, A61K 38/02, A61K 45/06, C12Q 1/6806

(54) **IDENTIFYING NON-PRODUCTIVE SPLICE SITES**
IDENTIFIZIERUNG NICHT-PRODUKTIVER SPLEISSSTELLEN
IDENTIFICATION DE SITES D'ÉPISSAGE NON PRODUCTIFS

(30) Priority: 04.12.2019 US 201962943670 P; 04.12.2019 US 201962943672 P
(43) Date of publication of application: 12.10.2022
(73) Proprietor: University of Massachusetts, Westborough, MA 01581 (US)
(72) Inventor: Pai, Athma A., Cambridge, MA 02139 (US); Watts, Jonathan K., Worcester, MA 01609 (US); Valla, Kaitlyn, Worcester, MA 01605 (US); Khokhar, Eraj Shafiq, Worcester, MA 01605 (US); Kartje, Zachary, Worcester, MA 01605 (US)
(74) Representative: Zwicker, Jörk
(86) International application number: PCT/US2020/063489
(87) International publication number: WO 2021/113773

(56) References cited:
- WO-A1-2017/087667
- WO-A1-2017/106283
- WO-A1-2017/106377
- WO-A1-2018/162538
- WO-A1-2018/169900
- WO-A1-2019/038533
- WO-A4-2015/193651
- US-A- 6 017 755
- US-A1- 2004 259 135
- US-A1- 2010 311 039
- US-A1- 2018 223 360
- US-A1- 2018 371 550
- US-A1- 2019 070 213
- US-A1- 2019 330 682
- PAI ATHMA A. ET AL: "Numerous recursive sites contribute to accuracy of splicing in long introns in flies", vol. 14, no. 8, 27 August 2018 (2018-08-27), pages e1007588, XP093088188, Retrieved from the Internet <URL:https://www.ncbi.nlm.nih.gov/pmc/articles/PMC6110457/pdf/pgen.1007588.pdf> DOI: 10.1371/journal.pgen.1007588
- ALTIERI JESSIE A. C. ET AL: "The influence of 4-thiouridine labeling on pre-mRNA splicing outcomes", PLOS ONE, vol. 16, no. 12, 13 December 2021 (2021-12-13), XP055972662, DOI: 10.1371/journal.pone.0257503
- LIM KIAN HUAT ET AL: "Antisense oligonucleotide modulation of non-productive alternative splicing upregulates gene expression", NATURE COMMUNICATIONS, vol. 11, no. 1, 1 December 2020 (2020-12-01), XP055805808, Retrieved from the Internet <URL:https://www.nature.com/articles/s41467-020-17093-9.pdf> DOI: 10.1038/s41467-020-17093-9
- TADASHI KAWASHIMA, STEPHEN DOUGLASS , JASON GABUNILAS , MATTEO PELLEGRINI , GUILLAUME F. CHANFREAU: "Widespread Use of Non-productive Alternative Splice Sites in Saccharomyces cerevisiae", PLOS GENETICS, vol. 10, no. 4, 10 April 2014 (2014-04-10), pages 1 - 15, XP055840085

## Description

### CROSS-REFERENCE TO RELATED APPLICATIONS

This application claims the benefit of U.S. Provisional Application Serial No. 62/943,672, filed December 4, 2019, and U.S. Provisional Application Serial No. 62/943,670, filed December 4, 2019.

### BACKGROUND

The mechanism of mRNA splicing is a conserved and highly-regulated process. Nevertheless, cryptic or nonproductive splicing occurs when the spliceosome utilizes erroneous splice sites and generates transcripts that undergo nonsense-mediated mRNA decay. In cryptic or nonproductive splicing, spliceosome components can often bind to cryptic sites (with either canonical or non-canonical sequence elements) and improperly splice an mRNA molecule. This improper splice site usage can be called noisy, cryptic, or nonproductive splicing. The phenomenon of nonproductive splicing is particularly common in genes with long introns or many introns.

Noisy splicing most often results in non-productive transcripts that are targeted for degradation, e.g. by nonsense-mediated decay pathways. Thus, while these isoforms are rarely observed in steady-state gene expression measurements, they are likely to represent a large amount of the total transcriptional output of a gene.

Due to the transient and variable nature of these non-productive isoforms and the increased probability of weaker splice site usage in these isoforms, sites at which cryptic splicing consistently occur are difficult to identify through standard RNA-sequencing (RNA-seq) methods. In fact, standard RNA-seq data does not show rapidly degraded mRNA splice forms even though these account for a large part of the transcriptional activity of many genes.

A method for identifying recursively spliced introns in nascent RNA sequencing data from Drosophila S2 cells has been described by Pai et al., 2018 (PLOS genetics, 14(8): e1007588).

The international application WO 2017/106283 A1 relates to the treatment of diseases and conditions associated with the liver that proceed via a deficiency in the expression of a gene resulting in a deficiency in the gene product. In particular, the disclosed method of treating a liver disease in a subject involves increasing the expression of a target protein or functional RNA by cells of the subject, wherein the cells have a retained-intron-containing pre-mRNA (RIC pre-mRNA), which encodes the target protein or functional RNA, by contacting the cell of the subject with an antisense oligomer (ASO) complementary to a targeted portion of the RIC pre-mRNA encoding the target protein or functional RNA, whereby the retained intron is constitutively spliced from the RIC pre-mRNA encoding the target protein or functional RNA, thereby increasing the level of mRNA encoding the target protein or functional RNA, and increasing the expression of the target protein or functional RNA in the cells of the subject.

Thus, there is a need to be able to systematically identify non-productive RNA transcript intermediates. Further, there is a need to be able to account for the usage of non-canonical cryptic sites, cell-type specific splicing intermediates, or the complexity of other molecular processes that may result in non-productive splicing intermediates. This is a challenging task given only information about steady-state mRNA levels and the fact that overall gene expression levels can be affected by many post-transcriptional mechanisms.

### SUMMARY

The invention is defined by the appended claims and based on the general teaching including the various aspects and facets described below.

In a first aspect, the disclosure provides a method of identifying a non-productive splice site in a target RNA transcript expressed from a gene that is related to a disease or a disorder of haploinsufficiency, the method comprising:
a) incubating a cell with an affinity label to facilitate incorporation of the affinity label into nascent RNA;
b) capturing the affinity labeled nascent RNA with a solid support comprising specificity for the affinity label;
c) isolating the affinity labeled nascent RNA;
d) sequencing the isolated affinity labeled nascent RNA or cDNA prepared therefrom; and
e) identifying split reads that do not map to exon-exon junctions of the target RNA transcript; and
f) calculating the probability that the split reads represent non-productive transcripts, thereby identifying non-productive splice sites in the target RNA transcript.

In a second aspect, the disclosure provides a method of identifying a non-productive splice site in a target RNA transcript expressed from a gene that is related to a disease or a disorder of haploinsufficiency, the method comprising:
a) incubating a cell with an affinity label to facilitate incorporation of the affinity label into nascent RNA;
b) capturing the affinity labeled nascent RNA with a solid support comprising specificity for the affinity label;
c) isolating the affinity labeled nascent RNA;
d) enriching the target RNA transcript from the isolated affinity labeled nascent RNA;
e) sequencing the enriched isolated affinity labeled nascent RNA or cDNA prepared therefrom; and
f) identifying nascent RNA transcript intermediates, thereby identifying non-productive splice sites in the target RNA transcript.

In an embodiment, step a) comprises incubating the cell for less than about 30 minutes in media containing the affinity label. In an embodiment, the affinity label comprises 4-thiouridine, 6-thio-guanosine, 5-ethynyl-uridine, or bromodeoxyuridine. In an embodiment, the 4-thiouridine labeled nascent RNA is biotinylated to produce biotinylated nascent RNA. In an embodiment, the biotinylated nascent RNA is captured in step b) with a streptavidin linked solid support. In an embodiment, the bromodeoxyuridine labeled nascent RNA is captured in step b) with an anti-bromodeoxyuridine antibody.

In an embodiment of the second aspect of the disclosure, the target enrichment in step d) comprises a pulldown step using nucleic acid probes complementary to the target RNA transcript. In an embodiment of the second aspect of the disclosure, the target enrichment in step d) comprises a pulldown step using nucleic acid primers complementary to the target RNA transcript for selective reverse transcription.

In an embodiment, the cell expresses the target RNA transcript.

In an embodiment of the first aspect of the disclosure, the target RNA transcript exon-exon junctions are annotated target RNA transcript exon-exon junctions or unannotated target RNA transcript exon-exon junctions.

In an embodiment of the second aspect of the disclosure, the method further comprises:
g) identifying split reads that do not map to target RNA transcript exon-exon junctions; and
h) calculating the probability that the split reads represent non-productive transcripts.

In an embodiment, the target RNA transcript exon-exon junctions are annotated target RNA transcript exon-exon junctions or unannotated target RNA transcript exon-exon junctions.

In an embodiment of the second aspect of the disclosure, the method further comprises:
g) identifying split reads that do not map to annotated target RNA transcript exon-exon junctions; and
h) calculating the probability that the split reads represent non-productive transcripts.

In an embodiment, the non-productive transcripts are rapidly degraded.

In an embodiment, the non-productive transcripts are not translated into a functional protein.

In an embodiment, the target RNA transcript comprises ADAR, ARSA, ATP1A2, CACNA1A, DNMT1, EIF2B1, EIF2B2, EIF2B5, IDUA, MFSD8, NF2, NPCl, PEX1, PRICKLE2, PRRT2, RAI1, SETD5, SHANK3, SLC6A1, STXBP1, STX1B, and TCF4.

In an embodiment, the target RNA transcript comprises CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1, and WDTC1.

In an embodiment, the target RNA transcript comprises ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPCl, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1, and WDTC1.

In another aspect, the disclosure provides a method of identifying a non-productive splice site in a target RNA transcript expressed from a gene that is related to a disease or a disorder of haploinsufficiency, the method comprising: a) incubating a cell with an affinity label to facilitate incorporation of the affinity label into nascent RNA; b) capturing the affinity labeled nascent RNA with a solid support comprising specificity for the affinity label; c) separating the affinity labeled total RNA; d) binding nascent RNA transcript intermediates among the total RNA with one or more affinity labeled probes complementary to the nascent RNA transcript intermediates; e) capturing the nascent RNA transcript intermediates bound to the one or more affinity labeled probes with a solid support comprising specificity for the affinity label; f) isolating the captured nascent RNA transcript intermediates; and g) sequencing the isolated nascent RNA transcript intermediates, thereby identifying non-productive splice sites in the target RNA transcript.

In an embodiment, step a) comprises incubating cells for less than about 30 minutes in media containing the affinity label.

In an embodiment, the affinity label comprises 4-thiouridine, 6-thio-guanosine, 5-ethynyl-uridine, or bromodeoxyuridine. In an embodiment, the 4-thiouridine labeled total RNA is biotinylated to produce biotinylated total RNA.

In an embodiment, the biotinylated total RNA is captured in step b) with a streptavidin linked solid support.

In an embodiment, the bromodeoxyuridine labeled total RNA is captured in step b) with an anti-bromodeoxyuridine antibody.

In one aspect, the disclosure provides a method of identifying a non-productive splice site in a target RNA transcript, the method comprising the steps of: a) incubating a cell with 4-thiouridine to facilitate incorporation of 4-thiouridine into newly generated total RNA; b) biotinylating the 4-thiouridine in the total RNA; c) capturing the biotinylated total RNA with a streptavidin linked solid support; d) separating the biotinylated total RNA; e) binding nascent RNA transcript intermediates among the total RNA with one or more biotinylated probes complementary to the nascent RNA transcript intermediates; e) capturing the nascent RNA transcript intermediates bound to the one or more biotinylated probes with a streptavidin linked solid support; f) isolating the captured nascent RNA transcript intermediates; and g) sequencing the isolated nascent RNA transcript intermediates, thereby identifying non-productive splice sites in the target RNA transcript.

In an embodiment, step a) comprises incubating cells for less than about 30 minutes in media containing 4-thiouridine.

In an embodiment, the cell expresses the target RNA transcript.

In an embodiment, the method further comprises: h) identifying split reads that do not map to annotated target RNA transcript exon-exon junctions; and i) calculating the probability that the split reads represent non-productive transcripts.

In an embodiment, the non-productive transcripts are rapidly degraded. In an embodiment, the non-productive transcripts are not translated into a functional protein.

In one aspect, the disclosure provides a method of identifying a non-productive splice site in an SLC6A1 RNA transcript, the method comprising: a) incubating a cell with an affinity label to facilitate incorporation of the affinity label into newly generated total RNA; b) capturing the affinity labeled total RNA with a solid support comprising specificity for the affinity label; c) separating the affinity labeled total RNA; d) binding nascent SLC6A1 RNA transcript intermediates among the total RNA with one or more affinity labeled probes complementary to the nascent SLC6A1 RNA transcript intermediates; e) capturing the nascent SLC6A1 RNA transcript intermediates bound to the one or more affinity labeled probes with a solid support comprising specificity for the affinity label; f) isolating the captured nascent SLC6A1 RNA transcript intermediates; and g) sequencing the isolated nascent SLC6A1 RNA transcript intermediates, thereby identifying non-productive splice sites in the target RNA transcript.

In an embodiment, step a) comprises incubating cells for less than about 30 minutes in media containing the affinity label.

In an embodiment, the affinity label comprises 4-thiouridine or bromodeoxyuridine. In an embodiment, the 4-thiouridine labeled total RNA is biotinylated to produce biotinylated total RNA.

In an embodiment, the biotinylated total RNA is captured in step b) with a streptavidin linked solid support.

In an embodiment, the bromodeoxyuridine labeled total RNA is captured in step b) with an anti-bromodeoxyuridine antibody.

In an embodiment, step a) comprises incubating cells for less than about 30 minutes in media containing 4-thiouridine.

In an embodiment, the cell expresses the SLC6A1 RNA transcript. In an embodiment, the cell comprises a neuronal cell and/or an astrocyte.

In an embodiment, the method further comprises: h) identifying split reads that do not map to annotated target RNA transcript exon-exon junctions; and i) calculating the probability that the split reads represent non-productive SLC6A1 RNA transcripts.

In an embodiment, the non-productive SLC6A1 RNA transcripts are rapidly degraded. In an embodiment, the non-productive SLC6A1 RNA transcripts are not translated into a functional protein.

In an embodiment, the one or more affinity labeled probes complementary to SLC6A1 RNA matches about ≤ 70% to a second location in the genome, and wherein the one or more affinity labeled probes are located within SLC6A1 exons that are included within at least 50% of annotated SLC6A1 mRNA isoforms.

In an embodiment, the target RNA transcript comprises ATP1A2, CACNA1A, SETD5, SHANK3, NF2, DNMT1, TCF4, RAI1, PEX1, ARSA, EIF2B5, EIF2B1, EIF2B2, NPCl, ADAR, STXBP1, PRICKLE2, PRRT2, MFSD8, IDUA, SLC6A1, or STX1B. In an embodiment, the target RNA transcript comprises SLC6A1.

In an embodiment, the target RNA transcript is associated with a disease or disorder of the CNS. In an embodiment, the disease or the disorder of the CNS comprises myoclonic-atonic epilepsy (MAE), epilepsy, attention deficit hyperactivity disorder (ADHD), familial hemiplegic migraine-2, familial basilar migraine, alternating hemiplegia of childhood, episodic ataxia type 2, familial hemiplegic migraine, Spinocerebellar ataxia type 6 , mental retardation -23, 3p25 microdeletion syndrome, Phelan-McDermid syndrome, shizophrenia-15, Neurofibromatosis (type 1or type 2, Meningioma, NF2-related, schwannomatosis 1, Hereditary sensory neuropathy type IE, autosomal dominant cerebellar ataxia, deafness, and narcolepsy, Pitt-Hopkins syndrome, Smith-Magenis syndrome, peroxisome biogenesis disorder la, Heimler syndrome-1, metachromatic leukodystrophy, leukoencephalopathy with vanishing white matter, Niemann-Pick disease type CI and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.

In one aspect, the disclosure provides a method for preparing an antisense oligonucleotide that binds to a target region in a targert RNA transcript associated with a disease or disorder of haploinsufficiency, wherein the target region comprises a non-productive splice site, and wherein the method is defined in the appended claims..

In an embodiment, the RNA transcript associated with a disease or disorder of haploinsufficiency is selected from the group consisting ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPCl, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1, and WDTC1.

In an embodiment, the RNA transcript associated with a disease or disorder of haploinsufficiency is selected from the group consisting CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1, and WDTC1.

In an embodiment, the RNA transcript associated with a disease or disorder of haploinsufficiency is selected from the group consisting of ADAR, ARSA, ATP1A2, CACNA1A, DNMT1, EIF2B1, EIF2B2, EIF2B5, IDUA, MFSD8, NF2, NPCl, PEX1, PRICKLE2, PRRT2, RAI1, SETD5, SHANK3, SLC6A1, STXBP1, STX1B, and TCF4.

In a facet, binding of the antisense oligonucleotide to the target region increases the expression of a functional protein encoded by the RNA transcript in a cell.

In a facet, the splice modulatory element comprises one or more of a non-productive splice site, a exonic splicing enhancer, an exonic splicing silencer, an intronic splicing enhancer, or an intronic splicing silencer.

In a facet, the antisense oligonucleotide comprises a region of complementarity to a target region of an RNA transcript corresponding to any one of the genomic sequences recited in Table 4.

In a facet, the antisense oligonucleotide comprises a region of complementarity to a target region of an RNA transcript corresponding to any one of the genomic sequences of any one of SEQ ID NOs: 128-300.

In a facet, the antisense oligonucleotide comprises a region of complementarity to a target region of an RNA transcript corresponding to the genomic sequence of SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186, SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219, SEQ ID NO: 220, SEQ ID NO: 221, SEQ ID NO: 222, SEQ ID NO: 223, SEQ ID NO: 224, SEQ ID NO: 225, SEQ ID NO: 226, SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 234, SEQ ID NO: 235, SEQ ID NO: 236, SEQ ID NO: 237, SEQ ID NO: 238, SEQ ID NO: 239, SEQ ID NO: 240, SEQ ID NO: 241, SEQ ID NO: 242, SEQ ID NO: 243, SEQ ID NO: 244, SEQ ID NO: 245, SEQ ID NO: 246, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 260, SEQ ID NO: 261, SEQ ID NO: 262, SEQ ID NO: 263, SEQ ID NO: 264, SEQ ID NO: 265, SEQ ID NO: 266, SEQ ID NO: 267, SEQ ID NO: 268, SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, or SEQ ID NO: 300.

In a facet, the antisense oligonucleotide comprises a region of complementarity to the reverse complement of any one of the genomic sequences recited in Table 4.

In a facet, the antisense oligonucleotide comprises a region of complementarity to the reverse complement of any one of the genomic sequences of any one of SEQ ID NOs: 128-300.

In a facet, the antisense oligonucleotide comprises a region of complementarity to the reverse complement the genomic sequence of SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186, SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219, SEQ ID NO: 220, SEQ ID NO: 221, SEQ ID NO: 222, SEQ ID NO: 223, SEQ ID NO: 224, SEQ ID NO: 225, SEQ ID NO: 226, SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 234, SEQ ID NO: 235, SEQ ID NO: 236, SEQ ID NO: 237, SEQ ID NO: 238, SEQ ID NO: 239, SEQ ID NO: 240, SEQ ID NO: 241, SEQ ID NO: 242, SEQ ID NO: 243, SEQ ID NO: 244, SEQ ID NO: 245, SEQ ID NO: 246, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 260, SEQ ID NO: 261, SEQ ID NO: 262, SEQ ID NO: 263, SEQ ID NO: 264, SEQ ID NO: 265, SEQ ID NO: 266, SEQ ID NO: 267, SEQ ID NO: 268, SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, or SEQ ID NO: 300.

In a facet, the antisense oligonucleotide comprises 8 to 80 nucleotides in length. In a facet, the antisense oligonucleotide comprises 15 to 25 nucleotides in length. In a facet, the antisense oligonucleotide comprises 15 nucleotides in length. In a facet, the antisense oligonucleotide comprises 16 nucleotides in length. In a facet, the antisense oligonucleotide comprises 17 nucleotides in length. In a facet, the antisense oligonucleotide comprises 18 nucleotides in length. In a facet, the antisense oligonucleotide comprises 19 nucleotides in length. In a facet, the antisense oligonucleotide comprises 20 nucleotides in length. In a facet, the antisense oligonucleotide comprises 21 nucleotides in length. In a facet, the antisense oligonucleotide comprises 22 nucleotides in length. In a facet, the antisense oligonucleotide comprises 23 nucleotides in length. In a facet, the antisense oligonucleotide comprises 24 nucleotides in length. In a facet, the antisense oligonucleotide comprises 25 nucleotides in length. In a facet, the antisense oligonucleotide comprises 18 to 20 nucleotides in length.

In a facet, the antisense oligonucleotide comprises one or more modified nucleotides.

In a facet, the one or more modified nucleotides comprise a modification of a ribose group, a phosphate group, a nucleobase, or a combination thereof.

In a facet, the modification of the ribose group comprises 2'-*O*-methyl, 2'-fluoro, 2'-deoxy, 2'-*O*-(2-methoxyethyl) (MOE), 2'-O-alkyl, 2'-O-alkoxy, 2'-O-alkylamino, 2'-NH₂, a constrained nucleotide, or a combination thereof. In an embodiment, the constrained nucleotide comprises a locked nucleic acid (LNA), an ethyl-constrained nucleotide, a 2'-(*S*)-constrained ethyl (S-cEt) nucleotide, a constrained MOE, a 2'-*O*,4'-C-aminomethylene bridged nucleic acid (2',4'-BNA^{NC}), an alpha-L-locked nucleic acid, a tricyclo-DNA, or a combination thereof.

In a facet, the modification of the ribose group comprises 2'-*O*-(2-methoxyethyl) (MOE).

In a facet, the modification of the phosphate group comprises a phosphorothioate, a phosphonoacetate (PACE), a thiophosphonoacetate (thioPACE), an amide, a triazole, a phosphonate, a phosphotriester modification, or a combination thereof.

In a facet, the modification of the phosphate group comprises phosphorothioate.

In a facet, the modification of the nucleobase group comprises 2-thiouridine, 4-thiouridine, N⁶-methyladenosine, pseudouridine, 2,6-diaminopurine, inosine, thymidine, 5-methylcytosine, 5-substituted pyrimidine, isoguanine, isocytosine, halogenated aromatic groups, or a combination thereof.

In a facet, the modification of the nucleobase group comprises 5-methylcytosine.

In a facet, the antisense oligonucleotide further comprises a ligand.

In a facet, the antisense oligonucleotide comprises a sequence modification pattern of X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}, X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}, or X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}, wherein: s represents a phosphorothioate internucleoside linkage; and X represents an adenosine, a guanosine, a cytidine, or a thymine comprising a 2'-*O*-(2-methoxyethyl) modification.

In one facet, the teaching provides a method of treating a disease or disorder characterized by haploinsufficiency of a target gene, comprising administering to a subject in need thereof the antisense oligonucleotide recited above, and treating the disease or disorder.

In one facet, the teaching provides a method of increasing expression of a target functional RNA transcript in a cell, the method comprising contacting the cell with the antisense oligonucleotide recited above, thereby increasing the expression of the functional RNA transcript in a cell.

In a facet, expression is increased by about 20%, about 30%, about 40%, about 50%, about 60%, about 70%, about 80%, about 90%, about 100%, or more, relative to a cell that is not contacted with the antisense oligonucleotide.

In one facet, the teaching provides a method of increasing expression of a protein encoded by a RNA transcript associated with a disease of haploinsufficiency in a cell, the method comprising contacting a cell with the antisense oligonucleotide recited above, thereby increasing expression of the protein.

In a facet, expression is increased by about 20%, about 30%, about 40%, about 50%, about 60%, about 70%, about 80%, about 90%, about 100%, or more, relative to a cell that is not contacted with the antisense oligonucleotide.

In one facet, the teaching provides an antisense oligonucleotide comprising a region of complementarity to a target region of an RNA transcript corresponding to any one of the genomic sequences recited in Table 4, wherein the antisense oligonucleotide inhibits cryptic splicing at the target region and activates gene expression.

In one facet, the teaching provides an antisense oligonucleotide comprising a region of complementarity to a target region of an RNA transcript corresponding to any one of the genomic sequences of any one of SEQ ID NOs: 128-300, wherein the antisense oligonucleotide inhibits cryptic splicing at the target region and activates gene expression.

In one facet, the teaching provides an antisense oligonucleotide comprising a region of complementarity to a target region of an RNA transcript corresponding to the genomic sequence of SEQ ID NO: 128, SEQ ID NO: 129, SEQ ID NO: 130, SEQ ID NO: 131, SEQ ID NO: 132, SEQ ID NO: 133, SEQ ID NO: 134, SEQ ID NO: 135, SEQ ID NO: 136, SEQ ID NO: 137, SEQ ID NO: 138, SEQ ID NO: 139, SEQ ID NO: 140, SEQ ID NO: 141, SEQ ID NO: 142, SEQ ID NO: 143, SEQ ID NO: 144, SEQ ID NO: 145, SEQ ID NO: 146, SEQ ID NO: 147, SEQ ID NO: 148, SEQ ID NO: 149, SEQ ID NO: 150, SEQ ID NO: 151, SEQ ID NO: 152, SEQ ID NO: 153, SEQ ID NO: 154, SEQ ID NO: 155, SEQ ID NO: 156, SEQ ID NO: 157, SEQ ID NO: 158, SEQ ID NO: 159, SEQ ID NO: 160, SEQ ID NO: 161, SEQ ID NO: 162, SEQ ID NO: 163, SEQ ID NO: 164, SEQ ID NO: 165, SEQ ID NO: 166, SEQ ID NO: 167, SEQ ID NO: 168, SEQ ID NO: 169, SEQ ID NO: 170, SEQ ID NO: 171, SEQ ID NO: 172, SEQ ID NO: 173, SEQ ID NO: 174, SEQ ID NO: 175, SEQ ID NO: 176, SEQ ID NO: 177, SEQ ID NO: 178, SEQ ID NO: 179, SEQ ID NO: 180, SEQ ID NO: 181, SEQ ID NO: 182, SEQ ID NO: 183, SEQ ID NO: 184, SEQ ID NO: 185, SEQ ID NO: 186, SEQ ID NO: 187, SEQ ID NO: 188, SEQ ID NO: 189, SEQ ID NO: 190, SEQ ID NO: 191, SEQ ID NO: 192, SEQ ID NO: 193, SEQ ID NO: 194, SEQ ID NO: 195, SEQ ID NO: 196, SEQ ID NO: 197, SEQ ID NO: 198, SEQ ID NO: 199, SEQ ID NO: 200, SEQ ID NO: 201, SEQ ID NO: 202, SEQ ID NO: 203, SEQ ID NO: 204, SEQ ID NO: 205, SEQ ID NO: 206, SEQ ID NO: 207, SEQ ID NO: 208, SEQ ID NO: 209, SEQ ID NO: 210, SEQ ID NO: 211, SEQ ID NO: 212, SEQ ID NO: 213, SEQ ID NO: 214, SEQ ID NO: 215, SEQ ID NO: 216, SEQ ID NO: 217, SEQ ID NO: 218, SEQ ID NO: 219, SEQ ID NO: 220, SEQ ID NO: 221, SEQ ID NO: 222, SEQ ID NO: 223, SEQ ID NO: 224, SEQ ID NO: 225, SEQ ID NO: 226, SEQ ID NO: 227, SEQ ID NO: 228, SEQ ID NO: 229, SEQ ID NO: 230, SEQ ID NO: 231, SEQ ID NO: 232, SEQ ID NO: 233, SEQ ID NO: 234, SEQ ID NO: 235, SEQ ID NO: 236, SEQ ID NO: 237, SEQ ID NO: 238, SEQ ID NO: 239, SEQ ID NO: 240, SEQ ID NO: 241, SEQ ID NO: 242, SEQ ID NO: 243, SEQ ID NO: 244, SEQ ID NO: 245, SEQ ID NO: 246, SEQ ID NO: 247, SEQ ID NO: 248, SEQ ID NO: 249, SEQ ID NO: 250, SEQ ID NO: 251, SEQ ID NO: 252, SEQ ID NO: 253, SEQ ID NO: 254, SEQ ID NO: 255, SEQ ID NO: 256, SEQ ID NO: 257, SEQ ID NO: 258, SEQ ID NO: 259, SEQ ID NO: 260, SEQ ID NO: 261, SEQ ID NO: 262, SEQ ID NO: 263, SEQ ID NO: 264, SEQ ID NO: 265, SEQ ID NO: 266, SEQ ID NO: 267, SEQ ID NO: 268, SEQ ID NO: 269, SEQ ID NO: 270, SEQ ID NO: 271, SEQ ID NO: 272, SEQ ID NO: 273, SEQ ID NO: 274, SEQ ID NO: 275, SEQ ID NO: 276, SEQ ID NO: 277, SEQ ID NO: 278, SEQ ID NO: 279, SEQ ID NO: 280, SEQ ID NO: 281, SEQ ID NO: 282, SEQ ID NO: 283, SEQ ID NO: 284, SEQ ID NO: 285, SEQ ID NO: 286, SEQ ID NO: 287, SEQ ID NO: 288, SEQ ID NO: 289, SEQ ID NO: 290, SEQ ID NO: 291, SEQ ID NO: 292, SEQ ID NO: 293, SEQ ID NO: 294, SEQ ID NO: 295, SEQ ID NO: 296, SEQ ID NO: 297, SEQ ID NO: 298, SEQ ID NO: 299, or SEQ ID NO: 300, wherein the antisense oligonucleotide inhibits cryptic splicing at the target region and activates gene expression.

### BRIEF DESCRIPTION OF THE DRAWINGS

The features and advantages of the present invention will be more fully understood from the following detailed description of illustrative embodiments taken in conjunction with the accompanying drawings.
***Fig. 1*** depicts qPCR-based expression of SLC6A1 in human SH-SY5Y cells.
***Fig. 2*** depicts the schematic of the *SLC6A1* genetic locus on human chromosome 3. Structures for the 37 most comprehensive annotated isoforms are shown (bottom), with common exons (i.e. represented in at least 50% of isoforms) highlighted in black. Computationally predicted cryptic splice sites (5' splice sites and 3' splice sites) are indicated by dashes at the top.
***Fig. 3*** depicts a bar graph of *SLC6A1* relative mRNA levels in KNS60 neuroblastoma cells transfected with various steric blocking antisense oligonucleotides at 50 nM.
***Fig. 4*** depicts a schematic of the distribution of annotated and unannotated 5' and 3' splice sites across cryptic junction sites, which are defined as either unannotated or using non-canonical dinucleotide sequences.
***Fig. 5*** depicts a schematic of the number of reads for individual cryptic junction sites in metabolic labeling sequencing data (4sU-seq, x-axis) relative to non-metabolic labeling data (polyA mRNA or mRNA from UPF1 knockdown, y-axis). Solid grey lines represent a linear fit to the data, while dotted lines represent a correlation of 1.

### DETAILED DESCRIPTION

The present disclosure provides methods of identifying non-productive RNA transcript intermediates.

It is to be understood that the methods described in this disclosure are not limited to particular methods and experimental conditions disclosed herein as such methods and conditions may vary. It is also to be understood that the terminology used herein is for the purpose of describing particular embodiments only, and is not intended to be limiting.

Unless otherwise defined herein, scientific and technical terms used herein have the meanings that are commonly understood by those of ordinary skill in the art. In the event of any latent ambiguity, definitions provided herein take precedent over any dictionary or extrinsic definition. Unless otherwise required by context, singular terms shall include pluralities and plural terms shall include the singular. The use of "or" means "and/or" unless stated otherwise. The use of the term "including" as well as other forms, such as "includes" and "included," is not limiting.

Generally, nomenclatures used in connection with cell and tissue culture, molecular biology, immunology, microbiology, genetics and protein and nucleic acid chemistry and hybridization described herein are those well-known and commonly used in the art. The nomenclatures used in connection with, and the laboratory procedures and techniques of, analytical chemistry, synthetic organic chemistry, and medicinal and pharmaceutical chemistry described herein are those well-known and commonly used in the art.

So that the invention may be more readily understood, certain terms are first defined.

The term "nucleoside" refers to a molecule having a purine or pyrimidine base covalently linked to a ribose or deoxyribose sugar. Exemplary nucleosides include adenosine, guanosine, cytidine, uridine and thymidine. Additional exemplary nucleosides include inosine, 1-methyl inosine, pseudouridine, 5,6-dihydrouridine, ribothymidine, 2N-methylguanosine and 2,2N,N-dimethylguanosine (also referred to as "rare" nucleosides). The term "nucleotide" refers to a nucleoside having one or more phosphate groups joined in ester linkages to the sugar moiety. Exemplary nucleotides include nucleoside monophosphates, diphosphates and triphosphates. The terms "polynucleotide" and "nucleic acid molecule" are used interchangeably herein and refer to a polymer of nucleotides joined together by a phosphodiester or phosphorothioate linkage between 5' and 3' carbon atoms.

The term "RNA" or "RNA molecule" or "ribonucleic acid molecule" refers to a polymer of ribonucleotides (e.g., 2, 3, 4, 5, 10, 15, 20, 25, 30, or more ribonucleotides). An RNA nucleotide refers to a single ribonucleotide. The term "DNA" or "DNA molecule" or "deoxyribonucleic acid molecule" refers to a polymer of deoxyribonucleotides. A DNA nucleotide refers to a single deoxyribonucleotide. As used herein, the term "DNA-like" refers to a conformation of, e.g. a modified nucleoside or nucleotide which is similar to the conformation of a corresponding unmodified DNA unit. For example, a DNA-like nucleotide may refer to a conformation of a modified deoxyribonucleotide similar to a corresponding unmodified deoxyribonucleotide. Examples of DNA-like nucleotides include, without limitation, e.g., 2'-deoxyribonucleotides, 2'-deoxy-2'-substituted arabinonucleotides (e.g., 2'-deoxy-2'-fluoroarabinonucleotides, also known in the art as 2'F-ANA or FANA), and corresponding phosphorothioate analogs. As used herein, the term "RNA-like" refers to a conformation of, e.g. a modified nucleoside or nucleotide which is similar to the conformation of a corresponding unmodified RNA unit. RNA-like conformations may adopt an A-form helix while DNA-like conformations adopt a B-form helix. Examples RNA-like nucleotides include, without limitation, e.g., 2'-substituted-RNA nucleotides (e.g., 2'-fluoro-RNA nucleotides also known in the art as 2'F-RNA), locked nucleic acid (LNA) nucleotides (also known in the art as bridged nucleic acids or bicyclic nucleotides), 2'-fluoro-4'-thioarabinonucleotide (also known in the art as 4'S-FANA nucleotides), 2'-O-alkyl-RNA, and corresponding phosphorothioate analogs.

DNA and RNA can be synthesized naturally (e.g., by DNA replication or transcription of DNA, respectively). RNA can be post-transcriptionally modified. DNA and RNA can also be chemically synthesized. DNA and RNA can be single-stranded (i.e., ssRNA and ssDNA, respectively) or multi-stranded (e.g., double stranded, i.e., dsRNA and dsDNA, respectively). "mRNA" or "messenger RNA" is single-stranded RNA that specifies the amino acid sequence of one or more polypeptide chains. This information is translated during protein synthesis when ribosomes bind to the mRNA.

As used herein, the term "small interfering RNA" ("siRNA") (also referred to in the art as "short interfering RNAs") refers to an RNA (or RNA analog) comprising between about 10-50 nucleotides (or nucleotide analogs) which is capable of directing or mediating RNA interference. In certain embodiments, a siRNA comprises between about 15-30 nucleotides or nucleotide analogs, or between about 16-25 nucleotides (or nucleotide analogs), or between about 18-23 nucleotides (or nucleotide analogs), or between about 19-22 nucleotides (or nucleotide analogs) (e.g., 19, 20, 21 or 22 nucleotides or nucleotide analogs). The term "short" siRNA refers to a siRNA comprising about 21 nucleotides (or nucleotide analogs), for example, 19, 20, 21 or 22 nucleotides. The term "long" siRNA refers to a siRNA comprising about 24-25 nucleotides, for example, 23, 24, 25 or 26 nucleotides. Short siRNAs may, in some instances, include fewer than 19 nucleotides, e.g., 16, 17 or 18 nucleotides, provided that the shorter siRNA retains the ability to mediate RNAi. Likewise, long siRNAs may, in some instances, include more than 26 nucleotides, provided that the longer siRNA retains the ability to mediate RNAi absent further processing, e.g., enzymatic processing, to a short siRNA.

The term "nucleotide analog" or "altered nucleotide" or "modified nucleotide" refers to a non-standard nucleotide, including non-naturally occurring ribonucleotides or deoxyribonucleotides. Exemplary modified nucleotides are modified at any position so as to alter certain chemical properties of the nucleotide yet retain the ability of the modified nucleotide to perform its intended function. Examples of positions of the nucleotide which may be derivatized include the 5 position, e.g., 5-(2-amino)propyl uridine, 5-bromo uridine, 5-propyne uridine, 5-propenyl uridine, etc.; the 6 position, e.g., 6-(2-amino)propyl uridine; the 8-position for adenosine and/or guanosines, e.g., 8-bromo guanosine, 8-chloro guanosine, 8-fluoroguanosine, etc. Modified nucleotides also include deaza nucleotides, e.g., 7-deaza-adenosine; O- and N-modified (e.g., alkylated, e.g., N6-methyl adenosine, or as otherwise known in the art) nucleotides; and other heterocyclically modified nucleotides such as those described in Herdewijn, Antisense Nucleic Acid Drug Dev., 2000 Aug. 10(4):297-310.

Modified nucleotides may also comprise modifications to the sugar portion of the nucleotides. For example, the 2' OH-group may be replaced by a group selected from H, OR, R, F, Cl, Br, I, SH, SR, NH₂, NHR, NR₂, COOR, or OR, wherein R is substituted or unsubstituted C₁-C₆ alkyl, alkenyl, alkynyl, aryl, etc. For another example, the ribose sugar may be replaced with a bicyclic or tricylic moiety, such as in Locked Nucleic Acid, constrained ethyl, tricycloDNA, or other bridged or bicyclic modifications. Other possible modifications include those described in U.S. Pat. Nos. 5,858,988, and 6,291,438.

The phosphate group of the nucleotide may also be modified, e.g., by substituting one or more of the oxygens of the phosphate group with sulfur (e.g., phosphorothioates), or by making other substitutions which allow the nucleotide to perform its intended function such as described in, for example, Eckstein, Antisense Nucleic Acid Drug Dev. 2000 Apr. 10(2):117-21, Rusckowski et al. Antisense Nucleic Acid Drug Dev. 2000 Oct. 10(5):333-45, Stein, Antisense Nucleic Acid Drug Dev. 2001 Oct. 11(5): 317-25, Vorobjev et al. Antisense Nucleic Acid Drug Dev. 2001 Apr. 11(2):77-85, and U.S. Pat. No. 5,684,143. Certain of the above-referenced modifications (e.g., phosphate group modifications) decrease the rate of hydrolysis of, for example, polynucleotides comprising said analogs *in vivo* or *in vitro.*

As used herein, the terms "unmodified nucleotide" or "non-modified nucleotide" refers to a nucleotide composed of naturally occurring nucleobases, sugar moieties, and internucleoside linkages. In some embodiments, a non-modified nucleotide is an RNA nucleotide (i.e. β-D-ribonucleoside) or a DNA nucleotide (i.e. β-D-deoxyribonucleoside).

The term "oligonucleotide" refers to a short polymer of nucleotides and/or modified nucleotides. As discussed above, the oligonucleotides may be linked with linkages which result in a lower rate of hydrolysis as compared to an oligonucleotide linked with phosphodiester linkages. For example, the nucleotides of the oligonucleotide may comprise triazole, amide, carbamate, methylenediol, ethylene diol, oxymethylthio, oxyethylthio, oxycarbonyloxy, phosphorodiamidate, phosphoroamidate, phosphonate, and/or phosphorothioate linkages. Alterations or modifications of the oligonucleotide can further include addition of non-nucleotide material, such as to the end(s) of the oligonucleotide or internally (at one or more nucleotides of the oligonucleotide).

The term "antisense" refers generally to any approach reliant upon agents, e.g., single-stranded oligonucleotides, that are sufficiently complementary to a target sequence to associate with the target sequence in a sequence-specific manner (e.g., hybridize to the target sequence). Exemplary uses of antisense in the instant application involve use of an oligoribonucleotide agent that hybridizes to a target pre-mRNA molecule and blocks an activity/effect (e.g., splicing pattern and/or blocking of non-productive splice sites) of the targeted pre-mRNA sequence, but antisense approaches commonly are used to target DNA or RNA for transcriptional inhibition, translational inhibition, degradation, etc. Antisense is a technology that can be initiated by the hand of man, for example, to modulate splicing and/or silence the expression of target genes.

As used herein, the term "antisense oligonucleotide" refers to a nucleic acid (e.g., an RNA or analog thereof), having sufficient sequence complementarity to a target RNA (i.e., the RNA for which splice site selection is modulated) to block a region of a target RNA (e.g., pre-mRNA) in an effective manner. Blocking of non-productive splice sites in SLC6A1 pre-mRNA may serve to modulate splicing, either by masking a binding site for a native protein that would otherwise modulate splicing and/or by altering the structure of the targeted RNA. The target RNA may be a target pre-mRNA (e.g., SLC6A1 pre-mRNA).

An antisense oligonucleotide having a "sequence sufficiently complementary to a target RNA sequence to modulate splicing of the target RNA" means that the antisense agent has a sequence sufficient to trigger the masking of a binding site for a native protein that would otherwise modulate splicing and/or alters the three-dimensional structure of the targeted RNA. Likewise, an oligonucleotide reagent having a "sequence sufficiently complementary to a target RNA sequence to modulate splicing of the target RNA" means that the oligonucleotide reagent has a sequence sufficient to trigger the masking of a binding site for a native protein that would otherwise modulate splicing and/or alters the three-dimensional structure of the targeted RNAs used herein.

As used herein, the term "intron" is a segment of DNA that is transcribed but removed from an RNA transcript by being spliced together with a sequence (exon) on either side of it. Introns are considered to be intervening sequences within the protein coding region of a gene and generally do not contain information represented in the protein produced from the gene. The term "exon" encompasses any segment of a gene that contains intervening sequences represented in the mature RNA product. An exon comprises information within a gene that is translated into a protein.

The term "nascent RNA intermediate" or "primary transcript RNA" refers to a newly synthesized RNA molecule that has not yet fully undergone posttranscriptional processing. It encompasses the first RNA product resulting from transcription of a gene by RNA polymerase. The RNA, termed nascent RNA, contains both intron and exon sequences and is therefore not processed by the cellular splicing machinery or only partially processed by the cellular machinery. The nascent RNA includes, but is not limited to, non-productively spliced RNA.

The term "posttranscriptional processing", as used herein, refers to the modifications made to nascent RNA molecules (or pre-mRNAs) before the nascent RNA molecules exit the nucleus of a cell. Such modifications include, for example, capping of the 5' end of nascent RNA (typically with a 7-methylguanosine linked to the first nucleotide via a 5 '-5 ' triphosphate bridge), polyadenylation of the 3 'end of the nascent RNA, or removal of introns via splicing.

The term "splicing" encompasses cellular events that occur in the nuclei of eukaryotic cells where introns are removed from the pre-mRNA species. In general, the process requires the formation of a spliceosome complex in which a 5 ' splice donor site is brought into proximity with a 3' splice acceptor site and an intervening intron sequence is removed from the transcript.

The term "noisy splicing", "cryptic splicing", or "non-productive splicing" refers to the event when the spliceosome utilizes erroneous splice sites and generates RNA transcripts that then undergo nonsense-mediated RNA decay. In cryptic or nonproductive splicing, spliceosome components, comprising enzymes that are involved in the RNA splicing process, can often bind to cryptic sites (with either canonical or non-canonical sequence elements) and improperly splice an RNA molecule. In the instance of an RNA transcript that contains at least one inefficiently spliced intron, i.e. the result of noisy or non-productive splicing, the RNA may be maintained in the nucleus, and if it is exported to the cytoplasm it is not translated into protein but is degraded. The phenomenon of nonproductive splicing is particularly common in genes with long introns or many introns.

As used herein, the term "increasing protein production" or "increasing expression of a target protein" means enhancing the amount of protein (e.g., a target protein) that is translated from an RNA molecule in a cell. A "target protein" may be any protein for which increased expression/production is desired. In some embodiments, the target protein is a disease-associated protein, such a GABA transporter protein encoded by SLC6A1. For example, contacting a cell with an antisense oligonucleotide that is complementary to a region in the nascent RNA molecular that contains a non-productive splicing site would results in a measurable increase in the amount of the protein (e.g., a target protein) encoded by the RNA. Methods of measuring or detecting production of a protein include, for example, Western blotting, flow cytometry, immunofluorescence microscopy, and ELISA.

As used herein, the term "metabolic labelling" refers to the incorporation of a biomolecule into a macromolecule. Metabolic labeling can be accomplished by contacting cells in medium that is supplemented with a metabolic label. For example, a metabolic label can be a label that is incorporated into newly synthesized RNA molecules, or nascent RNA. In one embodiment, the metabolic label is 4-thiouridine (4sU), which is a thiol-containing nucleoside that can be introduced into nucleoside salvage pathways in eukaryotic cells and allow non-disruptive labeling of newly transcribed RNA. Other examples of metabolic labels 6-thio-guanosine (6sG), 5-ethynyl-uridine (5eU), or bromodeoxyuridine (BdU). 4sU and 6sG can be biotinylated, 5eU can be modified by click chemistry, and BdU can be associated with antibodies for the purpose of capture/isolation of the RNA.

As used herein, the term "affinity label" refers to a group, moiety, or entity that specifically interacts/associates with a counterpart entity (e.g., capture agent). The affinity label/capture agent pair is often referred to as an "affinity pair". The affinity pair can be a biochemical pair. Examples of biochemical pairs include antibody-antigen, enzyme-inhibitor, biotin-streptavidin, hormone-receptor, sugar-lectin and complementary nucleic acid components. The biochemical interaction between members of the affinity pair can be non-covalent or covalent in nature. Examples of non-covalent interactions are those that involve hydrophobic, hydrophilic, or Van der Waals interactions between members of an affinity pair. Examples of covalent interactions involve the formation of a peptide bond or a disulfide bond between members of an affinity pair. In an exemplary embodiment, the members of the affinity pair are 4sU and EZ-Link^{™} Biotin-HPDP. Biotin-HPDP (N-[6-(biotinamido)hexyl]-3'-(2'-pyridyldithio)propionamide), is a sulfhydryl-reactive biotinylation agent. The pyridyl disulfide group at the end of biotin-HPDP reacts with free thiol groups on proteins and other molecules such as 4sU in 4sU-labeled RNA. The interaction between biotin-HPDP and 4sU leads to the formation of a covalent disulfide bond. The long spacer arm of biotin-HPDP enables the biotin part in biotin-HPDP to undergo further pairing with avidin or streptavidin. The covalent disulfide bond between 4sU and biotin-HPDP can be broken by the addition of reducing agents, such as dithiothreitol (DTT), or beta-mercapto ethanol. The term "target gene" or "target RNA transcript" is a gene or transcript (e.g., a pre-mRNA) whose expression is to be substantially modulated. This modulation can be achieved by steric blocking of a non-productive or cryptic splice site.

The term "non-target gene" is a gene whose expression is not to be substantially modulated. For example, a target gene of the present invention is SLC6A1, and a non-target gene of the present invention is a gene that is not SLC6A1. In one facet, the polynucleotide sequences of the target and non-target gene (e.g., mRNA encoded by the target and non-target genes) can differ by one or more nucleotides. In another facet, the target and non-target genes can differ by one or more polymorphisms (e.g., Single Nucleotide Polymorphisms or SNPs). In another facet, the target and non-target genes can share less than 100% sequence identity. In another facet, the non-target gene may be a homologue (e.g., an orthologue or paralogue) of the target gene.

The term "antisense activity" means any detectable or measurable activity attributable to the hybridization of an antisense compound to its target nucleic acid. In some embodiments, antisense activity is an increase in the amount or expression of a target nucleic acid or protein encoded by such target nucleic acid.

The term "target-recognition sequence" refers to the portion of an antisense compound that recognizes a target nucleic acid. The target-recognition sequence has a nucleobase sequence that permits hybridization to a corresponding region or segment of a target nucleic acid.

The term "conserved region" refers to a portion, or portions, of a nucleic acid sequence that is conserved, i.e. a portion, or portions of the nucleic acid sequence having a similar or identical sequence across species. A conserved region may be computationally identified, e.g., using any sequence alignment software available in the art.

As used herein, the term "sufficiently complementary" means that antisense oligonucleotide has a sequence (e.g., an antisense oligonucleotide having a target-recognition sequence) which is sufficient to bind the desired target transcript (e.g., a SLC6A1 transcript), and to trigger the inhibition of non-productive splicing of the target transcript (e.g., steric inhibition of splicing machinery of the target pre-mRNA). For example, a target-recognition sequence with at least 90% complementarity to a target nucleic acid sequence (e.g., a portion of a SLC6A1 transcript) may be sufficiently complementary to trigger modulation of the SLC6A1 transcript. The term "perfectly complementary" refers to, e.g., a target-recognition sequence with 100% complementarity to a target nucleic acid sequence. Complementary nucleic acid molecules hybridize to each other. The term "hybridization" means the annealing of complementary nucleic acid molecules. In certain embodiments, complementary nucleic acid molecules include an antisense compound and a target nucleic acid.

The term "about" or "approximately" means within 20%, such as within 10%, within 5%, or within 1% or less of a given value or range.

As generally used, "administer" or "administration" refers to the act of injecting or otherwise physically delivering a substance as it exists outside the body (e.g., an antisense compound provided herein) into a patient. Antisense oligonucleotides may be administered to the central nervous system of a patient. The central nervous system includes the brain and spinal cord. Administration methods to the central nervous system include, but not limited to, intrathecal, intraventricular or intrastriatal infusion or delivery and/or any other method of physical delivery or known in the art. Intraventricular infusion may comprise administration using an Ommaya reservoir.

When a disease, or a symptom thereof, is being managed or treated, administration of the substance typically occurs after the onset of the disease or symptoms thereof. When a disease, or symptom thereof, is being prevented, administration of the substance typically occurs before the onset of the disease or symptoms thereof and may be continued chronically to defer or reduce the appearance or magnitude of disease-associated symptoms, e.g., damage to the involved tissues and airways.

As generally used, the term "composition" is intended to encompass a product containing the specified ingredients (e.g., an antisense compound provided herein) in, optionally, the specified amounts.

"Effective amount" means the amount of active pharmaceutical agent (e.g., an antisense compound of the present disclosure) sufficient to effectuate a desired physiological outcome in an individual in need of the agent. The effective amount may vary among individuals depending on the health and physical condition of the individual to be treated, the taxonomic group of the individuals to be treated, the formulation of the composition, assessment of the individual's medical condition, and other relevant factors.

Generally , the terms "subject" and "patient" are used interchangeably. Generally , a subject can be a mammal, such as a non-primate (e.g., cows, pigs, horses, cats, dogs, rats, etc.) or a primate (e.g., monkey and human). Generally, the term "subject", refers to a vertebrate, such as a mammal. Mammals include, without limitation, humans, non-human primates, wild animals, feral animals, farm animals, sports animals, and pets. Generally, a subject may be a mammal, such as a human, having a disease of haploinsufficiency (e.g., myoclonic-atonic epilepsy (MAE)). Generally, a subject may be a mammal, such as a human, that is at risk for developing a disease of haploinsufficiency.

Generally, the term "therapy" refers to any protocol, method and/or agent that can be used in the prevention, management, treatment and/or amelioration of a disease or a symptom related thereto, such as a disease of haploinsufficiency (e.g., myoclonic-atonic epilepsy (MAE)). The term "therapy" may refer to any protocol, method and/or agent that can be used in the modulation of an immune response to an infection in a subject or a symptom related thereto. The terms "therapies" and "therapy" may refer to a biological therapy, supportive therapy, and/or other therapies useful in the prevention, management, treatment and/or amelioration of a disease or a symptom related thereto, such as a disease of haploinsufficiency . The terms "therapies" and "therapy" may refer to a biological therapy, supportive therapy, and/or other therapies useful in the modulation of an immune response to an infection in a subject or a symptom related thereto.

Generally, the terms "treat", "treatment" and "treating" refer to the reduction or amelioration of the progression, severity, and/or duration of a disease or a symptom related thereto, such as a SLC6A1-related disorder, resulting from the administration of one or more therapies (including, but not limited to, the administration of one or more prophylactic or therapeutic agents). The term "treating", as used herein, can also refer to altering the disease course of the subject being treated. Therapeutic effects of treatment include, without limitation, preventing occurrence or recurrence of disease, alleviation of symptom(s), diminishment of direct or indirect pathological consequences of the disease, decreasing the rate of disease progression, amelioration or palliation of the disease state, and remission or improved prognosis.

Generally, a "splice modulatory element" is a nucleic acid region in a target RNA transcript (e.g., a SLC6A1 transcript), which either enhances or silences the splicing of introns in the pre-mRNA, or in general regulates the constitutive or alternative splicing of the pre-mRNA. Examples of splice modulatory elements include, but are not limited to, non-productive splice sites, exonic splicing enhancers, exonic splicing silencers, intronic splicing enhancers, and intronic splicing silencers.

As used herein, a "non-productive splice site" or "cryptic splice site" is splice site in a pre-mRNA that is used by the cellular splicing machinery that leads to the inappropriate inclusion and/or exclusion of introns and/or exons, thereby producing a non-functional transcript. The non-functional transcript may be rapidly degraded in the cell via one or more mechanisms, such as nonsense-mediated decay (NMD). The non-functional transcript may be translated into a non-functional or deleterious protein.

As used herein, a "functional RNA transcript" is an RNA transcript that is translated into a functional protein encoded by said functional RNA transcript (e.g., functional SLC6A1 RNA transcript encoding the GABA Transporter 1, GAT-1, protein).

As used herein, "enriching" for or the "enrichment" of a target RNA transcript is a process of either isolating a target RNA transcript from a heterogeneous population of RNA transcripts, or amplifying the number of target RNA transcript molecules in a heterogeneous population of RNA transcripts. With respect to isolation, the target RNA transcript need not be completely purified away from the heterogeneous population of RNA transcripts. The purpose of enrichment is to enhance the sensitivity of a downstream sequencing step, improving the signal-to-noise ratio, to improve the identification of nascent RNA transcript intermediates, and thereby facilitating the identification of non-productive splice sites in the target RNA transcript.

An enrichment step may comprise a pulldown step, by using one or more nucleic acid probes that are complementary to the target RNA transcript. Alternatively, or in combination, an enrichment step may comprise using nucleic acid primers complementary to the target RNA transcript for selective reverse transcription (i.e., reverse transcribing the target RNA transcript to which the nucleic acid primers bind). The reverse transcribed DNA can be further amplified by PCR.

### Non-Productive Splice Site Identification

The present disclosure provides methods of identifying non-productive splice sites in target RNA transcripts expressed from a gene that is related to a disease or a disorder of haploinsufficiency (e.g., a target pre-mRNA). Cryptic or non-productive splicing occurs when the spliceosome utilizes erroneous splice sites and generates transcripts that then undergo nonsense-mediated mRNA decay (NMD) or an alternative mechanism of rapid mRNA degradation. This can occur in genes with long introns or many introns. While these isoforms are rarely observed in steady-state gene expression measurements, they are likely to represent a large amount of the total transcriptional output of a gene. Without wishing to be bound by theory, blocking non-productive splice sites can lead to an increase in productive mRNA levels as there are fewer molecular resources being wasted on the generation of non-productive transcripts. Due to the rapid degradation of these non-productive mRNA (generated from the use of non-productive splice sites in pre-mRNA), it is challenging to detect and characterize non-productive mRNA.

In one aspect, the disclosure provides a method of identifying non-productive splice sites in a target RNA transcript expressed from a gene that is related to a disease or a disorder of haploinsufficiency, the method comprising:
a) incubating a cell with an affinity label to facilitate incorporation of the affinity label into nascent RNA;
b) capturing the affinity labeled nascent RNA with a solid support comprising specificity for the affinity label;
c) isolating the affinity labeled nascent RNA;
d)sequencing the isolated affinity labeled nascent RNA or cDNA prepared therefrom ;
e) identifying split reads that do not map to exon-exon junctions of the target RNA transcript; and
f) calculating the probability that the split reads represent non-productive transcripts, thereby identifying non-productive splice sites in the target RNA transcript.

In certain facets, step a) comprises incubating the cells for less than about 30 minutes in media containing the affinity label. In certain facets, step a) comprises incubating cells for about 30 seconds, about 1 minute, about 2 minutes, about 3 minutes, about 4 minutes, about 5 minutes, about 7.5 minutes, about 10 minutes, about 12.5 minutes, about 15 minutes, about 17.5 minutes, about 20 minutes, about 22.5 minutes, about 25 minutes, about 27.5 minutes, or less than about 30 minutes in media containing the affinity label. In certain embodiments, step a) comprises incubating cells in media containing the affinity label for a time sufficient to facilitate incorporation of the affinity label into nascent RNA. As used herein, a "time sufficient to facilitate incorporation of the affinity label into nascent RNA" is the amount of time that allows for sufficient incorporation of the affinity label into nascent RNA such that the affinity labeled nascent RNA may be captured in sufficient quantity to identified one or more non-productive splice sites in a target RNA transcript.

In certain embodiments, the affinity label comprises 4-thiouridine, 6-thio-guanosine, 5-ethynyl-uridine, or bromodeoxyuridine. In certain embodiments, the 4-thiouridine labeled total RNA is biotinylated to produce biotinylated total RNA. In certain embodiments, the biotinylated total RNA is captured in step b) with a streptavidin linked solid support. In certain embodiment, the bromodeoxyuridine labeled total RNA is captured in step b) with an anti-bromodeoxyuridine antibody.

In certain embodiments, the cell incubated with an affinity label expresses the target RNA transcript.

In certain embodiments, the method further comprises:
h) identifying split reads that do not map to annotated target RNA transcript exon-exon junctions; and
i) calculating the probability that the split reads represent non-productive transcripts.

In certain embodiments, the non-productive transcripts are rapidly degraded. In certain facets, the non-productive transcripts are not translated into a functional protein.

In one aspect, the disclosure provides a method of identifying non-productive splice sites in a target RNA transcript, the method comprising:
a) incubating a cell with 4-thiouridine to facilitate incorporation of 4-thiouridine into nascent RNA;
b) biotinylating the 4-thiouridine in the nascent RNA;
c) capturing the biotinylated nascent RNA with a streptavidin linked solid support;
d) separating the biotinylated nascent RNA;
e) binding nascent RNA transcript intermediates among the nascent RNA with one or more biotinylated probes complementary to the nascent RNA transcript intermediates;
e) capturing the nascent RNA transcript intermediates bound to the one or more biotinylated probes with a streptavidin linked solid support;
f) isolating the captured nascent RNA transcript intermediates; and
g) sequencing the isolated nascent RNA transcript intermediates, thereby identifying non-productive splice sites in the target RNA transcript.

In certain embodiments, the target RNA transcript comprises ATP1A2, CACNA1A, SETD5, SHANK3, NF2, DNMT1, TCF4, RAI1, PEX1, ARSA, EIF2B5, EIF2B1, EIF2B2, NPCl, ADAR, STXBP1, PRICKLE2, PRRT2, MFSD8, IDUA, SLC6A1, or STX1B. In certain facets, the target RNA transcript comprises SLC6A1. In certain embodiments, the target RNA transcript is expressed from a gene that is related to a disease of haploinsufficiency. In certain embodiments, the target RNA transcript is associated with a disease or disorder. In certain embodiments, the disease or disorder is a disease or disorder of the CNS. In certain embodiments, the disease of the CNS comprises myoclonic-atonic epilepsy (MAE), epilepsy, attention deficit hyperactivity disorder (ADHD), familial hemiplegic migraine-2, familial basilar migraine, alternating hemiplegia of childhood, episodic ataxia type 2, familial hemiplegic migraine, Spinocerebellar ataxia type 6 , mental retardation -23, 3p25 microdeletion syndrome, Phelan-McDermid syndrome, shizophrenia-15, Neurofibromatosis (type 1 or type 2, Meningioma, NF2-related, schwannomatosis 1, Hereditary sensory neuropathy type IE, autosomal dominant cerebellar ataxia, deafness, and narcolepsy, Pitt-Hopkins syndrome, Smith-Magenis syndrome, peroxisome biogenesis disorder la, Heimler syndrome-1, metachromatic leukodystrophy, leukoencephalopathy with vanishing white matter, Niemann-Pick disease type CI and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.

In one aspect, the disclosure provides a method of identifying non-productive splice sites in an SLC6A1 RNA transcript, the method comprising:
a) incubating a cell with an affinity label to facilitate incorporation of the affinity label into nascent RNA;
b) capturing the affinity labeled nascent RNA with a solid support comprising specificity for the affinity label;
c) separating the affinity labeled nascent RNA;
d) binding nascent SLC6A1 RNA transcript intermediates among the total RNA with one or more affinity labeled probes complementary to the nascent SLC6A1 RNA transcript intermediates;
e) capturing the nascent SLC6A1 RNA transcript intermediates bound to the one or more affinity labeled probes with a solid support comprising specificity for the affinity label;
f) isolating the captured nascent SLC6A1 RNA transcript intermediates; and
g) sequencing the isolated nascent SLC6A1 RNA transcript intermediates, thereby identifying non-productive splice sites in the target RNA transcript.

In certain facets, the cell incubated with an affinity label expresses the SLC6A1 RNA transcript. In certain facets, the cell comprises a neuronal cell and/or an astrocyte.

In certain facets, the method further comprising:
h) identifying split reads that do not map to annotated target RNA transcript exon-exon junctions; and
i) calculating the probability that the split reads represent non-productive SLC6A1 RNA transcripts. In certain embodiments, the non-productive SLC6A1 RNA transcripts are rapidly degraded. In certain embodiments, the non-productive SLC6A1 RNA transcripts are not translated into a functional protein.

In certain facets, one or more affinity labeled probes complementary to SLC6A1 RNA matches about ≤ 70% to a second location in the genome, and wherein the one or more affinity labeled probes are located within SLC6A1 exons that are included within at least 50% of annotated SLC6A1 mRNA isoforms.

### Steric Blocking Antisense Oligonucleotides

Antisense oligonucleotides may be capable of sterically blocking non-productive splice sites in target RNA transcripts (e.g., target pre-mRNA). The non-productive splice sites in target RNA transcripts are identified by the methods recited in the section above. Upon identification, antisense oligonucleotides may be designed that comprise a region of complementarity to a target region containing the non-productive splice site. In certain facets, the antisense oligonucleotides comprise a region of complementarity to a target region of any one of SEQ ID NOs: 1-108, as recited in **Table 1** and **Table 2.**

In certain facets, the antisense oligonucleotides comprise at least one nucleotide that has complementarity to the non-productive splice site. The antisense oligonucleotides need not comprise complementarity to the non-productive splice site to reduce the level of non-productive target RNA splice forms or increase the generation of productive target mRNA isoforms. Rather, the antisense oligonucleotides may comprise complementarity to a region around the non-productive splice site. For example, but in no way limiting, the antisense oligonucleotides may comprise complementarity to a region upstream (5') of the non-productive splice site or a region downstream (3') of the non-productive splice site. The antisense oligonucleotides may comprise complementarity to a region 1, 2, 3, 4, 5, 10, 15, 20, 25, 30, 35, 40, 45, 50, 55, 60, 65, 70, 75, 80, 85, 90, 95, 100, or more nucleotides upstream of the non-productive splice site. The antisense oligonucleotides may comprise complementarity to a region about 1 to about 100 nucleotides upstream of the non-productive splice site. The antisense oligonucleotides may comprise complementarity to a region 1, 2, 3, 4, 5, 10, 15, 20, 25, 30, 35, 40, 45, 50, 55, 60, 65, 70, 75, 80, 85, 90, 95, 100, or more nucleotides downstream of the non-productive splice site. The antisense oligonucleotides may comprise complementarity to a region about 1 to about 100 nucleotides downstream of the non-productive splice site.

In certain facets, antisense oligonucleotides may comprise complementarity to an exonic splicing enhancer, an exonic splicing silencer, an intronic splicing enhancer, or an intronic splicing silencer. Generally, antisense oligonucleotides may possess a region of complementarity to a target exonic splicing enhancer, an exonic splicing silencer, an intronic splicing enhancer, or an intronic splicing silencer sufficient to reduce the level of non-productive target splice forms or increase the generation of productive target mRNA isoforms.

Sterically blocking non-productive splice sites in target transcripts may reduce the generation of non-productive splice forms of target transcripts. Non-productive splice forms of target transcripts may be target transcripts that are not translated into a functional protein encoded by the target transcript or target transcripts that are translated into non-functional proteins. In certain facets, antisense oligonucleotides reduce the level of target non-productive splice forms by at least about 20%, about 25%, about 30%, about 35%, about 40%, about 45%, about 50%, about 55%, about 60%, about 65%, about 70%, about 75%, about 80%, about 85%, about 90%, about 95%, or about 100%. The % reduction may be in comparison to a non-specific control antisense oligonucleotide or in comparison to the levels of target non-productive splice forms prior to administration of an antisense oligonucleotide. In certain facets, sterically blocking non-productive splice sites in a target transcript may increase the generation of productive target mRNA isoforms. Productive target mRNA isoforms are mRNA that are translated into a functional protein encoded by the target transcript. In certain facets, antisense oligonucleotides increase the level of productive target mRNA isoforms by at least about 20%, about 25%, about 30%, about 35%, about 40%, about 45%, about 50%, about 55%, about 60%, about 65%, about 70%, about 75%, about 80%, about 85%, about 90%, about 95%, or about 100%. The % increase may be in comparison to a non-specific control antisense oligonucleotide or in comparison to the levels of productive target mRNA isoforms forms prior to administration of an antisense oligonucleotide.

In certain facets, antisense oligonucleotides possess complementarity to a target non-productive splice site in a target transcript, thereby sterically blocking the non-productive splice site. In certain facets, antisense oligonucleotides possess complementarity to a target non-productive 5' splice site (5'ss). In certain facets, the antisense oligonucleotides possess complementarity to a target non-productive 3' splice site (3'ss). Antisense oligonucleotides may possess a region of complementarity to a target non-productive 5'ss or 3'ss sufficient to reduce the level of target non-productive splice forms or increase the generation of productive target mRNA isoforms.

In certain facets, antisense oligonucleotides that are capable of sterically blocking non-productive splice sites in target transcripts, have chemically modified subunits arranged in patterns, or motifs, to confer to the antisense compounds properties such as enhanced the inhibitory activity, increased binding affinity for a target nucleic acid, or resistance to degradation by *in vivo* nucleases.

A nucleoside is a base-sugar combination. The nucleobase (also known as base) portion of the nucleoside is normally a heterocyclic base moiety. Nucleotides are nucleosides that further include a phosphate group covalently linked to the sugar portion of the nucleoside. For those nucleosides that include a pentofuranosyl sugar, the phosphate group can be linked to the 2', 3' or 5' hydroxyl moiety of the sugar. Oligonucleotides are formed through the covalent linkage of adjacent nucleosides to one another, to form a linear polymeric oligonucleotide. Within the oligonucleotide structure, the phosphate groups are commonly referred to as forming the internucleoside linkages of the oligonucleotide.

Modifications to antisense compounds encompass substitutions or changes to internucleoside linkages, sugar moieties, or nucleobases. Modified antisense compounds are often preferred over native forms because of desirable properties such as, for example, enhanced cellular uptake, enhanced affinity for nucleic acid target, increased stability in the presence of nucleases, or increased inhibitory activity.

Chemically modified nucleosides may also be employed to increase the binding affinity of a shortened or truncated antisense oligonucleotide for its target nucleic acid. Consequently, comparable results can often be obtained with shorter antisense compounds that have such chemically modified nucleosides.

The naturally occurring internucleoside linkage of RNA and DNA is a 3' to 5' phosphodiester linkage. Antisense compounds having one or more modified, i.e. non-naturally occurring, internucleoside linkages are often selected over antisense compounds having naturally occurring internucleoside linkages because of desirable properties such as, for example, enhanced cellular uptake, enhanced affinity for target nucleic acids, and increased stability in the presence of nucleases.

Oligonucleotides having modified internucleoside linkages include internucleoside linkages that retain a phosphorus atom as well as internucleoside linkages that do not have a phosphorus atom. Representative phosphorus containing internucleoside linkages include, but are not limited to, phosphodiesters, phosphotriesters, methylphosphonates, phosphoramidate, and phosphorothioates. Methods of preparation of phosphorous-containing and non-phosphorous-containing linkages are well known.

In certain embodiments, antisense compounds targeted to a target nucleic acid comprise one or more modified internucleoside linkages. In certain embodiments, the modified internucleoside linkages are phosphorothioate linkages. In certain embodiments, each internucleoside linkage of an antisense compound is a phosphorothioate internucleoside linkage.

Antisense compounds can optionally contain one or more nucleosides wherein the sugar group has been modified. Such sugar modified nucleosides may impart enhanced nuclease stability, increased binding affinity or some other beneficial biological property to the antisense compounds. In certain facets, nucleosides comprise a chemically modified ribofuranose ring moieties. Examples of chemically modified ribofuranose rings include without limitation, addition of substituent groups (including 5' and 2' substituent groups, bridging of ring atoms to form bicyclic nucleic acids (BNA), replacement of the ribosyl ring oxygen atom with S, N(R), or C(R¹)(R²) (R=H, C₁-C₁₂ alkyl or a protecting group) and combinations thereof. Examples of chemically modified sugars include 2'-F-5'-methyl substituted nucleoside (see PCT International Application WO 2008/101157 Published on Aug. 21, 2008 for other disclosed 5',2'-bis substituted nucleosides) or replacement of the ribosyl ring oxygen atom with S with further substitution at the 2'-position (see published U.S. Patent Application US2005-0130923, published on Jun. 16, 2005) or alternatively 5'-substitution of a BNA (see PCT International Application WO 2007/134181 Published on Nov. 22, 2007) wherein LNA is substituted with for example a 5'-methyl or a 5'-vinyl group).

Examples of nucleosides having modified sugar moieties include without limitation nucleosides comprising 5'-vinyl, 5'-methyl (R or S), 4'-S, 2'-F (i.e., 2'-fluoro), 2'-OCH₃ (i.e., 2'-O-methyl) and 2'-O(CH₂)₂OCH₃ (i.e., 2'-O-methoxyethyl) substituent groups. The substituent at the 2' position can also be selected from allyl, amino, azido, thio, O-allyl, O-C1-C10 alkyl, OCF₃, O(CH₂)₂SCH₃, O(CH₂)₂-O-N(Rₘ)(Rₙ), and O-CH₂-C(=O)-N(Rₘ)(Rₙ), where each Rₘ and Rₙ is, independently, H or substituted or unsubstituted C1-C10 alkyl. 2'-modified nucleotides are useful, for example, 2'-O-methyl RNA, 2'-O-methoxyethyl RNA, 2'-fluoro RNA, and others envisioned by one of ordinary skill in the art.

Examples of bicyclic nucleic acids (BNAs) include without limitation nucleosides comprising a bridge between the 4' and the 2' ribosyl ring atoms. A BNA comprising a bridge between the 4' and 2' ribosyl ring atoms can be referred to as a locked nucleic acid (LNA), and is often referred to as inaccessible RNA. As used herein, the term "locked nucleotide" or "locked nucleic acid (LNA)" comprises nucleotides in which the 2' deoxy ribose sugar moiety is modified by introduction of a structure containing a heteroatom bridging from the 2' to the 4' carbon atoms. The term "non-locked nucleotide" comprises nucleotides that do not contain a bridging structure in the ribose sugar moiety. Thus, the term comprises DNA and RNA nucleotide monomers (phosphorylated adenosine, guanosine, uridine, cytidine, deoxyadenosine, deoxyguanosine, deoxythymidine, deoxycytidine) and derivatives thereof as well as other nucleotides having a 2'-deoxy-erythro-pentofuranosyl sugar moiety or a ribo-pentofuranosyl moiety. In certain facets, antisense compounds include one or more BNA nucleosides wherein the bridge comprises one of the formulas: 4'-(CH₂)-O-2' (LNA); 4'-(CH₂)-S-2'; 4'-(CH₂)-O-2' (LNA); 4'-(CH₂)2-O-2' (ENA); 4'-C(CH₃)2-O-2' (see PCT/US2008/068922); 4'-CH(CH₃)-O-2' and 4'-CH(CH₂OCH₃) -O-2' (see U.S. Pat. No. 7,399,845, issued on Jul. 15, 2008); 4'-CH₂-N(OCH₃)-2' (see PCT/US2008/064591); 4'-CH₂-O-N(CH₃)-2' (see published U.S. Patent Application US2004-0171570, published Sep. 2, 2004); 4'-CH₂-N(R)-O-2' (see U.S. Pat. No. 7,427,672, issued on Sep. 23, 2008); 4'-CH₂-C(CH₃)-2' and 4'-CH₂-C(=CH₂)-2' (see PCT/US2008/066154); and wherein R is, independently, H, C1-C12 alkyl, or a protecting group. Each of the foregoing BNAs include various stereochemical sugar configurations including for example α-L-ribofuranose and β-D-ribofuranose (see PCT international application PCT/DK98/00393, published on Mar. 25, 1999 as WO 99/14226).

In some facets, antisense compounds include one or more 2', 4'-constrained nucleotides. For example, antisense compounds may include those having one or more constrained ethyl (cEt) or constrained methoxyethyl (cMOE) nucleotides. In some facets, antisense compounds are antisense oligonucleotides comprising one or more constrained ethyl (cEt) nucleotides. The terms "constrained ethyl" and "ethyl-constrained" are used interchangeably.

In certain facets, nucleosides are modified by replacement of the ribosyl ring with a sugar surrogate. Such modification includes without limitation, replacement of the ribosyl ring with a surrogate ring system (sometimes referred to as DNA analogs) such as a morpholino ring, a cyclohexenyl ring, a cyclohexyl ring or a tetrahydropyranyl ring such as one having one of the formula:

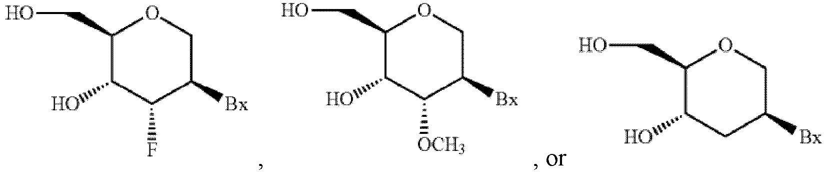

Many other bicyclo and tricyclo sugar surrogate ring systems are also known in the art that can be used to modify nucleosides for incorporation into antisense compounds (see for example review article: Leumann, J. C, Bioorganic & Medicinal Chemistry, 2002, 10, 841-854; Ito, K. R.; Obika, S., Recent Advances in Medicinal Chemistry of Antisense Oligonucleotides. In Comprehensive Medicinal Chemistry, 3rd edition, Elsevier: 2017). Such ring systems can undergo various additional substitutions to enhance activity.

Methods for the preparations of modified sugars are well known to those skilled in the art. In nucleotides having modified sugar moieties, the nucleobase moieties (natural, modified or a combination thereof) are maintained for hybridization with an appropriate nucleic acid target.

In certain facets, antisense compounds targeted to a target nucleic acid comprise one or more kinds of modified nucleotides. In one facet, antisense compounds targeted to a target nucleic acid comprise 2'-modified nucleotides. In one facet, antisense compounds targeted to a target nucleic acid comprise a 2'-O-methyl RNA, a 2'-O-methoxyethyl RNA, or a 2'-fluoro RNA. In one facet, antisense compounds targeted to a target nucleic acid comprise tricyclo-DNA. Tricyclo-DNA belongs to a class of constrained DNA analogs that display improved hybridizing capacities to complementary RNA, see, e.g., Ittig et al., Nucleic Acids Res. 32:346-353 (2004); Ittig et al., Prague, Academy of Sciences of the Czech Republic. 7:21-26 (Coll. Symp. Series, Hocec, M., 2005); Ivanova et al., Oligonucleotides 17:54-65 (2007); Renneberg et al., Nucleic Acids Res. 30:2751-2757 (2002); Renneberg et al., Chembiochem. 5:1114-1118 (2004); and Renneberg et al., JACS. 124:5993-6002 (2002). In one facet, antisense compounds targeted to a target nucleic acid comprise a locked nucleotide, an ethyl-constrained nucleotide, or an alpha-L-locked nucleic acid. Various alpha-L-locked nucleic acids are known by those of ordinary skill in the art, and are described in, e.g., Sorensen et al., J. Am. Chem. Soc. (2002) 124(10):2164-2176.

In certain facets, antisense compounds targeted to a mutant target nucleic acid comprise one or more modified nucleotides having modified sugar moieties. In some facets, the modified nucleotide is a locked nucleotide. In certain facets, the locked nucleotides are arranged in a gapmer motif, e.g. a 3-9-3 gapmer format wherein 9 non-locked nucleotides are flanked by 3 locked nucleotides on each side.

Nucleobase (or base) modifications or substitutions are structurally distinguishable from, yet functionally interchangeable with, naturally occurring or synthetic unmodified nucleobases. Both natural and modified nucleobases are capable of participating in hydrogen bonding. Such nucleobase modifications may impart nuclease stability, binding affinity or some other beneficial biological property to antisense compounds. Modified nucleobases include synthetic and natural nucleobases such as, for example, 5-methylcytosine (5-me-C). Certain nucleobase substitutions, including 5-methylcytosine substitutions, are particularly useful for increasing the binding affinity of an antisense compound for a target nucleic acid. For example, 5-methylcytosine substitutions have been shown to increase nucleic acid duplex stability by 0.6-1.2° C (Sanghvi, Y. S., Crooke, S. T. and Lebleu, B., eds., Antisense Research and Applications, CRC Press, Boca Raton, 1993, pp. 276-278).

Additional modified nucleobases include 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl (-C≡C-CH₃) uracil and cytosine and other alkynyl derivatives of pyrimidine bases, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 2-F-adenine, 2-amino-adenine, 8-azaguanine and 8-azaadenine, 7-deazaguanine and 7-deazaadenine and 3-deazaguanine and 3-deazaadenine.

Heterocyclic base moieties may also include those in which the purine or pyrimidine base is replaced with other heterocycles, for example 7-deaza-adenine, 7-deazaguanosine, 2-aminopyridine and 2-pyridone. Nucleobases that are particularly useful for increasing the binding affinity of antisense compounds include 5-substituted pyrimidines, 6-azapyrimidines and N-2, N-6 and O-6 substituted purines, including 2 aminopropyladenine, 5-propynyluracil and 5-propynylcytosine.

In certain facets, antisense compounds targeted to a target nucleic acid comprise one or more modified nucleotides having modified sugar moieties. In some facets, the modified nucleotide is a locked nucleotide. In certain facets, the locked nucleotides are arranged in a gapmer motif, e.g. a 3-9-3 gapmer format wherein 9 non-locked nucleotides are flanked by 3 locked nucleotides on each side. In certain facets, antisense compounds targeted to a target nucleic acid comprise one or more modified nucleotides. In some facets, the modified nucleotide is 5-methylcytosine. In certain facets, each cytosine is a 5-methylcytosine.

In certain facets, antisense oligonucleotides comprise a 2'-*O*-(2-methoxyethyl) modification at one or more nucleotides. In certain facets, antisense oligonucleotides comprise a 2'-*O*-(2-methoxyethyl) modification at 20% of the nucleotides, at 30% of the nucleotides, at 40% of the nucleotides, at 50% of the nucleotides, at 60% of the nucleotides, at 70% of the nucleotides, at 80% of the nucleotides, or at 90% of the nucleotides. In certain facets, antisense oligonucleotides comprise a 2'-*O*-(2-methoxyethyl) modification at every nucleotide (100% 2'-*O*-(2-methoxyethyl) modification).

In certain facets, antisense oligonucleotides comprise one or more phosphorothioate internucleoside linkages. In certain facets, antisense oligonucleotides comprise one or more phosphorothioate internucleoside linkages and one or more phosphodiester linkages. In certain facets, antisense oligonucleotides comprise phosphorothioate at every internucleoside linkage.

In certain facets, the antisense oligonucleotides comprise a sequence modification pattern of
X_{S}X_{S}X_{S}X_{S}X_{S}X_{S}X_{S}X_{S}X_{S}X_{S}X_{S}X_{S}X_{S}X_{S}X_{S}X_{S}X_{S}X_{S},
X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}X_{S},
X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}X_{S}X_{S},
wherein: s represents a phosphorothioate internucleoside linkage; and
X represents an adenosine, a guanosine, a cytidine, or a thymine comprising a 2'-*O*-(2-methoxyethyl) modification.

In certain facets, an antisense oligonucleotide that targets a target transcript is from about 8 to about 80 nucleotides in length. In other facets, the antisense oligonucleotide that targets a target transcript is from about 15 to about 25 nucleotides in length. In other facets, the antisense oligonucleotide that targets a target transcript is from about 18 to about 20 nucleotides in length. For example, the antisense oligonucleotides are 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, or 80 nucleotides in length, or a range defined by any two of the above values.

### Diseases and Disorders of Haploinsufficiency, and Related Genes.

The methods described herein can be used to identify non-productive splice sites in the target RNA transcripts expressed from genes that are related, or associated with, diseases or disorders of haploinsufficiency. For haploinsufficient genes, when one copy of a gene is deleted or contains a loss-of-function mutation, the dosage of normal product generated by the single wild-type, or otherwise functional gene, is not sufficient for the complete function of the expressed protein. That is, there is not enough of the protein being produced, and a disease state occurs (a disease or disorder of haploinsufficiency).

Identified non-productive splice sites in the target RNA transcripts can be targeted with steric blocking antisense oligonucleotides to increase the level of functional RNA transcript and functional protein encoded by said transcript.

Genes and corresponding target RNA transcripts related to diseases or disorders of haploinsufficiency include, but are not limited to, ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPCl, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1, and WDTC1.

Having now described certain embodiments in detail, the same will be more clearly understood by reference to the following examples, which are included for purposes of illustration only and are not intended to be limiting.

### EXAMPLES

The present invention is further illustrated by the following examples which should not be construed as further limiting.

### Example 1 - Materials and methods

### Antisense Oligonucleotides

All phosphoramidites will be purchased from ChemGenes. 0.1M DDTT (ChemGenes) will be used as the sulfurising reagent and 0.25M BTT (AIC) as the activator. Antisense oligonucleotides will be synthesized on Dr. Oligo 48, ABI394, AKTA Oligopilot10 or AKTA Oligopilot 100 synthesizers, according to the required scale. MOE phosphoramidites will be coupled for 8 minutes. Oligonucleotides will be deprotected in concentrated aqueous ammonia at 55°C for 18 h and purified using ion-exchange chromatography (eluting with 30% acetonitrile in water containing increasing gradients of NaClO₄). Final purification, desalting, concentration and pH adjustment will be effected by diafiltration in an Amicon centrifugal filter. All oligonucleotides will be characterized by LCMS.

### Cell line selection

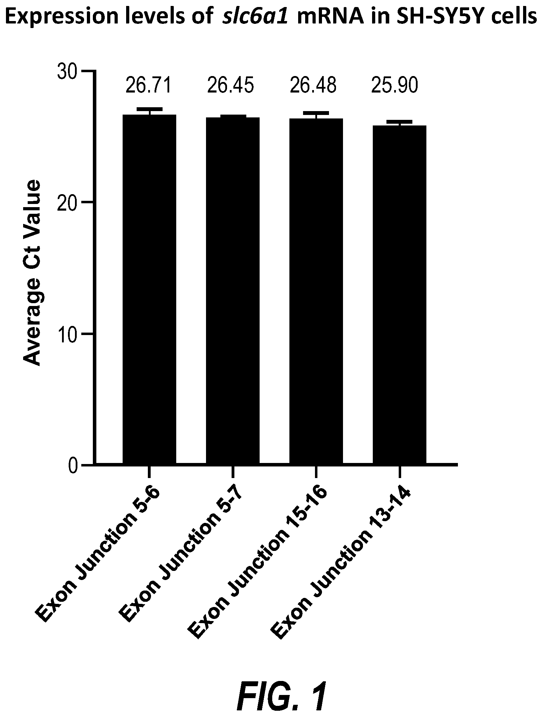

Splice site identification and antisense oligonucleotide testing experiments must be performed in a cell line in which SLC6A1 is transcriptionally active. Furthermore, since SLC6A1 intronic sequences are not well conserved between mouse and human and cryptic splice sites often occur in introns, experiments must be performed in a human cell line. The experimentally tractable human SH-SY5Y cell line (derived from neuroblastoma cells) expresses SLC6A1 **(****Figure 1****)**. However, it would be more optimal to perform these experiments in human GABAergic interneurons. Given that these are impossible to obtain from a living patient, approaches have been recently developed to differentiate GABAergic inhibitory neurons (iNs) from human embryonic stem cells (hESCs) or induced pluripotent stem cells (iPSCs). Thus, initial experiments and optimizations will be performed in parallel in SH-SY5Y cells and in iNs derived from commercial hESCs and an iPSC line derived from a clinical subject.

### 4sU labeling of nascent RNA intermediates

Short time point metabolic labelling of SH-SY5Y cells with 4-thiouridine (4sU) will be carried out as described (Dölken et al. 2008; Pai et al., 2017). 4sU incorporates into newly created RNA in the place of standard uridine nucleotides and can be selectively isolated to capture nascent RNA shortly after its biogenesis. SH-SY5Y cells will be cultured in DMEM supplemented with 10% FBS. Newly transcribed RNA from three independent replicates of SH-SY5Y cells will be labeled for various time intervals, for example, 2, 5, 15 or 30 min, using 500 µM 4-thiouridine (Sigma, T4509). Additionally, for analysis of steady-state RNA levels, two independent biological replicates of SH-SY5Ycells will be generated without 4sU labeling. To normalize samples and assess metabolic labeled RNA capture efficiency, several synthetic RNAs will be spiked into the Trizol preparation at specific quantities per 10⁶ cells. Quantities will be determined as described previously (Henriques et al., 2013).

### RNA Extraction and Quantitative RealTime-PCR

Total RNA will be isolated from SH-SY5Y cells using Trizol (Thermo Scientific) and subsequently treated with DNase I (Qiagen). One µg of total RNA will be reverse transcribed into cDNA using random hexamers and MultiScribe reverse transcriptase (ThermoScientific) following the manufacturer's instructions. Quantitative PCR will be performed on a StepOnePlus Real-Time PCR system using SYBR Green Master Mix (Applied Biosystems) and 0.2 µM of forward and reverse primers as described in (Jiang et al., Neuron, 2016, 90, 535-550; Tran et al, 2015, Neuron, 87, 1207-1214). Ct values for each sample and gene will be normalized to GAPDH. The 2(-ΔΔCt) method was used to determine the relative expression of each target gene.

### Biotinylation of nascent 4sU-labeled RNA intermediates

To purify metabolic labeled RNA 300 µg total RNA will be used for the biotinylation reaction. Separation of total RNA into newly transcribed and untagged pre-existing RNA will be performed as previously described (Windhager et al., 2012; Cleary et al., 2005). Specifically, 4sU-labeled RNA will be biotinylated using EZ-Link Biotin-HPDP (Thermo Fisher, Waltham MA), dissolved in dimethylformamide (DMF) at a concentration of 1 mg/ml. Biotinylation will be done in labeling buffer (10 mM Tris pH 7.4, 1 mM EDTA) and 0.2 mg/ml Biotin-HPDP for 2 hr at 25°C. Unbound Biotin-HPDP will be removed by extraction with chloroform/isoamylalcohol (24: 1) using MaXtract (high density) tubes (Qiagen, Germany). RNA will be precipitated at 20,000 g for 20 min with a 1:10 vol of 5 M NaCl and 2.5X volume of ethanol. The pellet will be washed with ice-cold 75% ethanol and precipitated again at 20,000 g for 5 min. The pellet will be resuspended in 1 ml RPB buffer (300 mM NaCl, 10 mM Tris pH 7.5, 1 mM EDTA).

### Capturing biotinylated 4sU RNA

Biotinylated 4sU RNA will be captured using Streptavidin MagneSphere Paramagnetic particles (Promega, Madison WI). Before incubation with biotinylated 4sU RNA, streptavidin beads will be washed four times with wash buffer (50 mM NaCl, 10 mM Tris pH 7.5, 1 mM EDTA) and blocked with 1% polyvinylpyrrolidone (Millipore Sigma, Burlington MA) for 10 min with rotation. Biotinylated 4sU RNA will then be incubated with 600 µl of beads with rotation for 30 min at 25°C. Beads will be magnetically fixed and washed 5 times with 4TU wash buffer (1 M NaCl, 10 mM Tris pH 7.5, 1 mM EDTA, 0.1% Tween 20). Unlabeled RNA present in the supernatant will be discarded. 4sU RNA will be eluted twice with 75 µL of freshly prepared 100 mM dithiothreitol (DTT). 4sU RNA will be recovered from eluates by ethanol precipitation.

### Library preparation

RNA quality will be assessed using a Bioanalyzer Nano ChIP (Agilent). Ribosomal RNA will be removed prior to library construction by hybridizing to ribo-depletion beads that contain biotinylated capture probes (Ribo-Zero, Epicentre, Madison WI). RNA will then be fragmented and libraries will be prepared according to the TruSeq Stranded Total RNA Gold Kit (Illumina, San Diego CA) using random hexamer priming. cDNA for the two 'total' RNA samples will be prepared using an equal mix of random hexamers and oligo-dT primers (Pai et al., 2017).

### Illumina sequencing

Libraries will be sequenced on an Illumina HiSeq machine with paired-end 150 nucleotide reads (100 nucleotide reads for the 'total' RNA samples), for an average of 100 million read pairs per library. Reads for each sample will be filtered, removing pairs where the mean quality score of one or both mates fell below 20. Mean fragment length and standard deviation will be assessed using CollectInsertSizeMetrics, a component of Picard Tools 1.62. All reads will subsequently be aligned to hg38 with STAR. Strand-specific alignments will be performed for the 4sU RNA-seq (--library-type first strand), while unstranded alignments will be performed for the total RNA-seq (--library-type unstranded).

### Identification of non-productive splicing

Sites of non-productive splicing will be identified by non-annotated junction reads with canonical or non-canonical splice site sequences within annotated introns using nascent RNA reads from short labeling periods. To do so, the raw 4sU-seq reads will be re-mapped with the STAR v2.5 software (Dobin et al., Bioinformatics. 2013; 29(1):15-21), with the mapping parameter-outSAMattribute NH HI AS nM jM to mark the intron motif category for each junction read in the final mapped file.

The jM attribute adds a jM:B:c SAM attribute to split reads arising from exon-exon junctions. All junction reads will be first isolated and separated based on the value assigned to the jM:B:c tag. Junction reads spanning splice sites in the following categories will be considered to be annotated or canonical: (1) any annotated splice site [jM:B:c,[20-26]], (2) intron motifs containing "GT-AG" (or the reverse complement) [jM:B:c,1 or jM:B:c,2], (3) intron motifs containing "GC-AG" (or the reverse complement) [jM:B:c,3 or jM:B:c,4], and (4) intron motifs containing "AT-AC" (or the reverse complement) [jM:B:c,5 or jM:B:c,6]. Junction reads with jM:B:c,0 will be considered to arise from non-canonical non-annotated splice sites.

### Statistical Analysis

All data will be graphed as mean SEM and analyzed using GraphPad Prism Software (Version7). Tests between two groups will use the two-tailed student-t test. Tests between multiple groups used one-way analysis of variance (ANOVA) corrected with Bonferroni multiple comparison post-hoc test. *p<0.05, **p<0.01, ***p<0.001, ****p<0.0001, ns not significant

### Example 2 - Identifying cryptic splice sites by using computational software

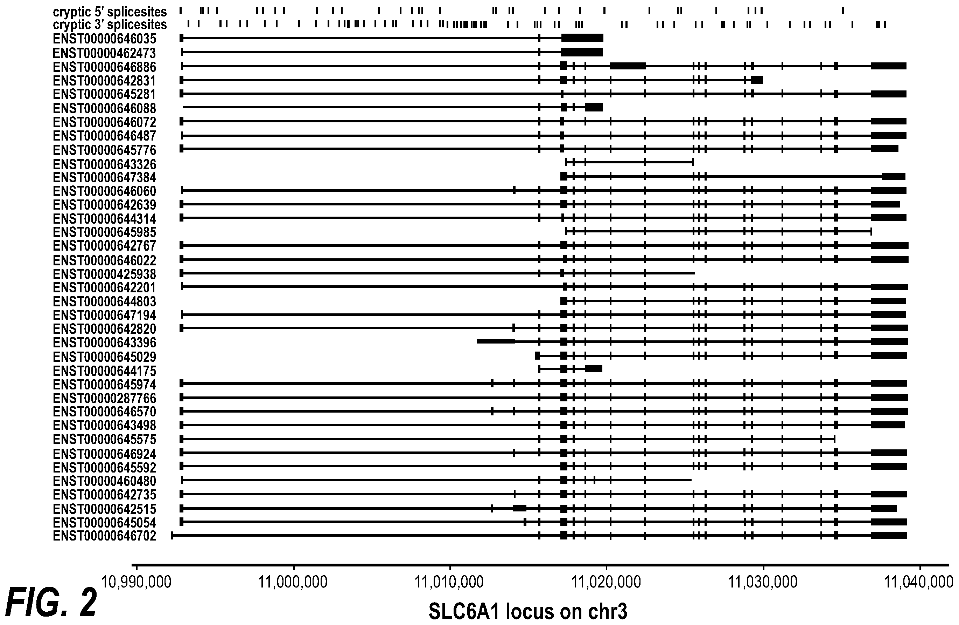

Identifying transcripts that are being created but not lasting until maturity would enable targeted optimization of mRNA processing pathways to allow for maturation of these transcripts. The first challenge is to identify sites at which non-productive splicing commonly occurs. Those sites can then be blocked by complementary antisense oligonucleotides to redirect the splicing machinery towards sites promoting productive splicing. SLC6A1 is a 46.5kb gene with 17 introns and extensive alternative splicing, increasing the probability that many cryptic splice sites exist within this genomic space. Two complementary approaches will be used to identify sites of non-productive splicing in SLC6A1-expressing neurons: (1) computational identification of strong cryptic splice sites and (2) targeted sequencing of SLC6A1 mRNA intermediates (see Example 3). Computational software will be used to identify the sites that may underlie non-productively spliced isoforms. The commonly used maxEnt splice site algorithm will be applied, which uses a maximum entropy model to score sites relative to the entropy of known 5' or 3' splice site elements (Yeo and Burge 2004). Publicly available MaxEnt resources are available at: http://hollywood.mit.edu/burgelab/maxent/Xmaxentscan_scoreseq.html; MaxEntScan::score5ss will be used for human 5' splice sites and MaxEntScan::score3ss will be used for human 3' splice sites. The SLC6A1 genetic locus on human chromosome 3 is shown in **Figure 2****.**

A sliding window algorithm will be used to scan every 9 and 23 nucleotide region segments in the human SLC6A1 gene sequence and the maximum entropy for 5' and 3' splice site motifs will be calculated, respectively. After removing annotated splice sites, an entropy threshold to identify high-scoring putative cryptic splice sites will be conditioned on.

Initial analyses of SLC6A1 RNA with maxEnt have identified 34 cryptic 5' splice sites, depicted as SEQ ID NOs. 1 to 34 in **Table 1,** and 74 cryptic 3' splice sites, depicted as SEQ ID NOs. 35-108 in **Table 2.** SEQ **ID** No. 109 depicts the entire SLC6A1 RNA sequence. Genomic scanning scripts will be used to identify high-scoring cryptic polyadenylation sites in SLC6A1, the usage of which might lead to truncated isoforms that are similarly targeted for degradation. Once identified, these sites can be targeted with antisense oligonucleotides to block the formation of non-productive, truncated transcripts.

The nucleic acid target sequences of **Table 1** and **Table 2,** and genomic sequences of **Table 4,** correspond to the genomic target sequence. An antisense oligonucleotide is designed to have sufficient complementarity to the corresponding RNA transcript expressed from said genomic target sequence (i.e., the reverse complement of the genomic target sequence, where each T is replaced by a U). For example, but in no way limiting, an antisense oligonucleotide may possess sufficient complementarity to CAGCCUGAUUCUGCCUGUGACUCACUUUGUGACCUCAGGAGAGUCCCUCC (the RNA transcript sequence corresponding to SEQ ID NO: 1 in **Table 1)** to block the formation of non-productive, truncated transcripts.

### Example 3 - Targeted high-throughput sequencing of SLC6A1 nascent RNA intermediates

Targeted high-throughput sequencing of SLC6A1 nascent RNA intermediates will be performed to experimentally identify short-lived non-productive isoforms. Nascent RNA intermediates will be captured with methods as described in Example 1. To obtain high-resolution information about nonproductive SLC6A1 splicing, biotinylated probes complementary to regions of the gene to selectively isolate SLC6A1 mRNA will be used from the pool of nascent RNA. Probes will be designed to have optimal nucleotide composition and chemistry, match a unique location in the human genome (<70% match to a second location), and be located within exons that are included within 50% of annotated SLC6A1 isoforms **(****Figure 1****).** The probes will tile across these exons within SLC6A1, located at the beginning, middle, and end of the gene to enable the most comprehensive capture of entire distribution of possible isoforms. Nascent SLC6A1 RNA across all intermediate lifetimes will be sequenced using a combination of short-read and long-read high-throughput sequencing. Short-read sequencing with the Illumina platform provides the ability to obtain high-resolution information about cryptic splice site usage with higher coverage. A total of 12 libraries (3 replicates for each of the 5, 15, 30-minute nascent RNA timepoints and 3 replicates of the steady state sample) will be sequenced in 1 NextSeq lane, with an estimated 450 million reads across all libraries. These data will be used to comprehensively identify sites of cryptic splicing leading to non-productive isoforms across a range of mRNA intermediate lifetimes. To do so, non-canonical splicing junctions will be identified by specifically analyzing splitreads that do not map to annotated SLC6A1 exon-exon junctions. Cryptic splice sites that recurrently have split-junction reads in multiple samples and after sub-sampling approaches will be considered to be major sites of cryptic splicing in SLC6A1. Open reading frames (ORFs) will be predicted and premature stop codon usage in all isoforms (both annotated and cryptic) expressed in neuronal systems and identified through this analysis. These predictions will be used to quantify the probability that cryptic splice site usage leads to isoform degradation through nonsense mediated decay (NMD) pathways. Splice sites that lead to NMD will be prioritized for downstream antisense oligonucleotide design and targeting approaches (Aim 2). Further Oxford Nanopore Minion cDNA libraries will be generated with the same 12 samples and sequence them across 2 Minion flowcells to assess the long-range connectivity between different cryptic sites. Since long-read datasets are inherently error prone, annotations of cryptic splice sites derived from the short-read Illumina data will be used to refine mapping of the long-read Minion data. These data will provide isoform level insights that may be useful for combinatorial targeting of multiple cryptic splice sites for maximal effect with a minimal number of antisense oligonucleotides.

### Example 4 - Use of publicly available datasets

For a more comprehensive picture of non-productive splicing isoforms in SLC6A1, publicly available sequencing datasets will be analyzed that are designed to capture total cellular mRNA, as opposed to only polyadenylated mature mRNA (Schwarzl et al. 2015; Rybak-Wolf et al. 2015; Pandey et al. 2014). We will also generate a genome-wide nascent RNA sequencing dataset with SH-SY5Y cells, neurons derived from human embryonic stem cells, and neurons derived from induced-pluripotent stem cells. These datasets will all be enriched for mature polyadenylated RNA but will also contain a small amount of information about intermediate RNA species. In all of these datasets, we will identify novel isoforms of SLC6A1 (using the MAGIQ splicing analysis software), and identify splicing junction reads.

### Example 5 - Antisense oligonucleotides targeting non-productive splice sites that increase the expression of productive SLC6A1 mRNA molecules

Computational software was used to identify cryptic splice sites that are likely to underlie non-productively spliced isoforms of SLC6A1. Specifically, the maxEnt splice site algorithm was applied, which uses a maximum entropy model to score sites relative to the entropy of known 5' or 3' splice site elements (Yeo and Burge 2004). A sliding window algorithm was used to scan every 9 and 23 nucleotide region segment in the human SLC6A1 gene sequence and calculate the maximum entropy for 5' and 3' splice site motifs, respectively. After removing annotated splice sites, an entropy threshold of 8.72 and 7.25 (mean entropy scores for annotated sites) were used to identify high-scoring putative cryptic 5' and 3' splice sites, respectively. Initial computational analyses identified 12 cryptic 5' splice sites and 84 cryptic 3' splice sites in SLC6A1. The number of predicted sites that would target was narrowed to 15 (5 5' splice sites and 10 3' splice sites) based on their position along the SLC6A1 locus (focusing on intron 1) and maximizing the specificity of those sites by selecting antisense oligonucleotides with minimal complementarity to other sites in the transcriptome using NCBI BLAST.

Three antisense oligonucleotides were selected for each of the 15 predicted sites. These antisense oligonucleotides were designed as steric blockers, with each nucleotide comprising a 2'-O-methoxyethyl RNA (MOE) modification and a phosphorothioate backbone. Each antisense oligonucleotide was 20 nucleotides in length. 45 antisense oligonucleotides were synthesized using standard methods on a Dr. Oligo 48 synthesizer, and their identity and purity was confirmed by high performance liquid chromatography coupled to mass spectroscopy (LCMS).

Each antisense oligonucleotide was transfected into KNS60 neuroblastoma cells (JCRB Cell Bank). These cells were cultured in DMEM (Sigma) supplemented with 5% fetal bovine serum at 37°C with 5% CO2. One day prior to transfection, cells were seeded at a density of 50,000 and incubated overnight. Cells were transfected with 50 nM antisense oligonucleotide using Lipofectamine RNAiMAX (Thermofisher) transfection reagent.

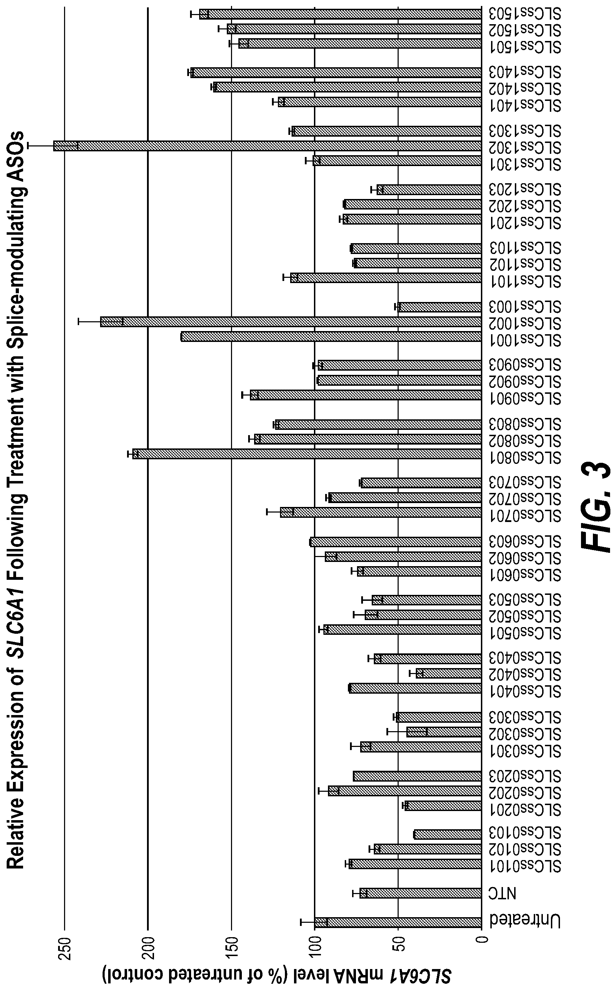

After 24 hours, RNA was collected using TRI Reagent (Sigma) and subjected to reverse transcription using the High-Capacity cDNA Reverse Transcription kit (ThermoFisher) according to the manufacturer's instructions. Resulting cDNA was used for qPCR reaction with IDT PrimeTime Taqman primers for SLC6A1 (Hs.PT.58.40113647) and GAPDH (Hs.PT.39a.22214836). The qPCR was performed in technical duplicate on a Bio-Rad CFX96 Real-Time System Thermal Cycler.
Several of the antisense oligonucleotides produced activation of SLC6A1 as seen by up to 2.5-fold increases in mRNA expression (Figure 3). Sequences, masses, target sites, and percent activation details of active antisense oligonucleotides are presented in **Table 3.**

### Example 6 - Identifying novel cryptic splice sites by enrichment and sequencing of nascent pre-mRNA

Publicly available high-throughput sequencing data was used to identify cryptic splice sites that are used only in splicing intermediates targeted for degradation (i.e., not present in steady-state mRNA sequencing data). Specially, raw data was downloaded from 3 datasets derived from human K562 erythroleukemia cells: (1) mRNA enriched by capturing a polyA tail (ENCODE Consortium), (2) mRNA after an shRNA knockdown of the UPF1 degradation machinery (ENCODE Consortium), and (3) pre-mRNA enriched by labeling nascent RNA with 4-thio-uridine for 5 minutes and using a biotin-streptavidin interaction to selectively isolate nascent RNA (Schwalb et al. Science. 2016;352(6290):1225-8). All datasets were mapped to the human reference genome (hg38) with the STAR transcriptome mapping software (Dobin 2013, *supra*) and a custom python script was used to annotate the split-junction reads from the *.SJ.out.tab file from the STAR output. Junction sites were described as being annotated or unannotated based on presence in the Ensembl GRCh38.95 annotation database. Junction sites were also described as canonical or non-canonical based on the dinucleotide sequences used for the 5' splice site and 3' splice sites (GT, GC, AT, and corresponding reverse complements, or AG, AC, and corresponding reverse complements were considered to be canonical 5' or 3' splice sites, respectively). Only junction sites for which both ends of the read mapped to the same gene and one or both of the splice site dinucleotides were either non-canonical or unannotated as splice sites were retained for further analysis. These junction reads were deemed to be cryptic splice sites by virtue of their lack of annotation or canonical dinucleotide status.

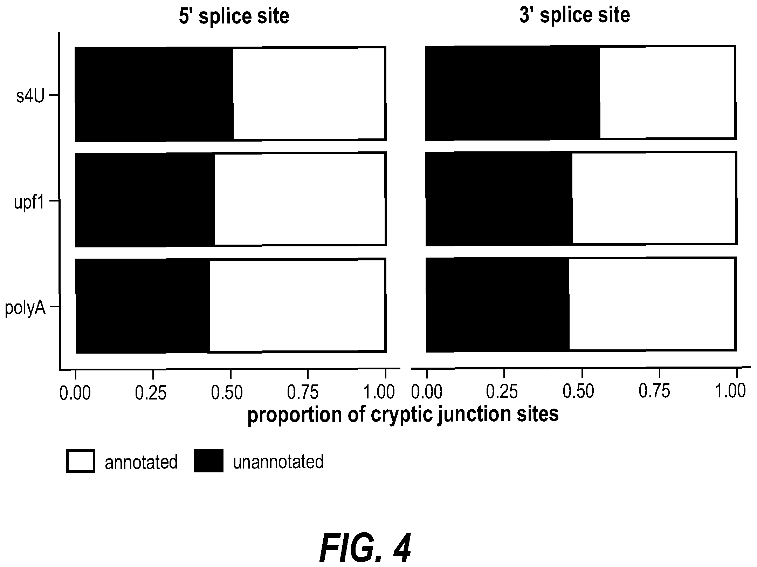

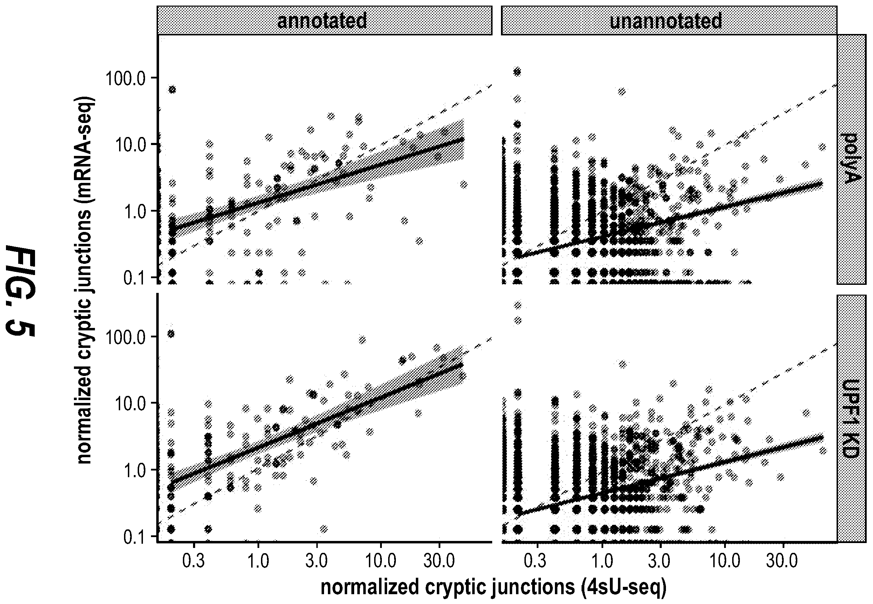

For each dataset, the proportion of cryptic sites that were previously annotated as being used as splice sites were evaluated **(****Figure 4****).** There is an increase of unannotated 5' and 3' splice sites in the nascent RNA 4sU-sequencing data relative to the steady-state polyA enriched mRNA or the mRNA from UPF1-depleted cells, which should contain more mRNAs that are quickly degraded in homeostatic conditions. Across individual cryptic sites, there is an overall increase in the number of junction reads for the majority of cryptic sites in the 4sU-seq data relative to both non-metabolic labeled datasets **(****Figure 5****).** Together, these data show that specifically isolating and sequencing nascent RNA with metabolic labeling have increased power to detect more active cryptic splice sites with higher signal than either steady-state polyA enriched mRNA sequencing or mRNA sequencing after perturbation of degradation machinery cells (i.e., UPF1-depleted cells).

Several of the novel cryptic sites that were identified using the 4sU-seq data were present in genes that are known to be haploinsufficient when mutated. From a representative list of 92 genes known to be involved in haploinsufficiency disorders and expressed in K562 cells, 190 novel cryptic splice sites were identified across 31 genes that were identified using the 4sU-seq data but absent in both the steady-state polyA mRNA-seq or UPF1 knockdown datasets **(Table 4)**.

**Table 1 - Target sequences for 34 cryptic 5' splice sites**

| SEQ ID NO. | Target Location | Nucleic acid target sequence |
|---|---|---|
| 1 | >SLC6A1::3:10992186-11039247;startsite=110290 76;maxEnt=8.4(+) | GGAGGGACTCTCCTGAGGTCACAAAGTGAG TCACAGGCAGAATCAGGCTG |
| 2 | >SLC6A1::3:10992186-11039247;startsite=110183 12;maxEnt=10.13(+) | TGACAGGCACCCAGGTAGATACATGGTGAG TCATGCTCACTGACCGAGGG |
| 3 | >SLC6A1::3:10992186-11039247;startsite=110269 99;maxEnt=8.49(+) | GTCACAGAGATCACATGCTCACAAGGTAAT AAAATATCACAAGGCAAATG |
| 4 | >SLC6A1::3:10992186-11039247;startsite=110080 10;maxEnt=8.63(+) | ATGGAGGGTTTGGGGGGTTCCACAGGTACC CCATTAGAGACAGCAGAGTG |
| 5 | >SLC6A1::3:10992186-11039247;startsite=109945 43;maxEnt=8.34(+) | GTGGCCAGTCCTTGGAGGACAAACAGTGAG TCCCAAGCAGAGAGACGCAG |
| 6 | >SLC6A1::3:10992186-11039247;startsite=109994 03;maxEnt=8.08(+) | TGTATTACATATGCATTTTTAGCAGGTTGGT TCAGCATAATGACACAGAA |
| 7 | >SLC6A1::3:10992186-11039247;startsite=109942 25;maxEnt=7.7(+) | TCCCCCAAATTCCAAAACAAAAGAGGTCAG TGAAAGCTACTCGCATTTTG |
| 8 | >SLC6A1::3:10992186-11039247;startsite=110169 80;maxEnt=7.62(+) | TTCCTTCCTCCAGGAGCAAGGGCGGGTGAG AGGAAGAGGGCTTATAGAGA |
| 9 | >SLC6A1::3:10992186-11039247;startsite=110198 59;maxEnt=7.52(+) | CTGCGCCTGGCAGAGAACAAGCCCTGTAAG TGTTTGCTGGTGTCGTTGCA |
| 10 | >SLC6A1::3:10992186-11039247;startsite=110030 74;maxEnt=9.99(+) | CTAAAAATACAAAAAAATTAGCCAGGTATG GTGGCAGGCACCTGTAGTCC |
| 11 | >SLC6A1::3:10992186-11039247;startsite=110247 24;maxEnt=8.55(+) | TATTTAGAGTCACATAAAATTGGAGGTAACC TGAATGCATAGCAGTGGGG |
| 12 | >SLC6A1::3:10992186-11039247;startsite=110245 66;maxEnt=8.49(+) | AGTTCCATTTCTCGAAAGCAACAAGGTAATA TGGATCAAAAGCCTGTGAT |
| 13 | >SLC6A1::3:10992186-11039247;startsite=110127 97;maxEnt=9.65(+) | TTTCTTCCGCCTGCTCCACCAGCAGGTAAAG GAGGCTGATCACAGGCTGG |
| 14 | >SLC6A1::3:10992186-11039247;startsite=110093 61;maxEnt=10.65(+) | CCTGCTTCTGCAAATTCCCTCTCAGGTACGT TGAGGCAGCTGAGGGGTTT |
| 15 | >SLC6A1::3:10992186-11039247;startsite=109927 82;maxEnt=8.19(+) | GACCGAGACAGCGGAGAGGTTGCGGGTGAG CTGCGCTGAGCCCAGGAGCC |
| 16 | >SLC6A1::3:10992186-11039247;startsite=110294 91;maxEnt=8.34(+) | TGTGCCAAGCTCTGGGCACATAACAGTGAG TCAGATGGGGTCCCTGCCCT |
| 17 | >SLC6A1::3:10992186-11039247;startsite=110068 34;maxEnt=9.21(+) | GTGTGATCATCATTCTTATTACAGGGTAAGA TGCGCCCTCTTTTCCTGCA |
| 18 | >SLC6A1::3:10992186-11039247;startsite=110227 17;maxEnt=8.34(+) | TACGCAGCACAGTGCGAAGCTCACAGTGAG TCCCATGGGATTCCACTGGG |
| 19 | >SLC6A1::3:10992186-11039247;startsite=109976 71;maxEnt=8.7(+) | GGGAGAGGGCAGCAGCCACAGGGAGGTGA GCAAAGACATTTGGTATCAGT |
| 20 | >SLC6A1::3:10992186-11039247;startsite=110054 43;maxEnt=10.08(+) | ACCTCAGGCTTCTCTTGGCTGAAAGGTAGGC TCCTTCCCTCCCTCCTTGG |
| 21 | >SLC6A1::3:10992186-11039247;startsite=110075 57;maxEnt=8.78(+) | GGACTGGCATAAGGTCACACAGCTAGTAAG TTTAGAGAGAGATTTCAAAC |
| 22 | >SLC6A1::3:10992186-11039247;startsite=110082 16;maxEnt=7.79(+) | CATTTAGAGTGGCCCCGGCACATTAGTAAGT GTGTCCAGCTCACTTCCTC |
| 23 | >SLC6A1::3:10992186-11039247;startsite=109951 32;maxEnt=7.7(+) | GCCATTTCTGGCCAGGTGACCTTGGGTACGT TCTTGTTCCTGTTGGAAGG |
| 24 | >SLC6A1::3:10992186-11039247;startsite=110160 45;maxEnt=10.57(+) | CCACGCTGCCCCTGGAAATAAAAAGGTAAG AAGGCTGCAGAGTGTCAGTA |
| 25 | >SLC6A1::3:10992186-11039247;startsite=110298 77;maxEnt=8.24(+) | AATCCCAGCTACTTGGGAGGCTGAGGTAGG AGAATTGCTTGAACCCGGGA |
| 26 | >SLC6A1::3:10992186-11039247;startsite=109980 50;maxEnt=8.83(+) | ACAGCCCTGGGAACTTCAAGTGAAGGTAAT TTTATTGTTATTATTGGTAC |
| 27 | >SLC6A1::3:10992186-11039247;startsite=110140 26;maxEnt=7.61(+) | AAATCACATGTATGTCTGCTTTATGGTGAGG TCTTCAGAGCAGCCACCGT |
| 28 | >SLC6A1::3:10992186-11039247;startsite=110014 71;maxEnt=10.07(+) | GCACTTCGTGGAGGTGCAGAGTCAGGTGAG GAGAGGTGAAGTGACTCATC |
| 29 | >SLC6A1::3:10992186-11039247;startsite=110350 89;maxEnt=9.06(+) | GAACGGAGATCAGTGTGGCTGGAAGGTAAA GTGGAAAGGGGTGCGAGGAG |
| 30 | >SLC6A1::3:10992186-11039247;startsite=109940 83;maxEnt=8.23(+) | CCTTTGTTTTCTATGACACACAAGGTGGGT GTTTGGCAAGAGAGTAGGG |
| 31 | >SLC6A1::3:10992186-11039247;startsite=110129 56;maxEnt=8.46(+) | AGGAAAAGTAACAACTTGCAAAAAGGTTGG TTCTGCTTCAGAGAAATGTT |
| 32 | >SLC6A1::3:10992186-11039247;startsite=109988 40;maxEnt=8.34(+) | GAACTTGTCCAGCTGGGCCCTGACAGTGAGT TCAGAGGGCCTGCTGGGCA |
| 33 | >SLC6A1::3:10992186-11039247;startsite=110024 99;maxEnt=9.45(+) | GCATTTGGCTGTTTGAAAAGCGCTGGTAAGA GCTGGGATCCTGATGATTG |
| 34 | >SLC6A1::3:10992186-11039247;startsite=110138 05;maxEnt=7.65(+) | AGCACCAGGGTGGCTTTGCTGCTGTGTAAGT CAAAGAGCCTTCTCCGTTC |

**Table 2 - Target sequences for 74 cryptic 3' splice sites**

| SE Q ID N O. | Target Location | Nucleic acid target sequence |
|---|---|---|
| 35 | >SLC6A1::3:10992186-1103 9247; startsite=109953 50 ;maxEnt=8.94(+) | TAACCCTACTGTTCTAATTTCCAGGCCAACTC TGTCCTATTGTGGGTCTC |
| 36 | >SLC6A1::3:10992186-11039247;startsite=11024297 ;maxEnt=9.97(+) | TGGCTGCCTTCCTTAATCCTGCAGAATCTCGG GGTCAAGTCAGGGAGGTG |
| 37 | >SLC6A1::3:10992186-11039247;startsite=11016703; maxEnt=8.34(+) | GGGGACACCTTTCTCCCCATTCAGCCACAGG TGGAGCTTCTTTCTTGCTC |
| 38 | >SLC6A1::3:10992186-11039247;startsite=11029029 ;maxEnt=9.21(+) | TCAGCCACCTTCTCCATTTTCCAGAAGGGGA AACTGATGCCCAGAGGGGA |
| 39 | >SLC6A1::3:10992186-11039247;startsite=11003960 ;maxEnt=7.75(+) | ATACCATAAAATTCCTTTCCACAGCTAAGTG AGTGAGTCAAGAACAGATG |
| 40 | >SLC6A1::3:10992186-11039247;startsite=11012234 ;maxEnt=8.97(+) | GTCTTGTTCTTACACCTCTTGCAGCACTTATC ACAGGGTATTATGATCAG |
| 41 | >SLC6A1::3:10992186-1103 9247; startsite=10998924 ;maxEnt=8.12(+) | GGTGCTACTCTCTCCTTCTGCCAGAGAGGAC CCTGAGCCCAAGGGTGGTG |
| 42 | >SLC6A1::3:10992186-11039247;startsite=11004488 ;maxEnt=7.9(+) | CATCACCACTTCCCTTCTCCAAAGCATGCAA GTTCAATTTCTAAAATTCA |
| 43 | >SLC6A1::3:10992186-11039247;startsite=11010695 ;maxEnt=8.06(+) | ATTAATTTCTTTCTTAAACCACAGAGTTTCAA AAAGAAATAAAGGCAAAC |
| 44 | >SLC6A1::3:10992186-11039247;startsite=11023592 ;maxEnt=8.93(+) | AAAGTATCTCTGATCCATTCCTAGGACTAGG GACGCCGGAGGAGGGCACA |
| 45 | >SLC6A1::3:10992186-11039247;startsite=11018094 ;maxEnt=7.61(+) | TGACATCACATATCCATCCACCAGGTGCATA TCTGAGCAGAGTGAGGGCT |
| 46 | >SLC6A1::3:10992186-11039247;startsite=11029153 ;maxEnt=8.31(+) | GCTGGGCCTCCCGTCCTTCCTTAGAGGGCCA GGCTTTGGGTGGGTTGGGG |
| 47 | >SLC6A1::3:10992186-11039247;startsite=11030256 ;maxEnt=8.3(+) | TCACCCCCACCCCCCCCCACCAGATCCAAA TGTAGTTCTGCTCCAGAGT |
| 48 | >SLC6A1::3:10992186-11039247;startsite=11006564 ;maxEnt=10.01(+) | TCTCCTCTCCTCCTCCCCACACAGCTGCTGGG CGGTCTTGCCAAGTCACC |
| 49 | >SLC6A1::3:10992186-11039247;startsite=11032621 ;maxEnt=7.74(+) | AATAATTTCTTTTCCTTTGGATAGATAGCCAG TAGTGGGATTGCTGGATC |
| 50 | >SLC6A1::3:10992186-11039247;startsite=11010424 ;maxEnt=8.08(+) | CAAGGCTGTTTCCTTATCTGTCAGATGGGTGA GGTAGCCCTGGCTTACAT |
| 51 | >SLC6A1::3:10992186-11039247;startsite=11012288 ;maxEnt=8(+) | AGGCAGAAATATTTCTCCTTCTAGGCCATGA CCTTGACAAGGGCAAGGGT |
| 52 | >SLC6A1::3:10992186-11039247;startsite=11021265 ;maxEnt=8.02(+) | AGGCCGTGCTGATTGTATCTTCAGGGGAGTA CCTTGTAGAGATGTGTGTC |
| 53 | >SLC6A1::3:10992186-11039247;startsite=11010986 ;maxEnt=9.85(+) | CCTGGCCTCTCGGTCTCTGCCTAGGTCCCCAC CCCACGCAGCCGCCTGTC |
| 54 | >SLC6A1::3:10992186-11039247;startsite=11003518 ;maxEnt=10.44(+) | AAAACTGGGTGTCCTTGCCCCTAGGGAAGGA CAAATTTTCTTTAAGTCCC |
| 55 | >SLC6A1::3:10992186-11039247;startsite=11018284 ;maxEnt=7.93(+) | TGACTTTCTTTGCCTCGTGCTCAGTGCCTGAC AGGCACCCAGGTAGATAC |
| 56 | >SLC6A1::3:10992186-11039247;startsite=10993935 ;maxEnt=7.97(+) | TGGAGCGCTCATTCCCTTTCCCAGGAAGCTC AGCCTTATCCCCATGAAGA |
| 57 | >SLC6A1::3:10992186-11039247; startsite=11003436 ;maxEnt=8.89(+) | TAGGTGTTATCACTTCTGTTTTAGACAGAGAG AGTAGATGACTAACCTAC |
| 58 | >SLC6A1::3:10992186-11039247;startsite=11013708 ;maxEnt=8.6(+) | TATTATTATCTTCCCATTTTATAGATGAGGGT CAGAGAGGTGAAGTAACT |
| 59 | >SLC6A1::3:10992186-11039247;startsite=11010272 ;maxEnt=9.79(+) | AAAAATCAGCTTCTCGTTCCACAGGTCTTGA GTGGGGCCCAAGATTCTGC |
| 60 | >SLC6A1::3:10992186-11039247;startsite=11012201 ;maxEnt=8.71(+) | GCTGTATTAATGCGTTTCTTCTAGGCCTCCCG TGTCTTGTTCTTACACCT |
| 61 | >SLC6A1::3:10992186-11039247;startsite=11027398 ;maxEnt=8.42(+) | GGCTCTGTTCCACCTGGCCCACAGGCAGCCA GACGTTAAGGTTATCTCCC |
| 62 | >SLC6A1::3:10992186-11039247;startsite=11011094 ;maxEnt=7.54(+) | CCCCTTGCCTGCCATCTGGTCCAGGGCTGGG CTGCTCACAGCCAATCATC |
| 63 | >SLC6A1::3:10992186-11039247;startsite=11023244 ;maxEnt=8.27(+) | ACTCCACCTTTTCTCCCTTTCAAGCCCTACCC CAGGAGCCTGGGGGCAGA |
| 64 | >SLC6A1::3:10992186-11039247;startsite=11014323 ;maxEnt=8.36(+) | TCTAGTTTTCTCCTTCCTCAGCAGACCAAATC TCACTCTGAGTACAAGAT |
| 65 | >SLC6A1::3:10992186-11039247;startsite=11027465 ;maxEnt=9.35(+) | CTGTTATCCTGTTTTTTTTCCAAGGTGCCCAG ATTTCATATTGTTTAAAC |
| 66 | >SLC6A1::3:10992186-11039247;startsite=11011378 ;maxEnt=8.24(+) | CCCACTTCCTCCCATCCCACTTAGAATGAAAC CTGAATCCTTGCTGTGAC |
| 67 | >SLC6A1::3:10992186-11039247;startsite=11025669 ;maxEnt=9.61(+) | GCATCTTTCTGACCCTCACTGTAGACCAGGTT TGTTGCCAGGGAGAGCTG |
| 68 | >SLC6A1::3:10992186-11039247;startsite=11006369 ;maxEnt=7.84(+) | GACCTGGGCCCCGTTCTTGCATAGGTGACAG TGCAGCTGGGAAGCTAAGA |
| 69 | >SLC6A1::3:10992186-11039247;startsite=11031715 ;maxEnt=8.13(+) | CTGCACCTTTGATTGTCCCATCAGAGCAAAT GTTTTTAAAGAAGCATGAT |
| 70 | >SLC6A1::3:10992186-11039247; startsite=11015440 ;maxEnt=8.64(+) | GCTGCCCTCATCCCACGCCCACAGCTGTCCC GAGGGCAGCGGGCCCCACT |
| 71 | >SLC6A1::3:10992186-11039247; startsite=11007633 ;maxEnt-11.03(+) | AAGCTAACTGCCCTTCCTCCTCAGGTCAGCTC CTCGCAGCAGCTGCAAGG |
| 72 | >SLC6A1::3:10992186-11039247; startsite=11009538 ;maxEnt=9.83(+) | CTGGGTTTTTTTCTTCATCTATAGAATGCCAT GGTAGACCAGGTGCACCA |
| 73 | >SLC6A1::3:10992186-11039247; startsite=11009362 ;maxEnt=8.03(+) | CTGCTTCTGCAAATTCCCTCTCAGGTACGTTG AGGCAGCTGAGGGGTTTA |
| 74 | >SLC6A1::3:10992186-11039247;startsite=11008143 ;maxEnt=7.54(+) | CACTTTAACCTCTCTGTGCCTCAGTTTACCCA TCAATAAAATGGGGGCTA |
| 75 | >SLC6A1::3:10992186-11039247; startsite=11006520 ;maxEnt=7.77(+) | ATCCTTGCACCATCCCTGTTACAGCAACCTCC ACCCATGCTCCCTCTCCT |
| 76 | >SLC6A1::3:10992186-11039247; startsite=11020973 ;maxEnt=7.72(+) | TCTCTGCCTCTAATCTCCTGCCAGCTCCTCCC AATGGTCAAACCCAGCTA |
| 77 | >SLC6A1::3:10992186-11039247; startsite=11011532 ;maxEnt=8.06(+) | GTGCCAGGCTCCTTCCTGCCTCAGGGCCTTTG CACTTGCTGCTCCCTCTG |
| 78 | >SLC6A1::3:10992186-1103 9247; startsite=11011985 ;maxEnt=10.27(+) | ATCAGCCCTGCCCCATCCCTGCAGCTAGTCCC CAGGTTCCTTAGTCCGGT |
| 79 | >SLC6A1::3:10992186-11039247; startsite=11009833 ;maxEnt=7.67(+) | CAGGGAGCTTTCCCTGACCTCCAGGACAGCG CGTGGCAAGCACTGCCCAC |
| 80 | >SLC6A1::3:10992186-11039247; startsite=11033989 ;maxEnt=8.03(+) | TATTGTCCCACTTTACCGCTGCAGGATCTGGG GCTCACCTAGCCAGCATC |
| 81 | >SLC6A1::3:10992186-11039247; startsite=11002235 ;maxEnt=8.56(+) | GAATGTGTGTTTTCTCTGTGCCAGCTATCTAA ATGACCCCATGCTGCAAA |
| 82 | >SLC6A1::3:10992186-11039247;startsite=10996585 ;maxEnt=7.73(+) | GCTGTGTGCTTTATATCGTTGCAGTTAATTTT CACAAAACCCTGTGAGAT |
| 83 | >SLC6A1::3:10992186-11039247; startsite=11002919 ;maxEnt=8.57(+) | TTCCTTTATATTGATTGCCTATAGGTTAAGAT AACACTGGGCCTGGCGCA |
| 84 | >SLC6A1::3:10992186-11039247;startsite=10993277 ;maxEnt=9.29(+) | CGAAGCTCCCTATTCATTCCCCAGGGCATGG AGGGGACGCGGAGTGAATG |
| 85 | >SLC6A1::3:10992186-11039247;startsite=11005217 ;maxEnt=7.83(+) | TGGGCACCCGGACCTGTTCCACAGGGGCTCG CCCTCGTGCCCAGCACAGG |
| 86 | >SLC6A1::3:10992186-11039247;startsite=10995733 ;maxEnt=9.08(+) | GAAGTCCATCTCCTTTGTCCTTAGAAGCCCAT CCCTGTTGCCTAGCCCAA |
| 87 | >SLC6A1::3:10992186-1103 9247; startsite=11004115 ;maxEnt=9.68(+) | TACACGCCTCCCTCATTCTTGCAGACTATTCT AAAGCAGAGTCTCTCTGC |
| 88 | >SLC6A1::3:10992186-11039247; startsite=11005822 ;maxEnt=7.69(+) | GGTTCGATGTTCCTCATCCTGCAGCAGACGTC TCTGCGGGCACCCACCAG |
| 89 | >SLC6A1::3:10992186-1103 9247; startsite=11011646 ;maxEnt=9.18(+) | TTTTTTTTTTTTTTTTTTGCTCAGGCCAAATAA AACAAGCCCAAGGGCCA |
| 90 | >SLC6A1::3:10992186-11039247;startsite=11015607 ;maxEnt=9.95(+) | CTGCACCCCTGCTGCCCTCTGTAGGAGCTGCC TGCCTGCCCCATCGCTGC |
| 91 | >SLC6A1::3:10992186-11039247; startsite=11010899 ;maxEnt=8.83(+) | GTCCTCGATTCCCTGGCTTTTCAGGGCTCCCC ACTCACTCCATGGCGGGG |
| 92 | >SLC6A1::3:10992186-1103 9247; startsite=11016969 ;maxEnt=9.36(+) | CAAAAGTCACCTTCCTTCCTCCAGGAGCAAG GGCGGGTGAGAGGAAGAGG |
| 93 | >SLC6A1::3: 10992186-11039247;startsite=11037771 ;maxEnt=8.21(+) | GATCTCTTTTTATCGCCATTCCAGGGGCCTCA GGTCCTACTGGGGAAACT |
| 94 | >SLC6A1::3:10992186-1103 9247; startsite=10998146 ;maxEnt=8.85(+) | TGACTCCTCATCTCTGTCCCCTAGTTTCCCAG CTGATGAAAATCACCTTT |
| 95 | >SLC6A1::3:10992186-11039247; startsite=11003262 ;maxEnt=8.32(+) | AACACTGGCCTGCCTTCCACCCAGGACTTTTT CCCATCACTAACTAAAAA |
| 96 | >SLC6A1::3:10992186-11039247;startsite=11028119 ;maxEnt=7.52(+) | TCCGGTTCTAATCTCCCCTTCAAGGGCAGCCA CCATCTCGTTTCTCTGCA |
| 97 | >SLC6A1::3:10992186-11039247; startsite=11037369 ;maxEnt=7.54(+) | TGACTGTTATCTCGGACTTTGCAGGAGTTCCT TTCCCTCCGAACGCTGCT |
| 98 | >SLC6A1::3:10992186-11039247; startsite=11032986 ;maxEnt=9.32(+) | CGAAGTCTCGCTCTTGTTCCCCAGGCTGGAGT ACAATGGCACGATCTCGG |
| 99 | >SLC6A1::3:10992186-1103 9247; startsite=11037267 ;maxEnt=7.93(+) | GGTAGATCATTTTTATCCCGCCAGGGAGTGT GATGCAGGAAGACCACATG |
| 10 0 | >SLC6A1::3:10992186-11039247; startsite=11018437 ;maxEnt=7.6(+) | AGTCCTGGCCCCCTGGCTTGTCAGATGTACAT GACCTTCAGCAAGTCACT |
| 10 1 | >SLC6A1::3:10992186-11039247; startsite=11001422 ;maxEnt=8.11(+) | TCTCTACTTCCATCCTTCCTACAGCCTTGTCA GAATGGGATACTTCCCTG |
| 10 2 | >SLC6A1::3:10992186-11039247; startsite=1103 5701 ;maxEnt=8.47(+) | TGGCCCATCTTCCTCCAACCTCAGATGAAGA AACTGGGGAACCACAGGGG |
| 10 3 | >SLC6A1::3:10992186-1103 9247; startsite=11026090 ;maxEnt=8.54(+) | TCTTCTGGCTCTGCCCTCCTCTAGCTTGCTCTT AGGGCCTGTGCATCTGG |
| 10 4 | >SLC6A1::3:10992186-11039247;startsite=10997049 ;maxEnt=7.66(+) | CCTAGATTTTAAATTCATTTGCAGCTAGTGCT GATGTCATGCACTCAGCC |
| 10 5 | >SLC6A1::3:10992186-11039247;startsite=11015791 ;maxEnt=7.83(+) | AGTGGAGTTCTCTCTCTTAACCAGGTTGGCG AAAAGCACTCTTGCAGCGA |
| 10 6 | >SLC6A1::3:10992186-11039247; startsite=11000356 ;maxEnt=8.27(+) | GGATGCTGTTCCTGCCCTTTGAAGATCCACTT GCATTGAAAACCGTAGAT |
| 10 7 | >SLC6A1::3:10992186-11039247;startsite=11008549 ;maxEnt=8.67(+) | CTTCCATTTATTCATCTGTCCTAGGACAGTGA GGCTAGGCAAACTCATCT |
| 10 8 | >SLC6A1::3:10992186-11039247;startsite=11034251 ;maxEnt=8.07(+) | CTCACCCTTGCTCTGCTTTTGAAGCTGGGGAA CTTGGAGGAAGGGGTTCC |

**Table 3 - Sequences and chemical modifications of selected steric blocker antisense oligonucleotides targeting cryptic splice sites within SLC6A1 introns (showing compounds that activate the expression of SLC6A1 mRNA in KNS60 neuroblastoma cells.)**

| **Oligo Name** | **Oligo Sequence** | **Molecular Weight (Da)** | **Target Site** | **Percent Activation** |
|---|---|---|---|---|
| SLCss0603 | TCTTGCAGACTATTCTAAAG (SEQ ID NO: 110) | 7933.92 | Intron 1: 11004094 - 11004117 | 102.81 |
| SLCss0701 | AGGGTGGGCACCCGGACCTG (SEQ ID NO: 111) | 8054.98 | Intron 1: 11005196 - 11005219 | 120.84 |
| SLCss0801 | GGAAGACCTGGGCCCCGTTC (SEQ ID NO: 112) | 8003.97 | Intron 1: 11006348 - 11006371 | 209.24 |
| SLCss0802 | TGGGCCCCGTTCTTGCATAG (SEQ ID NO: 113) | 7970.93 | Intron 1: 11006348 - 11006371 | 136.17 |
| SLCss0803 | TTGCATAGGTGACAGTGCAG (SEQ ID NO: 114) | 8025.94 | Intron 1: 11006348 - 11006371 | 123.55 |
| SLCss0901 | TGATCATCATTCTTATTACA (SEQ ID NO: 115) | 7883.89 | Intron 1: 11006831 - 11006840 | 138.59 |
| SLCss1001 | GCTTCTGCAAATTCCCTCTC (SEQ ID NO: 116) | 7877.93 | Intron 1: 11009358 - 11009367 | 180.25 |
| SLCss1002 | AATTCCCTCTCAGGTACGTT (SEQ ID NO: 117) | 7913.92 | Intron 1: 11009358 - 11009367 | 228.55 |
| SLCss1101 | GTTTAAAAATCAGCTTCTCG (SEQ ID NO: 118) | 7933.92 | Intron 1: 11010251 - 11010274 | 114.56 |
| SLCss1301 | TCTGGCTGTATTAATGCGTT (SEQ ID NO: 119) | 7957.89 | Intron 1: 11012180 - 11012203 | 101.13 |
| SLCss1302 | TATTAATGCGTTTCTTCTAG (SEQ ID NO: 120) | 7916.88 | Intron 1: 11012180 - 11012203 | 257.13 |
| SLCss1303 | TCTTCTAGGCCTCCCGTGTC (SEQ ID NO: 121) | 7909.92 | Intron 1: 11012180 - 11012203 | 113.69 |
| SLCss1401 | CCGTGTCTTGTTCTTACACC (SEQ ID NO: 122) | 7894.91 | Intron 1: 11012213 - 11012236 | 121.89 |
| SLCss1402 | TGTTCTTACACCTCTTGCAG (SEQ ID NO: 123) | 7904.91 | Intron 1: 11012213 - 11012236 | 160.77 |
| SLCss1403 | TCTTGCAGCACTTATCACAG (SEQ ID NO: 124) | 7922.93 | Intron 1: 11012213 - 11012236 | 174.72 |
| SLCss1501 | CTTCCGCCTGCTCCACCAGC (SEQ ID NO: 125) | 7890.97 | Intron 1: 11012794 - 11012803 | 145.5 |
| SLCss1502 | GCTCCACCAGCAGGTAAAGG (SEQ ID NO: 126) | 8006.99 | Intron 1: 11012794 - 11012803 | 152.72 |
| SLCss1503 | AGGTAAAGGAGGCTGATCAC (SEQ ID NO: 127) | 8043.97 | Intron 1: 11012794 - 11012803 | 169.43 |

**Table 4 - Genomic coordinates and sequences for novel cryptic splice sites in a representative list of genes known to be involved in human haploinsufficiency disorders. These sites were identified solely in nascent pre-mRNA derived from metabolic labeling high-throughput sequencing data. These cryptic 5' or 3' splice sites were found in at least one cryptic splice junction, and sometimes multiple cryptic splice junctions, in the nascent RNA sequencing data**

| SEQ ID NO: | Gene | Chromosome | Genome Coordinate | Type of Cryptic Site | Genomic Sequence |
|---|---|---|---|---|---|
| 128 | CHD7 | 8 | 60804104 | 5' splice site | TAAAACTCACCTGGCAGAGGC ACCGTAAGTCAGCACCTCATGT TAAATAGGGGTGGCTCAAGCA TTTATGTTGTTCTAAACCCAGG TTAATGTCTATGATACCTAGTT GCATGAAATAGTTGGTG |
| 129 | CTNNB1 | 3 | 41236710 | 5' splice site | ACAGAGCCAATGGCTTGGAAT GAGGTAGGGAAATGTGAGCAG TTATTTATCTGGTAGTTTCCTA GAGCAGGTATGGCAGCTTGTT CTTTCCTCTCAAAACACTTAGT ACACATTCATTTGCATTG |
| 130 | EHMT1 | 9 | 137653548 | 5' splice site | CTCTTTCTTTTTTTTTCTTTTG AGACAGAGTTTTGCTCTTGTCG CCCAGGCTGGAGTGCAGTGGT GTGATCTCAGCTCACTGCAACC TCTGCCTCCCAGTTTCAAGCGA TTCTCCTGCCTCAGC |
| 131 | EHMT1 | 9 | 137619050 | 5' splice site | GCCATGGCCGCCGCCGATGCC GAGGTGAGCAGCGGGGCCGGC GGGGGGCGGCGCGGGGGCGGC GGGCAGCGGCGGAGGCGGCGC GGGGGCGAAGAACCGGGCGGG GCGGCGGCAGGCGGCCGGCG |
| 132 | EHMT1 | 9 | 137629024 | 5' splice site | GTTGATGGATTACTTTATCCTA AGGTAAGTGGGCAGTTTGATTT TAAGTTGCTCACTCAGCAAACC AGGTAGGGAATAGTTATTTGCT TTTGTATTTTTTTTTTTTTTTG AGATGGAGTTTCGC |
| 133 | EHMT1 | 9 | 137720023 | 5' splice site | CCTCCACACCAGGACACAGTC GAGGTGCCGAACCTCCTCCAC ACCAGGGCACAGTCGAGGTGC CGAACCCCCTCCACACCAGGG CACAGTCGAGGTGCCGAACCC CCTCCACACCAGGGCACAGT |
| 134 | EHMT1 | 9 | 137782175 | 5' splice site | GGGCGGGTTCCGGCGTGGCTC GAGGTGGCTGTTTATGTGGAG GATGGTCATTTGTGAGTGCTTG CCAGCCATCGTGACAGTCCTG AGCTGGAGTCTGTGGCTACATC TGAAATCATTAATAAAAC |
| 135 | EHMT1 | 9 | 137818546 | 5' splice site | GCCATGTACCGAGACTGTAGA GAGGCCGACTGAGGGGCGCCG TGTACCGAGACCGTAGAGAGG CCGATTGAGGGGCGCCATGTA CCGAGACCGTAGAGAGGCCGA CTGAGGGGCGCCGTGTACCG |
| 136 | EHMT1 | 9 | 137669456 | 5' splice site | CGCCGCCCACAGCACGTGCAC TCACCTCCACCCAAGACGCCCC CCACAGCACGTGCACTGGACT CCTCCCAAGACGCCCCGCACA GCACGTGCACTCGACTCCTCCC AAGACGCCCCGCACAGCA |
| 137 | EHMT1 | 9 | 137669567 | 5' splice site | CGCCCCGCACAGCACGTGCAC TCACCGACTCCACCCACTGTCT ACCCAGGGCGCAGCCGCTCCA GCAGCTCTTCCCTGGCCCCTCC CCCACCTTCACTTTTTGCTCTCT TCGGAGCATTCATTGC |
| 138 | EHMT1 | 9 | 137721201 | 5' splice site | CCCCTCCCAGACTTCTCACACT CACCCCTCCCAGACTTCTCACA CTCACCCCTCCCAGACTTCTCA CACTCGCCCCCTCCCAGACTTC TCACACTCACCCCTCCCAGACT TCTCACACTCACCCC |
| 139 | EHMT1 | 9 | 137721274 | 5' splice site | CCCCTCCCAGACTTCTCACACT CACCCCTCCCAGACTTCTCACA CTCACCCCCTCCCAGACTTCTC ACACTCACCCCTCCCAGACTTC TCACACTCACCCCCTCCCCTCT CACCCCCTCCCACGC |
| 140 | EHMT1 | 9 | 137721298 | 5' splice site | CCCCTCCCAGACTTCTCACACT CACCCCCTCCCAGACTTCTCAC ACTCACCCCTCCCAGACTTCTC ACACTCACCCCCTCCCCTCTCA CCCCCTCCCACGCCTCACCCCC TCCCACGCCTCTCAC |
| 141 | EHMT1 | 9 | 137721212 | 5' splice site | CTTCTCACACTCACCCCTCCCA GACTTCTCACACTCACCCCTCC CAGACTTCTCACACTCGCCCCC TCCCAGACTTCTCACACTCACC CCTCCCAGACTTCTCACACTCA CCCCCTCCCAGACTT |
| 142 | EHMT1 | 9 | 137721261 | 5' splice site | TTCTCACACTCGCCCCCTCCCA GACTTCTCACACTCACCCCTCC CAGACTTCTCACACTCACCCCC TCCCAGACTTCTCACACTCACC CCTCCCAGACTTCTCACACTCA CCCCCTCCCCTCTCA |
| 143 | EHMT1 | 9 | 137721310 | 5' splice site | TTCTCACACTCACCCCCTCCCA GACTTCTCACACTCACCCCCTCC CAGACTTCTCACACTCACCCCC TCCCCTCTCACCCCCTCCCACG CCTCACCCCCTCCCACGCCTCT CACCCCCTCCCACGC |
| 144 | EHMT1 | 9 | 137721334 | 5' splice site | CTTCTCACACTCACCCCTCCCA GACTTCTCACACTCACCCCCCTC CCCTCTCACCCCCCTCCCACGCC TCACCCCCCTCCCACGCCTCTCA CCCCCTCCCACGCCTCTCACCC TCTCCCACGCCTCTC |
| 145 | EHMT1 | 9 | 137721323 | 5' splice site | CCCCTCCCAGACTTCTCACACT CACCCCTCCCAGACTTCTCACA CTCACCCCCTCCCCTCTCACCC CCTCCCACGCCTCACCCCCTCC CACGCCTCTCACCCCCTCCCAC GCCTCTCACCCTCTC |
| 146 | EHMT1 | 9 | 137818817 | 5' splice site | GTACCGAGACCGTAGAGAGGC CGACTGAGGGGCGCCATGTAC CGAGACCGTAGAGAGGCCGAC TGAGGGGCGCCGTGTACCGAG ACCGTAGAGAGGCCGACTGAG GGGCGCCGTGTACCGAGACC |
| 147 | EHMT1 | 9 | 137819425 | 5' splice site | GTACCGAGACCGTAGAGAGGC CGACTGAGGGGCGCCATGTAC CGAGACCGTAGAGAGGCCGAC TGAGGGGCGCCGTGTACCGAG ACCGTAGAGAGGCCGACTTAG GGGCGCCGTGTACCGAGACC |
| 148 | EHMT1 | 9 | 137721374 | 5' splice site | CCTCCCCTCTCACCCCCTCCCA CGCCTCACCCCCTCCCACGCCT CTCACCCCCTCCCACGCCTCTC ACCCTCTCCCACGCCTCTCACC CTCTCCCACGCCTCTCACCCTC TCCCACGCCTCTCAC |
| 149 | GRN | 17 | 44352619 | 5' splice site | GGCTGGGTATGGCCAGGGACC AGGTCCCACCTCGTCCAACCCT CTCGCCCCCCTCTGACCATCCA GGGCGTCTGTTGTGCTGATCGG CGCCACTGCTGTCCTGCTGGCT TCCGCTGCGCAGCCAG |
| 150 | GRN | 17 | 44349177 | 5' splice site | TGCAGGCAGACCATGTGGACC CTGGTGAGCTGGGTGGCCTTA ACAGCAGGGCTGGTGGCTGGA ACGCGGTGCCCAGATGGTCAG TTCTGCCCTGTGGCCTGCTGCC TGGACCCCGGAGGAGCCAG |
| 151 | GRN | 17 | 44351461 | 5' splice site | TGGGGCTGCTGCCCTTTTACCC AGGTACCCAGGGGTGGCGGGT GGGTGGGCTGAGCACAGTGTG GCAGGCAGCCGGGCCCCAGTG CCCACCTGCCCTTCTTCATCTG CCCTAGGCTGTGTGCTGT |
| 152 | HTR7 | 10 | 90810092 | 5' splice site | GGTATTGACGGCCAGGTTTCTA AACCTCTTAAAACTCCCCAACT CTGGTGCCACCTTAGACAATAC TCTTTTAAGCACTCCTTTTTAG TTATCCCCACCTGCCCAGTTCC CTTATTAGGCCAAGA |
| 153 | JAK2 | 9 | 5055789 | 5' splice site | CATAAGCAAGATGGTAAAAAT CTGGTAAGTTTGCTTTATGATT GAATAATGGTTTCATTTTATAG TTCTCAGAAATGTGTATTTTAG AATCTTAGTACCAAAATTATTT TCTGGTAGGAATTTTG |
| 154 | KCNQ4 | 1 | 40802257 | 5' splice site | TGTGAGGTGGAAGGCGAGGC CGAGGCGGCGGTGGCGTCGCG TCCCCCCTCTCCGCCCCTGGCC TCGGGGACGCGGCGGCACCTT CACACTTTCCGCTTGGCCCGCT GCTCGCCTTTCAGGCCGC |
| 155 | KCNQ4 | 1 | 40822403 | 5' splice site | TGACAGTATCCTCCCATCCTTC AGGTAGGTCCTGCTGGGGGTG GGGGTGGGTGGGGGGCTGGCA GCAATGCCCTTTGAGGACAAG TGGCTGAGACTCAACCCTGGA GGGTGGAAAGGGGGTGATG |
| 156 | LEPR | 1 | 65425379 | 5' splice site | GGGATGTGCCTTAGAGGATTA TGGGTAAGTTATCATTTCAAAA AGAACTATTCCTCTTTCTGTGT CTTTGTCACTATTAGTATGGGT GTTAGAGAGTTCGGTCAATTTA GCACCAAGTTCTAACC |
| 157 | LEPR | 1 | 65616235 | 5' splice site | GCCTATGAGCAAAGGTAAGAA GAGGTACAGAGTGGTAATCCA TTGCCTCTTTTAATATTTAACTT TTGCAAACTCTCCTATTTTAAA TTATCTTTCAAGCAGCCTGCAA TTCTCTGCATCTGAAA |
| 158 | LEPR | 1 | 65525690 | 5' splice site | TCCTCTCCTGAGAGTTGCCCCG CACCTTGGGCGAGGAGTTCGG AGCGGCCCCATCGCAGAGCCC ACGGCCAGCCGAGCGCGCGCG ACGCAGGTGCCCGAGCCCCGG CGCCGCCGCCATCTCTGCC |
| 159 | LIPC | 15 | 58520257 | 5' splice site | AAACCTAAAAGCAATTTTGTTT GGGTTGAAACAAGGTGGGCGG GCAGTCAGGAACCCACCCTTTT AACTTCTGTGTCCTCTGCCATC TCCTGTAGCAGCCCCCAAAGT AGTTCCACAAGATCCTT |
| 160 | MBD5 | 2 | 148089610 | 5' splice site | GAATGATCTTTGGGTCAACAAT GAAAACAAGATGGAAGTAAAA AATTATCTGAACTGAATGATA ATAGTGACACAACTTATCAAA ACCTCTGGGACACAGGAAAAA TGGTGCTAAGAGGAAAGTT |
| 161 | MBD5 | 2 | 148021047 | 5' splice site | ACCCGTCAGAGAGGGACATGC GCAGTGAGTGCCTCCCGTCTCT TCTACCCGAACCCCCCCTCCCC CCCAAGCAGAGAGACCCCAGC AGCAGCAGCAGCTGATGATGA AGAGAGAGGCAGTGGCAG |
| 162 | MBD5 | 2 | 148458872 | 5' splice site | TCAAAATGGAGTGCTTTATGTC AGGTAAGTTCTTATTATTACCT GTGGTACCTGCAAAGTTGTACT CCAAAGACTAAGGAGAGAACC AAGCATGGTGACTGATGGCAC CTCATATAAATGTGAAA |
| 163 | MBD5 | 2 | 148468947 | 5' splice site | TGTTCCACCACAAACCACCCCA AGGCCCACCTCCCCCTCCTCCA CCTTCTTGTGCTCTTCAGAAAA AGCCATTAACATCTGAGAAAG ATCCACTTGGCATTCTTGACCC TATTCCTAGTAAACCA |
| 164 | MNX1 | 7 | 156999923 | 5' splice site | CCCGGGCGCGCATTGTCCGC ACGGTGTGTACTCGGGGAGGG GCGCCAGGTGCGCGGGCGGAA TGTAGGGTCGGCGCGGGGCCG GGGGGCGGGCCCGGGCCGGG GGGTCCTCAGGAAGCCTCGG |
| 165 | NFIA | 1 | 61112344 | 5' splice site | TGATAACAAGAAATTAAAATC AAGGTACAGTATCATTTATATG TAGTAGTACAGAGTAACCATG ACTTACTAGGTGTTATGATGAA GGTGTATGTGTGTGTATATGTG TGCATGCATGTAGATAA |
| 166 | NFIA | 1 | 61277586 | 5' splice site | ACATTAAGGACCAGCCAGAAA ATGGTAAGTTTAGCTTGGGACT CTAGCTGCTGCTTTCAGAGTCC ACAGCAGCCAGCAGGCCGACT AAGTGTGAGGATTTGGGGGCC TACCCTATAAGTAGCCCA |
| 167 | NFIA | 1 | 61352568 | 5' splice site | TTTACCCAGCACATCCTCTACG AGGTAATTTTATTGGCAGCTCT TGAAGAAATTATGCTACATGG TTGCACACCTTGATTTTAACTC TGGGCCCACTATGGATTTAGCA ATGCGCCTTTAGTAAT |
| 168 | NMU | 4 | 55607436 | 5' splice site | TATAGATGTATGAAAATCATCT TACCCTTTTAGTATTATCTTTTT CATCTTGTTCCTATTGAAAAGA GATATTGTATATATCATTATAT ATTGTAAAATTTTACTATCTTA TATACAGTAATTTT |
| 169 | NOTCH1 | 9 | 136545725 | 5' splice site | GGCTCGCGGGTGGGTGGGCGC CTACCTCGTGCGGCGAGCGCG GGCAGCAGCGCCAGGCAGAGC AGGGGCGCCAGGAGCGGCGGC ATGCCTCCCCACCGGCTGCCCT CTGCGCCCGGGCGGCGGCC |
| 170 | NOTCH1 | 9 | 136535683 | 5' splice site | GTGCAGGGTGGGTCGGGGGA GCACTCAGGATCCCTCCCAGG GGCAATGGGTGCAGGGTGGGA GTGGGTGGAGGGGGGAGCACT CAGGATCCCTCCCGGGCAAT GAGTGCAGGGTGGGTGGAGA |
| 171 | NOTCH1 | 9 | 136542751 | 5' splice site | GCTGGGGTTGTCAAGGGAATG GGACCTTCTGCAGTTGGAATG AGGGCGCGCAGACTGGCTGGG GAGCCATTTCTTCATCTGTAGC TCCTGGGGGGCGGAGGGAACT CTTTTTCCAGACACCAAAC |
| 172 | NSD1 | 5 | 177254432 | 5' splice site | TTTTTAATCTGCGACAGAGTCT CACTCTGTCACCCAGGCCGGA GTGCAGTGGCGTGATCTCAGCT AACTGCAACCTCCACCTCCTGG GTTCAAGCGATTCTTGTGCCTC AGTCTCCCGAGTAGCA |
| 173 | NSD1 | 5 | 177147361 | 5' splice site | GCTCAAGCAATCCGCCTGCCTT GGCCTCCCAAATGGCTGGGAT TACAGGCAGGAGCCACCATGC CTGGCCTAGCCCCTCCATCTCT AGCCTTTGTCAGTTACTAAACT TTTTTTCCTGAAGTTTT |
| 174 | PAX6 | 11 | 31812498 | 5' splice site | GTTGCCAGAAAACCGGGGAGG AAAGTAAGTAAATGGCTTTTCT CTTCTGACCACACACCAGAAG TCCATTTGTTGAATGCCGCACG ATTAGGACACACCTCAGTACA CTCCAAAGGCGCCCCTCG |
| 175 | PHIP | 6 | 78958474 | 5' splice site | AATTACATATGAAAAGATAAC TTACTTTATAAAATGTAGAAGA AGATCAGTCACGAATTTAGCA GATTTCACAATAGGGCTTCCAG GCTCATTAAATGTTCGTGTATT ATGCTCTATATATCGAA |
| 176 | PKD1 | 16 | 2105000 | 5' splice site | GGGGAGGAGGGGAAGGGCTA GGGGAGGGGAGGAGGGGAGG GGCTAGGGGAGGGAAGGGGG AGGGGAGGGGAGGGGAGAGT GGAGGGCACAGAGCAGCATCT TCTTAGTCCCTCCCCACATCTG GG |
| 177 | PYY | 17 | 43975248 | 5' splice site | CTTTCCTTTCCACATACTTACA AACCTAGGGGTGGTGGAAAGG GTATGGATTCCGGAGTCAGAT GTTCTGGGTTTGAATCCCACTC GCATTTCTTAGCTGTGTGACTT TGAGAGAGTTTGCTTGG |
| 178 | RAI1 | 17 | 17787512 | 5' splice site | TGATCAGTACAGAACTGATAA TAGGTGAGCTCCTCCCGGCAG CCTTCCCTGGGGGGCCTAGGA ATGCCTGCAGCTGCTGTGGAAT CCCATGTTGCTCCCCCACACCT CACAGTGGAGGTGGGACT |
| 179 | RBPJ | 4 | 26326229 | 5' splice site | GTATTTGACTTTGGTTCTGTAA GGAGTAATTGCTGAAGTACCC AGAGGAATTGATATTTTCAGCC ACAAGCAATCTCATAGCCCCT GAGAACATTATAGTAAGGCTC TTAGGCAAAAGTTTTTTT |
| 180 | RBPJ | 4 | 26320816 | 5' splice site | GCCTGCGCATGCTCCATCGCCT GGGTAGGTTTCCAGGGAAGGC AGCGAGCAGGATCCCCTACTC TGCGGGCGGCGCGAGGCGTCT GGCTCTTCGCGGCGGCGGCGA GGGGAAAGGGAGCGCGGGG |
| 181 | RBPJ | 4 | 26321049 | 5' splice site | GAAGATGGCGCCTGTTGTGAC AGGGTAAGTCTGAGGGAATCG GAGCGCCGGGAACCGGGAAAG TTGCGGGCGTCTGGCAGCTCAC GGCGGGCAGCGGGTTCGGGGG CCGCGGCGCGCTTGGCGTT |
| 182 | RBPJ | 4 | 26367901 | 5' splice site | ACAGTGAAAAGATGAAGACTT CTGGTAAATTGTTTCACAGCAG TGGAATTTGTACATTAGTTTTC TACACTTGGGAGTTGAAAAAT GGAAGAATTATTTGCCAAAAG AGGAGGACAAAAGATAAT |
| 183 | RPS14 | 5 | 150445608 | 5' splice site | GCATTAGCTAGAGGGGGCAC TTACCAATCCGCCCGATCTTCA TACCCGAGCGGGCAAGGGCTC TGAGGGCCGACTGGGCCCCAG GTCCAGGGGTCTTGGTCCTAGA AAATGAAGGTTTAAGTTA |
| 184 | RPS14 | 5 | 150445629 | 5' splice site | TTACCAATCCGCCCGATCTTCA TACCCGAGCGGGCAAGGGCTC TGAGGGCCGACTGGGCCCCAG GTCCAGGGGTCTTGGTCCTAGA AAATGAAGGTTTAAGTTAAGA AGAGCCTTTGGCCAAGTC |
| 185 | RUNX2 | 6 | 45328457 | 5' splice site | CTACCAGCCACCGAGACCAAC AGAGTCAGTGAGTGCTCTCTA ACCACAGTCTATGCAGTAATA GTAGGTCCTTCAAATATTTGCT CATTCTCTTTTTGTTTTGTTTCT TTGCTTTTCACATGTTA |
| 186 | RUNX2 | 6 | 45438052 | 5' splice site | TAGATGGACCTCGGAACCCA GAAGTAAGTACTCCCCTTTTTA TTGAAGAAAGTAATAGAGTTT CCAGAGACCCTATGAGGAATT TATTCCAAATGAGTTAGTGTCA CTTTCATGTCCATAGTGT |
| 187 | RUNX2 | 6 | 45492963 | 5' splice site | GAGGTGGACTAGAATAGAGGG AAAGTAAGTCAAAACAAAATG TGTAATAAGCCCTCAACATGG ATCTTTTTTATGGTTAGATTTTT GTTTATAGTAGTTAGGCTCGAA TGTTCTTAATGGTCTCA |
| 188 | RUNX2 | 6 | 45377503 | 5' splice site | CCAGCCCCCCTCACCCCACGCC CACCCGCCAAAAGGGAGGAGT CGAGAGGCAACTCCACGCTCG GCAGCGGAACCAGGGCGCTGC GCCCGGCCAGCGCCTCCCACA GCCGCCCTCCCTTCAACAC |
| 189 | RUNX2 | 6 | 45421844 | 5' splice site | CCGAGATCCGCTGCCCACGAC TTACCTCTTGGCACCTTGAAGC GCGAGAGACAGGGGAGCTTTG CAAATTGCAGGAGGGAGTGGA TGCAAAGAGGTGGCTGTGAGA GGGCGAGAAGAAAAGAAGG |
| 190 | SETBP1 | 18 | 45063890 | 5' splice site | TTGGGAAAGCAAAGCAGGGAG ACACCTTCAGAAGAAGCTTGT CTGAGCTTCACCGCAGCTCCCA CCACGCGGCGCTTCAGTACGG CTGGATCCTCCGCAGGCGAGC GGAAGGCCCCCAGGAGGAG |
| 191 | SETBP1 | 18 | 45063576 | 5' splice site | CGAGGCGGAAAGAGGAAACAC AAACCGCAGGCCCCCGCTCAG CCCCCACAGCAGTCGCCCCCG CAGCAGCCCCTTCCCCAGGAA GAGGAGGTGAAAGCCAAAAGG CAGAGGAAGTCCCGAGGGAG |
| 192 | SETD5 | 3 | 9431826 | 5' splice site | CATGCAACTTTCCTTTTTCCTG TCCCCCTCCCACCAAAAAAAA ATCCATTCCTAATAAACAGCTA TCATGTTTCACTCCATGTGTTC AGTGGTACAAAACCTATATAA AATATGTACAGCTCTTT |
| 193 | SETD5 | 3 | 9446005 | 5' splice site | TTTTTTTTACTAACAATCTGGT TTCCGCTGGGCGCGGTGGCTCA CGCCTGTAATCCCAGCACTTTG GGAGGGCGAGGTGGGCGGATC ACGAGGTCAGGAGATCGAGAC CATCCTCGCTAACACGG |
| 194 | SETD5 | 3 | 9426133 | 5' splice site | AGGCTTAGAAGATGTACTGCC AAGGTCTCTGTTTGGATATACT GTTAGGGAAAGGAAACATACA TGTGTAAGACTGGCCTAAAGG CAAAAAAGTTCATCAGTCCCTT TTTTTTTTTTTTTTTTTT |
| 195 | SETD5 | 3 | 9443418 | 5' splice site | ATTTGATTATGAGTATAGTAAC TGGTAAGACCTCAGAAACCTTT CCTAACAGGAATATCCATGTCT TACATATTAAGCTATTCACTCT ATTTAAAGTCTGCTCTTGTTTC TTGCCTTTTCCTTTG |
| 196 | SETD5 | 3 | 9445741 | 5' splice site | GAGAACCTAGCTCATAGCAGG AGGGTGAGTACTGTCTGACATT ACTTTGCTCCTTCTCATTCCTG CTACCTCCATCATGTGAACCTC TTCTGTTCTCTTGACTTTGTATC ATCTCATTGATTTGA |
| 197 | SETD5 | 3 | 9475157 | 5' splice site | AGCCCTTCCAGATACAGCTACC AGGTGAGATGAGAAATTGCTG GTCTCTAGCCATAGGAGTGTGT TCTGGGTCCCAAATTGTCCTGG TCATCCTTTGCCATTGAGATGC TGTCTTTGCATATAGT |
| 198 | SHANK3 | 22 | 50706153 | 5' splice site | TGACAGCTGAGCTCGAGGAAC TTGGTGAGTGGCGGGGGTGGC GGTGGAGGTGGACGCAGGTGG ACGGTCCATGATGGGCAGACA GGGCCGGGAGACACAGGTGGT ACGGGAGGGCAGGCGGTCCA |
| 199 | SYNGAP1 | 6 | 33437439 | 5' splice site | TGCTGGGATTACAGGAATGAG ATACCATGGCTGGCCTCTTCTT AGTTTGATTACATTTGCAGGGA TCCTGTTTCCAAATAAGATCAC ATTCACAGGTTCAGGGTAGAC ATGAGTTTTGGTGGGAT |
| 200 | TBX1 | 22 | 19762274 | 5' splice site | CCGGGAGCCCACGTCCCGCAG CACCCTCCAGCCTGCAGAGGC TTCGGGTGGGGGAGGGAGCGG GCCTGTCCTAGAGGCTGGCTCC TGGCATCTGTCTTCTGCCCACC GCCTGCAGGGAGTTGCTT |
| 201 | TBX1 | 22 | 19765917 | 5' splice site | CGCCCGCAGGCCCCGGAACCA CCGGCCCGGCGCACTGCCGCT CATGAGCGCCTTCGCGCGCTCG CGGAACCCCGTGGCTTCCCCG ACGCAGCCCAGCGGCACGGAG AAAGGTAGGGCCGGGGTCG |
| 202 | TBX1 | 22 | 19766926 | 5' splice site | ACAGCCCCGAAGTTCGCCGGG CCCGGCCACCCTGCCCCAAGG GCAAGCAAGGAATACGTTCCC CCAGCCCCAGGGGCCACCGCG GCTCTCCCCTTCCCCAGCCTCG AAGCCATGGGGGCCCCCTC |
| 203 | TBX1 | 22 | 19765114 | 5' splice site | GTCACTGCCTACCAGAACCATC GGGTGAGGGCCTGTGGGGAGG ACCTGAGCGGATTCAACGCCT CTGGAAAAGCGGGTGTAATTT TCAGTTGCCGTTTGGGGACAGT GGGTCCGCTTAGACCTGC |
| 204 | TCF4 | 18 | 55310455 | 5' splice site | CTAGCTCCAAAATGTGGCACCT AAGGAGTGAGGTCTAGAAGTT GGCAGATGTAAGACTCAGCCA CCAGATCCTGCCACCTTCCTCC ACAGGGTGCTACAGAAGGCAA CTGAGGTTTCAAAATCAC |
| 205 | TCF4 | 18 | 55546093 | 5' splice site | TCATGCCTGTAAATCTCAGCAC TTTGGGAGGCCGAGGCAGGAG AATGATTTGAGCCCAGGAGTA AGAGACCAGCCTCCGTAACAA AGTGAGACCTCGTCTCCACAA AAAAAATTTTTAAATCAG |
| 206 | TCF4 | 18 | 55365219 | 5' splice site | GTGTGTGTGTGTGTGTATATAT ATGTGTGTGTGTGTGTGTATAT ATATGTGTGTGTGTATATATATAT ATATATACACACACACACACA TATATATATGATGGCTGGGCTT AGCAGATGTGTAGATT |
| 207 | TCF4 | 18 | 55365243 | 5' splice site | GTGTGTGTGTGTGTGTATATAT ATGTGTGTGTATATATATATAT ATATACACACACACACACATA TATATATGATGGCTGGGCTTAG CAGATGTGTAGATTCTCCTATT GCTGGGTCCCACTTTT |
| 208 | TCF4 | 18 | 55589976 | 5' splice site | GCGGAGGCGGGTGGCTGTTCT CGGGTAGGCGTCGCGCGTGGG GCGGCACTGTGGGAGTTCCCG AGGCCGAGGTCGTTCGCAGGC CCCATTTCCCTGCGCTCTTATT CGTGTTGCCGCTTCTTGGC |
| 209 | TCF4 | 18 | 55585279 | 5' splice site | AACACTAAGAAAAGAATTTAC ATACTTGAGCCAGTAAAATGT CCACTTGCCAAAGAAGTTGGT CCATTTTTCCCACTGCTCACAG GAGGTGAAAACATCTAAAAGA AACAAAGAAATATTACAGT |
| 210 | TGIF1 | 18 | 3456747 | 5' splice site | AAGCTAATAACTAGCTATTTAG AGAACACAGAAACACTTGACA GTCATCTATCAGATAGCATTTC CTTTAATGCCTAAAAGAACCTT CCTTTATGCAACAGACATTAAA AGGAGGTTAAACCTTA |
| 211 | TGIF1 | 18 | 3453560 | 5' splice site | ATTAGCCGGGCGTGGTGGCTTT TGCCTGTAATCCCAGCTATTCG GGAGGCTGAGGCAAGAGAATC GCTTGAACCCTGGAGGCGGAG GCTGCGGTGAGCCGAGGTTGC GGCATTGCACTCCAGCCT |
| 212 | WDTC1 | 1 | 27234952 | 5' splice site | CGGTCGGGGAAGAGACCTGA CAGGTACGGGTCACCGCCGCC CCCTGCCCTCCGCGGGCGCCG AGGCGGGGACCGGCTCCCGCA GCCCCGGTGAACGGCCGCCC GCTCTGGGCCGGCTCCAGCC |
| 213 | CHD7 | 8 | 60808216 | 3' splice site | TGCCTGAAATTTTCTTTTGTTG CAGAAGGAATCTGGAGAGGAG GTAGAAATTGAGGAATTCTAT GTGAAATACAAAAACTTGTAA GTAAATTGTGATTCTGTTTTA ATGGGGGCTATATTTTC |
| 214 | CTNNB1 | 3 | 41237599 | 3' splice site | GTTTTCACTGTCTTCTCTGTCTT AGTTAATGTCAGCTTTGTCTGT CCAGCTGCTCAGGCTAAAACTT TTCTTTCATATAACACATCCTA TCAGCAGCTCCTGTTTGTGGGT AGGCATTTTGCCTT |
| 215 | EHMT1 | 9 | 137654342 | 3' splice site | TACACCACTGCACTCCAGCCTG GGCGACAGAGCGAGGAGACCC CACCCCCCACCCCTTACCACCT CCCCCCCACCAAAAAAAAAAG AGAAAAATTAATTGACCATAA ATATGTGAGTCTGTTTCT |
| 216 | EHMT1 | 9 | 137669068 | 3' splice site | CCCCCGCTAATTTTCTATTTTT AGTACAGACGGGGTTTCACCA TGTTGGGCAAGATCGTCTTGAA CTCCTGATCTCAGGTGATCCGC CTGCCTAGGCCTCTCAAAGTGC TGAGATTACAGGCGT |
| 217 | EHMT1 | 9 | 137688817 | 3' splice site | TGTCATATCACTGCATGGACAG CAGGTGTGTGAAGGTTTGTGTC ATCCACCGAGTCTGCCCTGACT GAACTGGTCACATCCGCGGAT GGCCGTGGTGACCTCTTCTGAG CTCTGATCTGCTATTG |
| 218 | EHMT1 | 9 | 137710966 | 3' splice site | GACGGCTGTTGTTTCTCTCTAA CAGGCAGTTCCGGCGAGGGGG GAGCCTCAGCAGGATTGCTGT GTGAAAACCGAGCTGCTGGGA GAAGGTGAGGGCGGTGTGCAC CGAGGGACAGGAGCAGCGC |
| 219 | EHMT1 | 9 | 137720057 | 3' splice site | TCCTCCACACCAGGGCACAGT CGAGGTGCCGAACCCCCTCCA CACCAGGGCACAGTCGAGGTG CCGAACCCCCTCCACACCAGG GCACAGTCGAGGTGCCGAACC CCCTCCACACCAGGACACAG |
| 220 | EHMT1 | 9 | 137720092 | 3' splice site | CCCTCCACACCAGGGCACAGT CGAGGTGCCGAACCCCCTCCA CACCAGGGCACAGTCGAGGTG CCGAACCCCCTCCACACCAGG ACACAGTCGAGGTGCCGAACC CCCTTCACACCAGGGCACAG |
| 221 | EHMT1 | 9 | 137782290 | 3' splice site | TTAATAAAACTGTGTTTGTTCA CAGTGGACGGAATTGACCCCA ACTTCAAAATGGAGCACCAGA ATAAGCGCTCTCCACTGCACGC CGCGGCAGAGGCTGGACACGT GGACATCTGCCACATGCT |
| 222 | EHMT1 | 9 | 137818583 | 3' splice site | CGCCGTGTACCGAGACCGTAG AGAGGCCGATTGAGGGGCGCC ATGTACCGAGACCGTAGAGAG GCCGACTGAGGGGCGCCGTGT ACCGAGACCGTAGAGAGGCCG ACTGAGGGGCGCCGTGTACC |
| 223 | EHMT1 | 9 | 137669420 | 3' splice site | CCCCACAGCACGTGCACTCCA CGACTGCACCCAAGACGCCGC CCACAGCACGTGCACTCACCTC CACCCAAGACGCCCCCCACAG CACGTGCACTGGACTCCTCCCA AGACGCCCCGCACAGCAC |
| 224 | EHMT1 | 9 | 137669457 | 3' splice site | GCCGCCCACAGCACGTGCACT CACCTCCACCCAAGACGCCCC CCACAGCACGTGCACTGGACT CCTCCCAAGACGCCCCGCACA GCACGTGCACTCGACTCCTCCC AAGACGCCCCGCACAGCAC |
| 225 | EHMT1 | 9 | 137721155 | 3' splice site | CTCCCAGACTTCTCACACTCAC CCCTCCCAGACTTCTCACACTC ACCCCCTCCCAGACTTCTCACA CTCACCCCTCCCAGACTTCTCA CACTCACCCCTCCCAGACTTCT CACACTCGCCCCCTC |
| 226 | EHMT1 | 9 | 137721180 | 3' splice site | TCCCAGACTTCTCACACTCACC CCCTCCCAGACTTCTCACACTC ACCCCTCCCAGACTTCTCACAC TCACCCCTCCCAGACTTCTCAC ACTCGCCCCCTCCCAGACTTCT CACACTCACCCCTCC |
| 227 | EHMT1 | 9 | 137721188 | 3' splice site | TTCTCACACTCACCCCCTCCCA GACTTCTCACACTCACCCCTCC CAGACTTCTCACACTCACCCCT CCCAGACTTCTCACACTCGCCC CCTCCCAGACTTCTCACACTCA CCCCTCCCAGACTTC |
| 228 | EHMT1 | 9 | 137721204 | 3' splice site | CTCCCAGACTTCTCACACTCAC CCCTCCCAGACTTCTCACACTC ACCCCTCCCAGACTTCTCACAC TCGCCCCCTCCCAGACTTCTCA CACTCACCCCTCCCAGACTTCT CACACTCACCCCCTC |
| 229 | EHMT1 | 9 | 137721253 | 3' splice site | TCCCAGACTTCTCACACTCGCC CCCTCCCAGACTTCTCACACTC ACCCCTCCCAGACTTCTCACAC TCACCCCCTCCCAGACTTCTCA CACTCACCCCTCCCAGACTTCT CACACTCACCCCCTC |
| 230 | EHMT1 | 9 | 137721277 | 3' splice site | CTCCCAGACTTCTCACACTCAC CCCTCCCAGACTTCTCACACTC ACCCCCTCCCAGACTTCTCACA CTCACCCCTCCCAGACTTCTCA CACTCACCCCCTCCCCTCTCAC CCCCTCCCACGCCTC |
| 231 | EHMT1 | 9 | 137721310 | 3' splice site | TTCTCACACTCACCCCCTCCCA GACTTCTCACACTCACCCCCTCC CAGACTTCTCACACTCACCCCC TCCCCTCTCACCCCCTCCCACG CCTCACCCCCTCCCACGCCTCT CACCCCCTCCCACGC |
| 232 | EHMT1 | 9 | 137818779 | 3' splice site | GTACCGAGACTGTAGAGAGGC CGACTTAGGGGCGCCGTGTAC CGAGACCGTAGAGAGGCCGAC TGAGGGGCGCCATGTACCGAG ACCGTAGAGAGGCCGACTGAG GGGCGCCGTGTACCGAGACC |
| 233 | EHMT1 | 9 | 137721294 | 3' splice site | CTCACCCCTCCCAGACTTCTCA CACTCACCCCCTCCCAGACTTC TCACACTCACCCCTCCCAGACT TCTCACACTCACCCCCTCCCCT CTCACCCCCTCCCACGCCTCAC CCCCTCCCACGCCTC |
| 234 | GRN | 17 | 44352873 | 3' splice site | CCTCTGCTCAGGCCTCCCTAGC ACCTCCCCCTAACCAAATTCTC CCTGGACCCCATTCTGAGCTCC CCATCACCATGGGAGGTGGGG CCTCAATCTAAGGCCTTCCCTG TCAGAAGGGGGTTGTG |
| 235 | GRN | 17 | 44350227 | 3' splice site | GTATCCTGGGTCATCTTGTCCA CAGGTAACAACTCCGTGGGTG CCATCCAGTGCCCTGATAGTCA GTTCGAATGCCCGGACTTCTCC ACGTGCTGTGTTATGGTCGATG GCTCCTGGGGGTGCTG |
| 236 | GRN | 17 | 44352014 | 3' splice site | ACGCCCTTTCCTGCCCACCCCC CAGGCTGTCTGCTGCTCGGACC ACCAGCACTGCTGCCCCCAGG GCTACACGTGTGTAGCTGAGG GGCAGTGTCAGCGAGGAAGCG AGATCGTGGCTGGACTGG |
| 237 | HTR7 | 10 | 90809677 | 3' splice site | AGCGGCCAGGCGTTCCTCCAG AACCTCCTCTGCCAGGAGCTTG CTACAAGTGCCAGAAATCTGG CCACCAGGCCAAGGAATGCCT GCAGCCCAGGATTCCTCCTAA GTCACGTCCCATCTGTGCA |
| 238 | JAK2 | 9 | 5056554 | 3' splice site | ATTCAGCATAGTTTTCTGTTCT TAGATAAAAATATGATAGTAC ACCTACTGCCTAGTAGACTGTG AGCCTTTTGAAGGTAAGGATC ATATTTTTATGTGTGTTTGTAC CCCCTACACCTTGCACA |
| 239 | KCNQ4 | 1 | 40802306 | 3' splice site | TCTCCGCCCCTGGCCTCGGGGA CGCGGCGGCACCTTCACACTTT CCGCTTGGCCCGCTGCTCGCCT TTCAGGCCGCCGCGTTTTCAAT TGTTAATTTGGAAACGGAAAA AGTAGCCGGCCGGGCG |
| 240 | KCNQ4 | 1 | 40826370 | 3' splice site | CATGGAGTGTTTGTTCAAAATG CAGATACCTGGACCTGTCTCCA AGCCCACAGGATCAGAATCTC TGGAGAAGAGACACAGCCTTC TGCATATGTAACATGGTCCCCA GGGGATCCCTATGCAAA |
| 241 | LEPR | 1 | 65493866 | 3' splice site | TTCTAATCTAATGTGCACTCCA CAGGAACCCTATTCTGTCAACA GACCTGGATTCTGATATCAATG AAATAGTGCACATCTTGAAAA CATTTCCACTAATGTAAACAAG AAAGATGAAAGTCAAC |
| 242 | LEPR | 1 | 65617963 | 3' splice site | ATTAATTTCCTTCTTTTCCCCTC AGTAAATATCGTGCAGTCACTC AGTGCTTATCCTTTAAACAGCA GTTGTGTGATTGTTTCCTGGAT ACTATCACCCAGTGATTACAA GCTAATGTATTTTAT |
| 243 | LEPR | 1 | 65515132 | 3' splice site | TGGGTGATGGTTGAGGGAGGG ATGCTGTGGAATGAAGGAATA ACATTCAGGATGGCATAGCAT GAACAAAACCAGGAAGTGAAA ATAAATGCAAAGATGCTCTTA AGTCACTGTGAGTTCTCTGC |
| 244 | LIPC | 15 | 58520979 | 3' splice site | GGGGTCTGGAGGGCTTTAAGA TGAGGGCGAAACAAGGACCAC CCATGATTCCAAAGAATATCC GGAACTTGCTTGTTGTCTGTTG GTGCCTTTCTTTCAGCTCATTC CTAGAGGGCGGACAGAGG |
| 245 | MBD5 | 2 | 148089789 | 3' splice site | AAGGTCAGACCTCAAGGAAGT AGAGAAACAAGAACAAACCAA ATCCAAACCCAACAGAAGAAA AGAAATAACAAAGATCAGAGC AGAACTAAATGAAATTGAAAC ACAAAACAATACAAAAGAAA |
| 246 | MBD5 | 2 | 148021437 | 3' splice site | GAGAAAGAAACCAAAAGCCTC TTAGCAACACAGACCCTTTGCT GCTGCTGTTGCTGCTGCTGCTG CTGTTGCTGCTGCTGCTGCTAC TGCTGCTGCTGCTACTGCTGCT GCTTGGCCCTGGCTGG |
| 247 | MBD5 | 2 | 148468340 | 3' splice site | ATTCTTTTTTCTCTTTCACATC AGATGCAACTCCAGTAGTACC TTCTCGGGCAGCAACTCCAAG ATCAGTAAGAAATAAGTCTCA TGAAGGAATTACAAATTCTGT AATGCCTGAATGTAAGAA |
| 248 | MBD5 | 2 | 148469445 | 3' splice site | CAAGGTCAACAATAGGGTCCC CAAGGCCATCAATGCCATCAA GCCCTTCTACCAAGTCCGATGG ACATCATCAGTACAAGGATAT CCCTAACCCATTAATTGCTGGA ATAAGTAATGTACTAAAT |
| 249 | MNX1 | 7 | 157005081 | 3' splice site | TTTTCTTTTTCCAAATACTCTGC AGAATGGCGGCTCCAGAGGCG GTTTCAAGTTTCATAAGTCAGG TAACACTGTGGGTTTCCGCCTT CTCGGACGCGGGGAAAGGGGA GACAGGAGGCTTCCCC |
| 250 | MNX1 | 7 | 157006266 | 3' splice site | GCGGCCTGGGGATCACCTTCTT CAGAATGAAAGGAGGGGTGGT TAAGTGCTGATTCTTGGGCCCC ACCCGAAGCTACTGAATCGGC GCTCTGGGCACCTTAGATGAA CCCGTGCGCCCGCCGTCT |
| 251 | NFIA | 1 | 61132694 | 3' splice site | AAACTAAAATCTGTTTCTTTTT CAGGAGGAGTTGGAAGATGAG GAGTAACACAACACTTTAGAT ATACATGTTAAAAACTTATAAC AAATCATAGATCATACTAAAG GATTACCGAAGTTTAGAA |
| 252 | NFIA | 1 | 61310172 | 3' splice site | CTTTGTGTTTTGAAAATCTCTT TAGTGTGCTGGGCCGATGGCC ACATTTTCTGCTGTGTATATGT GATATCAAACTTTAACAAGTCT GTTGCAACAAGGATAAGACAC AGCAGTAGGAAAAGACC |
| 253 | NFIA | 1 | 61383236 | 3' splice site | CTTACCAGTTGATCCTTCTTGC CAGGAATGCCATCTCCAACCA CACTGAAGAAGTCGGAGAAGT CTGGTTTCAGCAGCCCCTCCCC TTCACAGACCTCCTCCCTGGGA ACGGCGTTCACACAGCA |
| 254 | NMU | 4 | 55599182 | 3' splice site | TGCTGACCTTCTTCCATTCCGT GGCTGAAAAATAATAGATTAG AAATAAATCAGTGTAACTAAA GATGCAAGCTAACAGTCATAG AAACAGATTGATGATTTAGAG GAGTGTTTAACGTGCATCA |
| 255 | NOTCH1 | 9 | 136523980 | 3' splice site | GGGCCCACGAAGGCCCCGCCA CAGCTGTTGGCAGATGTGCCA GGGCAGTTAGTTCCCACCTGCT TCCCCAGCGCCCCCGCCACTCA GCACCGGGAACCTGTCATGGG CACAGCCGCCTCCCCCCA |
| 256 | NOTCH1 | 9 | 136535634 | 3' splice site | GCAGGGTGGGTGGAGAGGGGA GCACTCAGGATCCCTCCTGGG GCAATGGGTGCAGGGTGGGTC GGGGGGAGCACTCAGGATCCC TCCCAGGGGCAATGGGTGCAG GGTGGGAGTGGGTGGAGGGG |
| 257 | NOTCH1 | 9 | 136542685 | 3' splice site | GTGGGGGCCGGGGAGGGAGGG GGCCTGCTGCCCACAGGCTGG GCAGTCGGCACAGGCAGGAGC CGGGCTGGGGTTGTCAAGGGA ATGGGACCTTCTGCAGTTGGA ATGAGGGCGCGCAGACTGGC |
| 258 | NSD1 | 5 | 177161724 | 3' splice site | TTGTTTTTTGAGATAGAGTCCA GCCTGGAGTGCTGTGGCATGA TCTTGGCACACTTGCAACCTCT GCCTCCTGGGTTCAGGTGATTC TCCTCCTCTAGCCTCCTCCACG CCTGGCTAATATTTGT |
| 259 | NSD1 | 5 | 177146699 | 3' splice site | CTGTATAGGTTAGTATATCCAT GACTCCCGTCAACTTTCTAAAT GTTCGCTGGGTGCAGTGGCTCA TGCCTGTAATCCCAGCACTTTG GGAGGCTGAGGCGGCTGGATC ACCTGAGGTCAGTAGT |
| 260 | PAX6 | 11 | 31812817 | 3' splice site | GTACGCTAGACTCTTCTTGACT TAGGGAAAGTGCTGAAAGTCA GAGCTCATCAATAAAATCTGC GCGACCTGAACTCGCTGCTGA CTGGCCGCGTCGCCGCAGCCC CTGGCAGCCGGGACAGTGA |
| 261 | PHIP | 6 | 78954964 | 3' splice site | TAATCTTCTTCTAGGCTGATGG TCCTGTGATAAAAGTGTTCAAA TATATTAATAAAAGAGCACTT ACACAATAAAATTTGTACTTTT AATGTAGTCTTAGATAATTGGG TAATATACAATAATTC |
| 262 | PKD1 | 16 | 2105069 | 3' splice site | GAGGGGAGAGTGGAGGGCACA GAGCAGCATCTTCTTAGTCCCT CCCCACATCTGGGCCCCTCTTT ACACCCTGGGTCCCCCGAGAG GCACCCTGCGTTCACACAGGA CAGAACGGCTGAGGCTAC |
| 263 | PYY | 17 | 43966533 | 3' splice site | TGAACTTGCCTGAATGAGTTTC CTCTGGAATACAAGGAAGTCA TATGAAATCGCAGTTTAGGCA GTGCATTCAAGAGTACATGGC AGACATCGTTTCGTGTTAATAA CTATATCCTGCCCTTTAC |
| 264 | RAI1 | 17 | 17792932 | 3' splice site | CCTCCCTTCCTTTTCTTTTCAC AGATAACCAGCCCGAGTCATG CAGTCTTTTCGAGAAAGGTGTG GTTTCCATGGCAAACAACAGA ACTACCAGCAGACCTCGCAGG AAACATCACGCCTAGAG |
| 265 | RBPJ | 4 | 26335132 | 3' splice site | GCTGCCACAGGTAAACGTTAA CTTCAGTAACTTCCTTATTTCC TCCCACTATCAGTTTTTCTTATT TTCTTATTATTATTATTATTGTT TTCGAGATGAAGTTTTGCTCTG TCACCCAGGCTGGA |
| 266 | RBPJ | 4 | 26406174 | 3' splice site | CAGTAATATTTGTATTTGTTTT TAGGGAAGCTATGCGAAATTA TTTAAAAGAGCGAGGGGATCA AACAGTACTTATTCTTCATGCA AAAGTTGCACAGAAGTCATAT GGAAATGAAAAAAGGTAA |
| 267 | RBPJ | 4 | 26383884 | 3' splice site | GTTTCTTTCTTTGTTTTTGAGAC AGGGTCTTGCTCTGTCATCCAG GCTAGAGTGGACTGGTGCGAT CTCAGCTCACTGCAGCCTCGAC TTCCTGGACTCAAGTGATCCTC TTACCTCAGCCTCCT |
| 268 | RBPJ | 4 | 26386352 | 3' splice site | ATTTTCTTTATTTTTTTTTTCC AGGAAATTTGGTGAGCGGCCT CCACCTAAACGACTTACTAGGT GAGTATTATATTAGTCAGCTTT TTACACATACATTTTATGAAAG TATAAATCTTATTGT |
| 269 | RPS14 | 5 | 150444326 | 3' splice site | CGGCGACCCCCCTTCCTGCGAG TGCTGTCAGAGGGGATGGGGG TGACATCCTCTGTGGGGAGGA AGAGAAAGCGTCATTGCCTGG AGCTGGATGGGAAGGGCCCCC AGGACTCCCTAGACCAATG |
| 270 | RPS14 | 5 | 150444354 | 3' splice site | CAGAGGGGATGGGGGTGACAT CCTCTGTGGGGAGGAAGAGAA AGCGTCATTGCCTGGAGCTGG ATGGGAAGGGCCCCCAGGACT CCCTAGACCAATGGCCTAACC AATCAGCAACAGATCCTGCT |
| 271 | RUNX2 | 6 | 45328660 | 3' splice site | GTTTGGGTATGGTTTGTATTTT CAGTTTAAGGCTGCAAGCAGT ATTTACAACAGAGGGTACAAG TTCTATCTGAAAAAAAAAGGA GGGACTATGGCATCAAACAGC CTCTTCAGCACAGTGACAC |
| 272 | RUNX2 | 6 | 45470323 | 3' splice site | ACTTTCTTTCCATCTCTTGCCTC AGATGCATTTGAGCCTCGCCCA GAGCCCAGCTTGATAGAAAAA TGCCAGACTTGGGTTGAATCA GTCAATTAAAAACCAGCTCTTC TTGTAGTTTGAGAGAC |
| 273 | RUNX2 | 6 | 45512245 | 3' splice site | AGATTTTTCTTTTTCTTTTTCCC AGACCCCAGGCAGGCACAGTC TTCCCCGCCGTGGTCCTATGAC CAGTCTTACCCCTCCTACCTGA GCCAGATGACGTCCCCGTCCAT CCACTCTACCACCCC |
| 274 | RUNX2 | 6 | 45365302 | 3' splice site | TGGACTAGCTGCCGTATTATTC ATCTAAATAAAAAGGAGAAAT AAAGCTTACAAAATGTACCTA GCTATAAGTAAATGCAAAAAA AAAAGAAAGCAAATATAAATT ACTTCAAAAAGTAGAGAAA |
| 275 | RUNX2 | 6 | 45368432 | 3' splice site | CACGCCTTTCCTCAGAAAATAT TGCTATCAAAAAAAAAGTTTT AATTGTAGTGAAGTTAATCAC AAAAAGTGAGTTAAACAAAAT GCACTGAACATGCTACTGGGTT TCTAGTTAGATCTCATAT |
| 276 | RUNX2 | 6 | 45371775 | 3' splice site | AATCTTAGGTAGATGGATAAG CAACTATAACAGACAAACATA TATGCAAATTGGTGGGAGGGA ACCAAAGACATCTGAGCAGAC TTCAGAACTACAGTTTGAAAAT CACTAATTTCCTCCAAATC |
| 277 | RUNX2 | 6 | 45421660 | 3' splice site | CCTACAAAGAGGGGTTCCTT GTCCTAGGGGTGGGGAGAGCA AGAGGACGCGACCTGGCGAGA TTGCCCCTAGTCTGGGGCCGCC TCTAGGCTTCGGAAACTGCAC ACGGCGCGCGGGGGGCTCC |
| 278 | SETBP1 | 18 | 45063222 | 3' splice site | AACAAGATCCTGAAGGCCAAG CGGCTGCAGAGACAATCAAAA ACAGGCAACAACTTCGTGAAG AAGAGGCGCGGGCGTCCCAGG AAGCAGCCCACCCAGTTCGAT GAGGACTCCAGAGACCAAAT |
| 279 | SETBP1 | 18 | 45063348 | 3' splice site | CCGGTGCTGGAAAAATGCATC GACCTGCCCAGCAAAAGAGGC CAGAAGCCCAGCCTGAGCCCG CTGGTGCTGGAGCCCGCCGCC AGCCAAGACACCATCATGGCC ACCATCGAGGCGGTCATCCA |
| 280 | SETD5 | 3 | 9432166 | 3' splice site | AATACTGACCTGCATTCTTGAT GTACCCCTCCCCCGTTCCCATC TTGCAGACACTTTTAAGTAATA AAATGCATGCACCTCACTAAC AGAGAATACTTTCCTCTGTCTA AAGTTCTTCACTTAGA |
| 281 | SETD5 | 3 | 9446775 | 3' splice site | AAAGTACTGGGATTACAGGCG TGAGCCACTGCACCCATCCTGT TTTTCTTTCATATTAAAGTTTC AAAATAACTGACCCTTTTTGAA AAGTTTATGTTTGACCTTGCCT TTATTTACAACTATTC |
| 282 | SETD5 | 3 | 9428822 | 3' splice site | TAGATATTTTCCTTTTCTGTTAC AGGATTCCTCATGTCCATAACA TGTTGGATGAGGCTCTGCAGCT CACCCCCACTCTCAGAGTGGTC AGTCTCCATTAATTGGACCCCG TGATTTCCAATCTC |
| 283 | SETD5 | 3 | 9445124 | 3' splice site | CAAAAAAGGAATCCTAATGCT ACAGAACTGCCACTCCTACCA CCTCCTCCAAGCCTACCCACCA TTGGAGCAGAGACTAGACGTA GAAAAGCACGACGGAAAGAGC TAGAGATGGAGCAGCAGAA |
| 284 | SETD5 | 3 | 9446677 | 3' splice site | TGGGTAATTTTTGTATTTTAG TAGAGACGGAGTTTCACCATG TTGGCCACGACGGTCTCAATCT CCCGACCTCATGATCTTCCCAC CTCAGCCTCCCAAAGTACTGG GATTACAGGCGTGAGCC |
| 285 | SETD5 | 3 | 9475299 | 3' splice site | TAAGTCATCATCTGCTTGTCCT CAGGTAATAAATTATGCCAGA AGATGAATACGGTGATCAAAG ACAGACATTTTACTGCCTTTGG TTTCCTAAAAAGAATACATGGT TAAAAGATGAAGAAAAA |
| 286 | SHANK3 | 22 | 50714916 | 3' splice site | GAGATGGAGCCTCCTTGCTGTG CAGAGAAGCTGGACGAGATGC TGGCAGCCGCCGCAGAGCCAA CGCTGCGGCCAGACATCGCAG ACGCAGACTCCAGAGCCGCCA CCGTCAAACAGAGGCCCAC |
| 287 | SYNGAP1 | 6 | 33437091 | 3' splice site | ATCTCTGTGTCTCCAAATCTCC CTCTCCATATAAAGACACCAGT TTTTATAAGGTGGGTTAAGGGT CCACTCTAATTCAGTATGGCCT CTTTTTTTTGAGATGGAATATC GCTCTTGTTTCCCAG |
| 288 | TBX1 | 22 | 19763485 | 3' splice site | ATGCTGCCCGATCAACCCGCTC CCTCCTCCACTCCCATCTGACC CCAGACCCACAACCCTACTCC ATGCCCTCTCAGAACCCGCCTC TGGAGCCGCAGGCTGCAGACA GCTCTTGCTCCCCTGGG |
| 289 | TBX1 | 22 | 19766590 | 3' splice site | CGAACCCCGAGCTGCGCCTGG AGGCGCCCGGCGCATCGGAGC CGCTGCACCACCACCCCTACA AATATCCGGCCGCCGCCTACG ACCACTATCTCGGGGCCAAGA GCCGGCCGGCGCCCTACCCG |
| 290 | TBX1 | 22 | 19766975 | 3' splice site | AGGAATACGTTCCCCCAGCCC CAGGGGCCACCGCGGCTCTCC CCTTCCCCAGCCTCGAAGCCAT GGGGGCCCCCTCGCCACCCCC AGCCCCTTGGGCTATCGAAGT ATCCGGTTCCCCAGTCCCT |
| 291 | TBX1 | 22 | 19765591 | 3' splice site | TCCTCAGACCCCCGCCCCCCTG CAGGAGGGAGCACCAGCTCCG TAGAGGAGGGGCAGACGTGGA CTGGTTCTTGTCAGGGCAGCAG AAAGGCCCTTGGTGCGCTTCTC CTAACACTCCCCTATCC |
| 292 | TCF4 | 18 | 55314928 | 3' splice site | CTATAATTCTCACCCTTAGGTT TGTGGAGGTGAAAAAGCCAAG TCAAATGTTAGCCTCCATTGTT AACATCCCCTATTCATTTTCAG GACAGCTACAATGGAAGCAGC TGGAATTCAACTCTCCC |
| 293 | TCF4 | 18 | 55551211 | 3' splice site | TGCCCACCTCAGCCTCCCAAAG TGCTGGGATTACAGGCGTGAG TCATATCACCCAGCCCTCTTTA CATATTATTCTTCAAACACCTC AAAAATCATTCTCCTGCTGCAT GCTGTGGCTCCTGCAA |
| 294 | TCF4 | 18 | 55365308 | 3' splice site | TATATATGATGGCTGGGCTTAG CAGATGTGTAGATTCTCCTATT GCTGGGTCCCACTTTTCTGTTA AGGCTTACTTCCTTACTGCTTT TGCCCAAATTATTTCTTGTAGA AGCACTGGATAAATG |
| 295 | TCF4 | 18 | 55597079 | 3' splice site | CTCTCTCCTGCTGTGCCATGTG AAGATGTGCCTGCTTCCCCTTT GTCTGCTGTGACTATAGGTTTC CTGAGGCCTCCCCAGCCATGC ATCCTGTACAGCCTGTGGAACT GTGAGTCAATTAAACT |
| 296 | TCF4 | 18 | 55461116 | 3' splice site | ATAGGGAGTCCCATCTCCATA GTTCTGTAAATAAAATGACAG TGTAAGTTATTATTTTATATTA ATAAACAGCACATAAACAAAC ATAGCTCCACTACCAAAACTTC TCCCCTCCAAAGAAATTG |
| 297 | TGIF1 | 18 | 3457709 | 3' splice site | CTGCCAGTCGGTCGGTGTGGG ACAAAACACAGATATACAGCA GATAGCGGCCAAAAACTTCAC AGACACCTCTCTCATGTACCCA GAGGACACTTGTAAATCTGGA CCAAGTACGAATACACAGA |
| 298 | TGIF1 | 18 | 3453427 | 3' splice site | AGGGCTGGGCGCGGTGGTTCA CGCCTGTAATCCCAGCACTTTG GGAGGCCTAGGCGAGCAGATC ATCTGAGGTCGGGAGTTCGAG ACCAGCCTGACCAACATGGAG AAACCCCGTCTCTCCTAAA |
| 299 | WDTC1 | 1 | 27263151 | 3' splice site | TCACGAATTTGTTTCTGTCCCC TAGGAGCGGGGTGCCCTGAGC TTTGAGCGGCGCTACCATGTCA CTGACCCCTTTATCCGGCGGCT GGGCCTGGAAGCAGAGCTGCA GGTAAGAGATCCAGTTT |
| 300 | WDTC1 | 1 | 27271658 | 3' splice site | TTTAATTTAATTTATTTTATTTG AGATGGAGTCTCACTCTGTTGC CCAGGCTGGAGTGCAATGGCA TGATCTCATCTCACCACAGCCT CCGCCTCCTGGGTTCAAGTGAT TCTCCTGCCTCAGCC |

## Claims

1. A method of identifying a non-productive splice site in a target RNA transcript expressed from a gene that is related to a disease or a disorder of haploinsufficiency, the method comprising:
a) incubating a cell with an affinity label to facilitate incorporation of the affinity label into nascent RNA;
b) capturing the affinity labeled nascent RNA with a solid support comprising specificity for the affinity label;
c) isolating the affinity labeled nascent RNA;
d) sequencing the isolated affinity labeled nascent RNA or cDNA prepared therefrom;
e) identifying split reads that do not map to exon-exon junctions of the target RNA transcript; and
f) calculating the probability that the split reads represent non-productive transcripts, thereby identifying non-productive splice sites in the target RNA transcript.

2. A method of identifying a non-productive splice site in a target RNA transcript expressed from a gene that is related to a disease or a disorder of haploinsufficiency, the method comprising:
a) incubating a cell with an affinity label to facilitate incorporation of the affinity label into nascent RNA;
b) capturing the affinity labeled nascent RNA with a solid support comprising specificity for the affinity label;
c) isolating the affinity labeled nascent RNA;
d) enriching the target RNA transcript from the isolated affinity labeled nascent RNA;
e) sequencing the enriched isolated affinity labeled nascent RNA or cDNA prepared therefrom; and
f) identifying nascent RNA transcript intermediates, thereby identifying non-productive splice sites in the target RNA transcript.

3. The method of claim 1 or 2, wherein step a) comprises incubating the cell for less than about 30 minutes in media containing the affinity label, optionally
wherein the affinity label comprises 4-thiouridine, 6-thio-guanosine, 5-ethynyl-uridine, or bromodeoxyuridine, optionally
wherein the 4-thiouridine labeled nascent RNA is biotinylated to produce biotinylated nascent RNA, optionally
wherein the biotinylated nascent RNA is captured in step b) with a streptavidin linked solid support, optionally
wherein the bromodeoxyuridine labeled nascent RNA is captured in step b) with an anti-bromodeoxyuridine antibody.

4. The method of claim 2, wherein:
(a) the target enrichment in step d) comprises a pulldown step using nucleic acid probes complementary to the target RNA transcript, or
(b) the target enrichment in step d) comprises a pulldown step using nucleic acid primers complementary to the target RNA transcript for selective reverse transcription; optionally wherein the cell expresses the target RNA transcript.

5. The method of any one of claims 1 and 3-4, wherein the target RNA transcript exon-exon junctions are annotated target RNA transcript exon-exon junctions or unannotated target RNA transcript exon-exon junctions.

6. The method of any one of claims 2-5, further comprising:
g) identifying split reads that do not map to annotated target RNA transcript exon-exon junctions; and
h) calculating the probability that the split reads represent non-productive transcripts.

7. The method of any one of claims 1-6, wherein:
(a) the non-productive transcripts are rapidly degraded, or
(b) the non-productive transcripts are not translated into a functional protein; optionally wherein:
(i) the target RNA transcript comprises ADAR, ARSA, ATP1A2, CACNA1A, DNMT1, EIF2B1, EIF2B2, EIF2B5, IDUA, MFSD8, NF2, NPCl, PEX1, PRICKLE2, PRRT2, RAI1, SETD5, SHANK3, SLC6A1, STXBP1, STX1B, and TCF4, or
(ii) the target RNA transcript comprises CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1, and WDTC1, or
(iii)the target RNA transcript comprises ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPCl, NSD1, PAX6, PEX1, PHIP, PKD1, PRICKLE2, PRRT2, PYY, RAI1, RBPJ, RPS14, RUNX2, SETBPI1, SETD5, SHANK3, SLC6A1, STXBP1, STX1B, SYNGAP1, TBX1, TCF4, TGIF1, and WDTC1.

8. The method of any one of claims 1-7, wherein the disease or the disorder is of the CNS, optionally wherein the disease or the disorder of the CNS comprises myoclonic-atonic epilepsy (MAE), epilepsy, attention deficit hyperactivity disorder (ADHD), familial hemiplegic migraine-2, familial basilar migraine, alternating hemiplegia of childhood, episodic ataxia type 2, familial hemiplegic migraine, Spinocerebellar ataxia type 6, mental retardation -23, 3p25 microdeletion syndrome, Phelan-McDermid syndrome, shizophrenia-15, Neurofibromatosis (type 1 or type 2, Meningioma, NF2-related, schwannomatosis 1, Hereditary sensory neuropathy type IE, autosomal dominant cerebellar ataxia, deafness, and narcolepsy, Pitt-Hopkins syndrome, Smith-Magenis syndrome, peroxisome biogenesis disorder la, Heimler syndrome-1, metachromatic leukodystrophy, leukoencephalopathy with vanishing white matter, Niemann-Pick disease type CI and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.

9. A method for preparing an antisense oligonucleotide binding to a target region in a target RNA transcript associated with a disease or a disorder of haploinsufficiency, wherein the target region comprises a non-productive splice site, wherein the method comprises:
a) incubating a cell with an affinity label to facilitate incorporation of the affinity label into nascent RNA;
b) capturing the affinity labeled nascent RNA with a solid support comprising specificity for the affinity label;
c) isolating the affinity labeled nascent RNA;
d) sequencing the isolated affinity labeled nascent RNA or cDNA prepared therefrom;
e) identifying split reads that do not map to exon-exon junctions of the target RNA transcript;
optionally identifying split reads that do not map to annotated target RNA transcript exon-exon junctions;
f) calculating the probability that the split reads represent non-productive transcripts, thereby identifying non-productive splice sites in the target RNA transcript; and
g) designing an antisense oligonucleotide comprising a region of complementarity to a target region containing the non-productive splice site, thereby preparing the antisense oligonucleotide.

10. The method of claim 9, wherein step a) comprises incubating the cell for less than about 30 minutes in media containing the affinity label, optionally
wherein the affinity label comprises 4-thiouridine, 6-thio-guanosine, 5-ethynyl-uridine, or bromodeoxyuridine, optionally
wherein the 4-thiouridine labeled nascent RNA is biotinylated to produce biotinylated nascent RNA, optionally
wherein the biotinylated nascent RNA is captured in step b) with a streptavidin linked solid support, optionally
wherein the bromodeoxyuridine labeled nascent RNA is captured in step b) with an anti-bromodeoxyuridine antibody.

11. The method of claim 9 or 10, wherein the target RNA transcript exon-exon junctions are annotated target RNA transcript exon-exon junctions or unannotated target RNA transcript exon-exon junctions.

12. The method of any one of claims 9-11, wherein:
(a) the non-productive transcripts are rapidly degraded, or
(b) the non-productive transcripts are not translated into a functional protein; optionally wherein:
(i) the target RNA transcript comprises ADAR, ARSA, ATP1A2, CACNA1A, DNMT1, EIF2B1, EIF2B2, EIF2B5, IDUA, MFSD8, NF2, NPCl, PEX1, PRICKLE2, PRRT2, RAI1, SETD5, SHANK3, SLC6A1, STXBP1, STX1B, and TCF4, or
(ii) the target RNA transcript comprises CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1, and WDTC1, or
(iii)the target RNA transcript comprises ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPCl, NSD1, PAX6, PEX1, PHIP, PKD1, PRICKLE2, PRRT2, PYY, RAI1, RBPJ, RPS14, RUNX2, SETBPI1, SETD5, SHANK3, SLC6A1, STXBP1, STX1B, SYNGAP1, TBX1, TCF4, TGIF1, and WDTC1.

13. The method of any one of claims 9-12, wherein the disease or the disorder is of the CNS, optionally wherein the disease or the disorder of the CNS comprises myoclonic-atonic epilepsy (MAE), epilepsy, attention deficit hyperactivity disorder (ADHD), familial hemiplegic migraine-2, familial basilar migraine, alternating hemiplegia of childhood, episodic ataxia type 2, familial hemiplegic migraine, Spinocerebellar ataxia type 6, mental retardation -23, 3p25 microdeletion syndrome, Phelan-McDermid syndrome, shizophrenia-15, Neurofibromatosis (type 1or type 2, Meningioma, NF2-related, schwannomatosis 1, Hereditary sensory neuropathy type IE, autosomal dominant cerebellar ataxia, deafness, and narcolepsy, Pitt-Hopkins syndrome, Smith-Magenis syndrome, peroxisome biogenesis disorder 1a, Heimler syndrome-1, metachromatic leukodystrophy, leukoencephalopathy with vanishing white matter, Niemann-Pick disease type CI and Niemann-Pick disease type D, Aicardi-Goutieres syndrome-6, early infantile epileptic encephalopathy-4, progressive myoclonic epilepsy 5, familial infantile convulsion with paroxysmal choreoathetosis, episodic kinesigenic dyskinesia 1, benign familial infantile seizuers-2, or generalized Epilepsy with febrile seizures plus type 9.

14. The method of any one of claims 9-13, wherein
(A) the target RNA transcript associated with the disease or the disorder of haploinsufficiency is selected from the group consisting of ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPCl, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1, and WDTC1, or
(B) the target RNA transcript associated with the disease or the disorder of haploinsufficiency is selected from the group consisting of CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBPI1, SETD5, SHANK3, SYNGAP1, TBX1, TCF4, TGIF1, and WDTC1, or
(C) the target RNA transcript associated with the disease or the disorder of haploinsufficiency is selected from the group consisting of ADAR, ARSA, ATP1A2, CACNA1A, DNMT1, EIF2B1, EIF2B2, EIF2B5, IDUA, MFSD8, NF2, NPCl, PEX1, PRICKLE2, PRRT2, RAI1, SETD5, SHANK3, SLC6A1, STXBP1, STX1B, and TCF4.

15. The method of any one of claims 9-14, wherein the antisense oligonucleotide comprises a region of complementarity to a target region of a target RNA transcript corresponding to any one of the genomic sequences of any one of SEQ ID NOs: 128-300.

## Patentansprüche

1. Verfahren zur Identifizierung einer nicht-produktiven Spleißstelle in einem Ziel-RNA-Transkript, das aus einem Gen exprimiert wird, das mit einer Haploinsuffizienzkrankheit oder - störung in Zusammenhang steht, wobei das Verfahren umfasst:
a) Inkubieren einer Zelle mit einem Affinitätsmarker, um den Einbau des Affinitätsmarkers in entstehende RNA zu erleichtern;
b) Einfangen der affinitätsmarkierten entstehenden RNA mit einem festen Träger, der Spezifität für den Affinitätsmarker umfasst;
c) Isolieren der affinitätsmarkierten entstehenden RNA;
d) Sequenzieren der isolierten affinitätsmarkierten entstehenden RNA oder cDNA, die daraus hergestellt ist;
e) Identifizieren von geteilten Reads, die Exon-Exon-Übergängen des Ziel-RNA-Transkripts nicht zugeordnet werden; und
f) Berechnen der Wahrscheinlichkeit, dass die geteilten Reads nicht-produktive Transkripte darstellen, wodurch nicht-produktive Spleißstellen in dem Ziel-RNA-Transkript identifiziert werden.

2. Verfahren zur Identifizierung einer nicht-produktiven Spleißstelle in einem Ziel-RNA-Transkript, das aus einem Gen exprimiert wird, das mit einer Haploinsuffizienzkrankheit oder - störung in Zusammenhang steht, wobei das Verfahren umfasst:
a) Inkubieren einer Zelle mit einem Affinitätsmarker, um den Einbau des Affinitätsmarkers in entstehende RNA zu erleichtern;
b) Einfangen der affinitätsmarkierten entstehenden RNA mit einem festen Träger, der Spezifität für den Affinitätsmarker umfasst;
c) Isolieren der affinitätsmarkierten entstehenden RNA;
d) Anreichern des Ziel-RNA-Transkripts aus der isolierten affinitätsmarkierten entstehenden RNA;
e) Sequenzieren der angereicherten isolierten affinitätsmarkierten entstehenden RNA oder cDNA, die daraus hergestellt ist; und
f) Identifizieren von entstehenden RNA-Transkript-Zwischenprodukten, wodurch nicht-produktive Spleißstellen in dem Ziel-RNA-Transkript identifiziert werden.

3. Verfahren nach Anspruch 1 oder 2, wobei Schritt a) das Inkubieren der Zelle für weniger als etwa 30 Minuten in Medien, die den Affinitätsmarker enthalten, umfasst,
wobei optional der Affinitätsmarker 4-Thiouridin, 6-Thio-Guanosin, 5-Ethinyluridin oder Bromodesoxyuridin umfasst,
wobei optional die 4-Thiouridin-markierte entstehende RNA biotinyliert ist, um biotinylierte entstehende RNA zu erzeugen,
wobei optional die biotinylierte entstehende RNA in Schritt b) mit einem Streptavidinverknüpften festen Träger eingefangen wird,
wobei optional die Bromodesoxyuridin-markierte entstehende RNA in Schritt b) mit einem Anti-Bromodesoxyuridin-Antikörper eingefangen wird.

4. Verfahren nach Anspruch 2, wobei:
(a) die Zielanreicherung in Schritt d) einen Pulldown-Schritt unter Verwendung von Nukleinsäuresonden umfasst, die komplementär zu dem Ziel-RNA-Transkript sind, oder
(b) die Zielanreicherung in Schritt d) einen Pulldown-Schritt unter Verwendung von Nukleinsäureprimern, die komplementär zu dem Ziel-RNA-Transkript sind, zur selektiven reversen Transkription umfasst; wobei optional die Zelle das Ziel-RNA-Transkript exprimiert.

5. Verfahren nach einem der Ansprüche 1 und 3-4, wobei die Ziel-RNA-Transkript-Exon-Exon-Übergänge annotierte Ziel-RNA-Transkript-Exon-Exon-Übergänge oder nicht-annotierte Ziel-RNA-Transkript-Exon-Exon-Übergänge sind.

6. Verfahren nach einem der Ansprüche 2-5, ferner umfassend:
g) Identifizieren von geteilten Reads, die annotierten Ziel-RNA-Transkript-Exon-Exon-Übergängen nicht zugeordnet werden; und
h) Berechnen der Wahrscheinlichkeit, dass die geteilten Reads nicht-produktive Transkripte darstellen.

7. Verfahren nach einem der Ansprüche 1-6, wobei:
(a) die nicht-produktiven Transkripte schnell abgebaut werden, oder
(b) die nicht-produktiven Transkripte nicht in ein funktionelles Protein translatiert werden;, wobei optional:
(i) das Ziel-RNA-Transkript ADAR, ARSA, ATP1A2, CACNA1A, DNMT1, EIF2B1, EIF2B2, EIF2B5, IDUA, MFSD8, NF2, NPC1, PEX1, PRICKLE2, PRRT2, RAI1, SETDS, SHANK3, SLC6A1, STXBP1, STX1B und TCF4 umfasst, oder
(ii) das Ziel-RNA-Transkript CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1 und WDTC1 umfasst, oder
(iii) das Ziel-RNA-Transkript ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPC1, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1 und WDTC1 umfasst.

8. Verfahren nach einem der Ansprüche 1-7, wobei die Krankheit oder die Störung eine Krankheit oder Störung des ZNS- ist, , wobei optional die Krankheit oder die Störung des ZNS myoklonisch-atonische Epilepsie (MAE), Epilepsie, Aufmerksamkeitsdefizit-Hyperaktivitätsstörung (ADHS), familiäre hemiplegische Migräne-2, familiäre basiläre Migräne, alternierende Hemiplegie der Kindheit, episodische Ataxie Typ 2, familiäre hemiplegische Migräne, spinocerebelläre Ataxie Typ 6, mentale Retardierung -23, Mikrodeletionssyndrom 3p25, Phelan-McDermid-Syndrom, Schizophrenie-15, Neurofibromatose (Typ 1 oder Typ 2), Meningiom, NF2-bezogen, Schwannomatose 1, hereditäre sensorische Neuropathie Typ IE, autosomale dominante cerebelläre Ataxie, Taubheit und Narkolepsie, Pitt-Hopkins-Syndrom, Smith-Magenis-Syndrom, Peroxisom-Biogenese-Störung 1a, Heimler-Syndrom-1, metachromatische Leukodystrophie, Leukoenzephalopathie mit verschwindender weißer Substanz, Niemann-Pick-Krankheit-Typ CI und Niemann-Pick-Krankheit Typ D, Aicardi-Goutieres-Syndrom-6, frühe infantile epileptische Enzephalopathie-4, progressive myoklonische Epilepsie 5, familiäre infantile Konvulsion mit paroxysmaler Choreoatheose, episodische kinesigene Dyskinäsie 1, benigne familiäre infantile Anfälle-2 oder generalisierte Epilepsie mit febrilen Anfällen plus Typ 9 umfasst.

9. Verfahren zur Herstellung eines Antisense-Oligonukleotids, das an eine Zielregion in einem Ziel-RNA-Transkript bindet, das mit einer Haploinsuffizienzkrankheit oder -störung assoziiert ist, wobei die Zielregion eine nicht-produktive Spleißstelle umfasst, wobei das Verfahren umfasst:
a) Inkubieren einer Zelle mit einem Affinitätsmarker, um den Einbau des Affinitätsmarkers in entstehende RNA zu erleichtern;
b) Einfangen der affinitätsmarkierten entstehenden RNA mit einem festen Träger, der Spezifität für den Affinitätsmarker umfasst;
c) Isolieren der affinitätsmarkierten entstehenden RNA;
d) Sequenzieren der isolierten affinitätsmarkierten entstehenden RNA oder cDNA, die daraus hergestellt ist;
e) Identifizieren von geteilten Reads, die Exon-Exon-Übergängen des Ziel-RNA-Transkripts nicht zugeordnet werden;
optional Identifizieren von geteilten Reads, die annotierten Ziel-RNA-Transkript-Exon-Exon-Übergängen nicht zugeordnet werden;
f) Berechnen der Wahrscheinlichkeit, dass die geteilten Reads nicht-produktive Transkripte darstellen, wodurch nicht-produktive Spleißstellen in dem Ziel-RNA-Transkript identifiziert werden; und
g) Entwerfen eines Antisense-Oligonukleotids, das eine Region der Komplementarität zu einer Zielregion umfasst, welche die nicht-produktive Spleißstelle enthält, wodurch das Antisense-Oligonukleotid hergestellt wird.

10. Verfahren nach Anspruch 9, wobei Schritt a) das Inkubieren der Zelle für weniger als etwa 30 Minuten in Medien, welche den Affinitätsmarker enthalten, umfasst,
wobei optional der Affinitätsmarker 4-Thiouridin, 6-Thio-Guanosin, 5-Ethinyluridin oder Bromodesoxyuridin umfasst,
wobei optional die 4-Thiouridin-markierte entstehende RNA biotinyliert ist, um biotinylierte entstehende RNA zu erzeugen,
wobei optional die biotinylierte entstehende RNA in Schritt b) mit einem Streptavidinverknüpften festen Träger eingefangen wird,
wobei optional die Bromodesoxyuridin-markierte entstehende RNA in Schritt b) mit einem Anti-Bromodesoxyuridin-Antikörper eingefangen wird.

11. Verfahren nach Anspruch 9 oder 10, wobei die Ziel-RNA-Transkript-Exon-Exon-Übergänge annotierte Ziel-RNA-Transkript-Exon-Exon-Übergänge oder nicht-annotierte Ziel-RNA-Transkript-Exon-Exon-Übergänge sind.

12. Verfahren nach einem der Ansprüche 9-11, wobei:
(a) die nicht-produktiven Transkripte schnell abgebaut werden, oder
(b) die nicht-produktiven Transkripte nicht in ein funktionelles Protein translatiert werden;wobei optional:
(i) das Ziel-RNA-Transkript ADAR, ARSA, ATP1A2, CACNA1A, DNMT1, EIF2B1, EIF2B2, EIF2B5, IDUA, MFSD8, NF2, NPC1, PEX1, PRICKLE2, PRRT2, RAI1, SETD5, SHANK3, SLC6A1, STXBP1, STX1B und TCF4 umfasst, oder
(ii) das Ziel-RNA-Transkript CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1 und WDTC1 umfasst, oder
(iii) das Ziel-RNA-Transkript ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPC1, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1 und WDTC1 umfasst.

13. Verfahren nach einem der Ansprüche 9-12, wobei die Krankheit oder die Störung eine Krankheit oder Störung des ZNS ist, wobei optional die Krankheit oder die Störung des ZNS myoklonisch-atonische Epilepsie (MAE), Epilepsie, Aufmerksamkeitsdefizit-Hyperaktivitätsstörung (ADHS), familiäre hemiplegische Migräne-2, familiäre basiläre Migräne, alternierende Hemiplegie der Kindheit, episodische Ataxie Typ 2, familiäre hemiplegische Migräne, spinocerebelläre Ataxie Typ 6, mentale Retardierung -23, Mikrodeletionssyndrom 3p25, Phelan-McDermid-Syndrom, Schizophrenie-15, Neurofibromatose (Typ 1 oder Typ 2), Meningiom, NF2-bezogen, Schwannomatose 1, hereditäre sensorische Neuropathie Typ IE, autosomale dominante cerebelläre Ataxie, Taubheit und Narkolepsie, Pitt-Hopkins-Syndrom, Smith-Magenis-Syndrom, Peroxisom-Biogenese-Störung 1a, Heimler-Syndrom-1, metachromatische Leukodystrophie, Leukoenzephalopathie mit verschwindender weißer Substanz, Niemann-Pick-Krankheit-Typ CI und Niemann-Pick-Krankheit Typ D, Aicardi-Goutieres-Syndrom-6, frühe infantile epileptische Enzephalopathie-4, progressive myoklonische Epilepsie 5, familiäre infantile Konvulsion mit paroxysmaler Choreoatheose, episodische kinesigene Dyskinäsie 1, benigne familiäre infantile Anfälle-2 oder generalisierte Epilepsie mit febrilen Anfällen plus Typ 9 umfasst.

14. Verfahren nach einem der Ansprüche 9-13, wobei
(A) das Ziel-RNA-Transkript, das mit der Haploinsuffizienzkrankheit oder -störung assoziiert ist, ausgewählt ist aus der Gruppe bestehend aus ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPC1, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1 und WDTC1, oder
(B) das Ziel-RNA-Transkript, das mit der Haploinsuffizienzkrankheit oder -störung assoziiert ist, ausgewählt ist aus der Gruppe bestehend aus CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1 und WDTC1, oder
(C) das Ziel-RNA-Transkript, das mit der Haploinsuffizienzkrankheit oder -störung assoziiert ist, ausgewählt ist aus der Gruppe bestehend aus ADAR, ARSA, ATP1A2, CACNA1A, DNMT1, EIF2B1, EIF2B2, EIF2B5, IDUA, MFSD8, NF2, NPC1, PEX1, PRICKLE2, PRRT2, RAI1, SETD5, SHANK3, SLC6A1, STXBP1, STX1B und TCF4.

15. Verfahren nach einem der Ansprüche 9-14, wobei das Antisense-Oligonukleotid eine Region der Komplementarität zu einer Zielregion eines Ziel-RNA-Transkripts umfasst, das einer beliebigen der genomischen Sequenzen einer beliebigen von SEQ ID NR.: 128-300 entspricht.

## Revendications

1. Procédé d'identification d'un site d'épissage non productif dans un transcrit d'ARN cible exprimé à partir d'un gène qui est lié à une maladie ou à un trouble d'haplo-insuffisance, le procédé comprenant :
a) l'incubation d'une cellule avec un marqueur d'affinité pour faciliter l'incorporation du marqueur d'affinité dans un ARN naissant ;
b) la capture de l'ARN naissant marqué par affinité avec un support solide comprenant une spécificité pour le marqueur d'affinité ;
c) l'isolement de l'ARN naissant marqué par affinité ;
d) le séquençage de l'ARN naissant marqué par affinité et isolé ou de l'ADNc préparé à partir de celui-ci ;
e) l'identification de lectures fractionnées qui ne s'alignent pas sur des j onctions exon-exon du transcrit d'ARN cible ; et
f) le calcul de la probabilité que les lectures fractionnées représentent des transcrits non productifs, identifiant ainsi des sites d'épissage non productifs dans le transcrit d'ARN cible.

2. Procédé d'identification d'un site d'épissage non productif dans un transcrit d'ARN cible exprimé à partir d'un gène qui est lié à une maladie ou à un trouble d'haplo-insuffisance, le procédé comprenant :
a) l'incubation d'une cellule avec un marqueur d'affinité pour faciliter l'incorporation du marqueur d'affinité dans un ARN naissant ;
b) la capture de l'ARN naissant marqué par affinité avec un support solide comprenant une spécificité pour le marqueur d'affinité ;
c) l'isolement de l'ARN naissant marqué par affinité ;
d) l'enrichissement du transcrit d'ARN cible à partir de l'ARN naissant marqué par affinité et isolé ;
e) le séquençage de l'ARN naissant marqué par affinité, isolé et enrichi ou de l'ADNc préparé à partir de celui-ci ; et
f) l'identification d'intermédiaires de transcrit d'ARN naissant, identifiant ainsi des sites d'épissage non productifs dans le transcrit d'ARN cible.

3. Procédé de l'une des revendications 1 ou 2, dans lequel l'étape a) comprend l'incubation de la cellule pendant moins d'environ 30 minutes dans des milieux contenant le marqueur d'affinité, éventuellement
dans lequel le marqueur d'affinité comprend la 4-thiouridine, la 6-thioguanosine, la 5-éthynyl-uridine ou la bromodésoxyuridine, éventuellement
dans lequel l'ARN naissant marqué à la 4-thiouridine est biotinylé pour produire de l'ARN naissant biotinylé, éventuellement
dans lequel l'ARN naissant biotinylé est capturé à l'étape b) avec un support solide lié à la streptavidine, éventuellement
dans lequel l'ARN naissant marqué à la bromodésoxyuridine est capturé à l'étape b) avec un anticorps anti-bromodésoxyuridine.

4. Procédé de la revendication 2, dans lequel :
(a) l'enrichissement de cible à l'étape d) comprend une étape de récupération à l'aide de sondes d'acide nucléique complémentaires au transcrit d'ARN cible, ou
(b) l'enrichissement de cible à l'étape d) comprend une étape de récupération à l'aide d'amorces d'acide nucléique complémentaires au transcrit d'ARN cible pour une transcription inverse sélective ; éventuellement, ladite cellule exprimant le transcrit d'ARN cible.

5. Procédé de l'une quelconque des revendications 1 et 3 à 4, dans lequel les jonctions exon-exon de transcrit d'ARN cible sont des jonctions exon-exon de transcrit d'ARN cible annotées ou des jonctions exon-exon de transcrit d'ARN cible non annotées.

6. Procédé de l'une quelconque des revendications 2 à 5, comprenant en outre :
g) l'identification de lectures fractionnées qui ne s'alignent pas sur des jonctions exon-exon de transcrit d'ARN cible annotées ; et
h) le calcul de la probabilité que les lectures fractionnées représentent des transcrits non productifs.

7. Procédé de l'une quelconque des revendications 1 à 6, dans lequel :
(a) les transcrits non productifs sont rapidement dégradés, ou
(b) les transcrits non productifs ne sont pas traduits en une protéine fonctionnelle ; éventuellement dans lequel :
(i) le transcrit d'ARN cible comprend ADAR, ARSA, ATP1A2, CACNA1A, DNMT1, EIF2B1, EIF2B2, EIF2B5, IDUA, MFSD8, NF2, NPC1, PEX1, PRICKLE2, PRRT2, RAI1, SETD5, SHANK3, SLC6A1, STXBP1, STX1B et TCF4, ou
(ii) le transcrit d'ARN cible comprend CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1 et WDTC1, ou
(iii) le transcrit d'ARN cible comprend ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPC1, NSD1, PAX6, PEX1, PHIP, PKD1, PRICKLE2, PRRT2, PYY, RAI1, RBPJ, RPS14, RUNX2, SETBP1, SETDS, SHANK3, SLC6A1, STXBP1, STX1B, SYNGAP1, TBX1, TCF4, TGIF1, et WDTC1.

8. Procédé de l'une quelconque des revendications 1 à 7, dans lequel la maladie ou le trouble touche le SNC, éventuellement dans lequel la maladie ou le trouble du SNC comprend l'épilepsie myoclonique-atonique (EMA), l'épilepsie, le trouble d'hyperactivité avec déficit de l'attention (TDAH), la migraine hémiplégique familiale-2, la migraine basilaire familiale, l'hémiplégie alternante de l'enfance, l'ataxie épisodique de type 2, la migraine hémiplégique familiale, l'ataxie spinocérébelleuse de type 6, le retard mental-23, le syndrome de microdélétion 3p25, le syndrome de Phelan-McDermid, la schizophrénie-15, la neurofibromatose (type 1 ou type 2, méningiome, forme liée à NF2, schwannomatose 1, la neuropathie sensorielle héréditaire de type IE, l'ataxie cérébelleuse autosomique dominante, la surdité et la narcolepsie, le syndrome de Pitt-Hopkins, le syndrome de Smith-Magenis, le trouble de la biogenèse des peroxisomes 1a, le syndrome de Heimler-1, la leucodystrophie métachromatique, la leucoencéphalopathie avec substance blanche évanescente, la maladie de Niemann-Pick de type CI et la maladie de Niemann-Pick de type D, le Syndrome d'Aicardi-Goutières-6, l'encéphalopathie épileptique infantile précoce-4, l'épilepsie myoclonique progressive 5, les convulsions infantiles familiales avec choréoathétose paroxystique, la dyskinésie épisodique kinésigénique 1, les crises infantiles bénignes familiales-2 ou l'épilepsie généralisée avec crises fébriles plus de type 9.

9. Procédé permettant la préparation d'un oligonucléotide antisens se liant à une région cible dans un transcrit d'ARN cible associé à une maladie ou à un trouble d'haplo-insuffisance, ladite région cible comprenant un site d'épissage non productif, ledit procédé comprenant :
a) l'incubation d'une cellule avec un marqueur d'affinité pour faciliter l'incorporation du marqueur d'affinité dans un ARN naissant ;
b) la capture de l'ARN naissant marqué par affinité avec un support solide comprenant une spécificité pour le marqueur d'affinité ;
c) l'isolement de l'ARN naissant marqué par affinité ;
d) le séquençage de l'ARN naissant marqué par affinité et isolé ou de l'ADNc préparé à partir de celui-ci ;
e) l'identification de lectures fractionnées qui ne s'alignent pas sur des j onctions exon-exon du transcrit d'ARN cible ;
éventuellement, l'identification de lectures fractionnées qui ne s'alignent pas sur des jonctions exon-exon de transcrit d'ARN cible annotées ;
f) le calcul de la probabilité que les lectures fractionnées représentent des transcrits non productifs, identifiant ainsi des sites d'épissage non productifs dans le transcrit d'ARN cible ; et
g) la conception d'un oligonucléotide antisens comprenant une région de complémentarité avec une région cible contenant le site d'épissage non productif, préparant ainsi l'oligonucléotide antisens.

10. Procédé de la revendication 9, dans lequel l'étape a) comprend l'incubation de la cellule pendant moins d'environ 30 minutes dans des milieux contenant le marqueur d'affinité, éventuellement
dans lequel le marqueur d'affinité comprend la 4-thiouridine, la 6-thioguanosine, la 5-éthynyl-uridine ou la bromodésoxyuridine, éventuellement
dans lequel l'ARN naissant marqué à la 4-thiouridine est biotinylé pour produire de l'ARN naissant biotinylé, éventuellement
dans lequel l'ARN naissant biotinylé est capturé à l'étape b) avec un support solide lié à la streptavidine, éventuellement
dans lequel l'ARN naissant marqué à la bromodésoxyuridine est capturé à l'étape b) avec un anticorps anti-bromodésoxyuridine.

11. Procédé de l'une des revendications 9 ou 10, dans lequel les jonctions exon-exon de transcrit d'ARN cible sont des jonctions exon-exon de transcrit d'ARN cible annotées ou des jonctions exon-exon de transcrit d'ARN cible non annotées.

12. Procédé de l'une quelconque des revendications 9 à 11, dans lequel :
(a) les transcrits non productifs sont rapidement dégradés, ou
(b) les transcrits non productifs ne sont pas traduits en une protéine fonctionnelle ; éventuellement dans lequel :
(i) le transcrit d'ARN cible comprend ADAR, ARSA, ATP1A2, CACNA1A, DNMT1, EIF2B1, EIF2B2, EIF2B5, IDUA, MFSD8, NF2, NPC1, PEX1, PRICKLE2, PRRT2, RAI1, SETD5, SHANK3, SLC6A1, STXBP1, STX1B et TCF4, ou
(ii) le transcrit d'ARN cible comprend CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1 et WDTC1, ou
(iii) le transcrit d'ARN cible comprend ADAR, ARSA, ATP1A2, CACNA1A, CHD7, CTNNB1, DNMT1, EHMT1, EIF2B1, EIF2B2, EIF2B5, GRN, HTR7, IDUA, JAK2, KCNQ4, LEPR, LIPC, MBD5, MFSD8, MNX1, NF2, NFIA, NMU, NOTCH1, NPC1, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1, et WDTC1.

13. Procédé de l'une quelconque des revendications 9 à 12, dans lequel la maladie ou le trouble touche le SNC, éventuellement dans lequel la maladie ou le trouble du SNC comprend l'épilepsie myoclonique-atonique (EMA), l'épilepsie, le trouble d'hyperactivité avec déficit de l'attention (TDAH), la migraine hémiplégique familiale-2, la migraine basilaire familiale, l'hémiplégie alternante de l'enfance, l'ataxie épisodique de type 2, la migraine hémiplégique familiale, l'ataxie spinocérébelleuse de type 6, le retard mental-23, le syndrome de microdélétion 3p25, le syndrome de Phelan-McDermid, la schizophrénie-15, la neurofibromatose (type 1 ou type 2, méningiome, forme liée à NF2, schwannomatose 1, la neuropathie sensorielle héréditaire de type IE, l'ataxie cérébelleuse autosomique dominante, la surdité et la narcolepsie, le syndrome de Pitt-Hopkins, le syndrome de Smith-Magenis, le trouble de la biogenèse des peroxisomes 1a, le syndrome de Heimler-1, la leucodystrophie métachromatique, la leucoencéphalopathie avec substance blanche évanescente, la maladie de Niemann-Pick de type CI et la maladie de Niemann-Pick de type D, le Syndrome d'Aicardi-Goutières-6, l'encéphalopathie épileptique infantile précoce-4, l'épilepsie myoclonique progressive 5, les convulsions infantiles familiales avec choréoathétose paroxystique, la dyskinésie épisodique kinésigénique 1, les crises infantiles bénignes familiales-2 ou l'épilepsie généralisée avec crises fébriles plus de type 9.

14. Procédé de l'une quelconque des revendications 9 à 13, dans lequel
(A) le transcrit d'ARN cible associé à la maladie ou au trouble d'haplo-insuffisance est choisi dans le groupe constitué par ADAR, ARSA, ATP1A2, CACNA1A, CHD7, CTNNB1, DNMT1, EHMT1, EIF2B 1, EIF2B2, EIF2B 5, GRN, HTR7, IDUA, JAK2, KCNQ4, LEPR, LIPC, MBD5, MFSD8, MNX1, NF2, NFIA, NMU, NOTCH1, NPC1, NSD1, PAX6, PEX1, PHIP, PKD1, PRICKLE2, PRRT2, PYY, RAI1, RBPJ, RPS14, RUNX2, SETBP1, SETD5, SHANK3, SLC6A1, STXBP1, STX1B, SYNGAP1, TBX1, TCF4, TGIF1, et WDTC1, ou
(B) le transcrit d'ARN cible associé à la maladie ou au trouble d'haplo-insuffisance est choisi dans le groupe constitué par CHD7, CTNNB1, EHMT1, GRN, HTR7, JAK2, KCNQ4, LEPR, LIPC, MBD5, MNX1, NFIA, NMU, NOTCH1, NSD1, PAX6, PHIP, PKD1, PYY, RAI1, RBPJ, RPS14, RUNX2, SETBP1, SETD5, SHANK3, SYNGAP1, TBX1, TCF4, TGIF1 et WDTC1, ou
(C) le transcrit d'ARN cible associé à la maladie ou au trouble d'haplo-insuffisance est choisi dans le groupe constitué par ADAR, ARSA, ATP1A2, CACNA1A, DNMT1, EIF2B1, EIF2B2, EIF2B5, IDUA, MFSD8, NF2, NPC1, PEX1, PRICKLE2, PRRT2, RAI1, SETDS, SHANK3, SLC6A1, STXBP1, STX1B, et TCF4.

15. Procédé de l'une quelconque des revendications 9 à 14, dans lequel l'oligonucléotide antisens comprend une région de complémentarité avec une région cible d'un transcrit d'ARN cible correspondant à l'une quelconque des séquences génomiques de l'une quelconque des SEQ ID N° : 128 à 300.
